(19)

Europäisches
Patentamt
European
Patent Office
Office européen
des brevets

(11)  **EP 4 206 971 A1**

(12)  **EUROPEAN PATENT APPLICATION**
published in accordance with Art. 153(4) EPC

(43) Date of publication:
**05.07.2023 Bulletin 2023/27**

(21) Application number: **21861365.1**

(22) Date of filing: **19.08.2021**

(51) International Patent Classification (IPC):
*G06F 30/12* (2020.01)　　　*G06F 30/27* (2020.01)

(52) Cooperative Patent Classification (CPC):
**G06F 30/12; G06F 30/27**

(86) International application number:
**PCT/JP2021/030303**

(87) International publication number:
**WO 2022/044937 (03.03.2022 Gazette 2022/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.08.2020　JP 2020145336**

(71) Applicant: **Panasonic Intellectual Property
Management Co., Ltd.
Osaka-shi, Osaka 540-6207 (JP)**

(72) Inventors:
• **TANAKA, Takehiro
  Osaka-shi, Osaka 540-6207 (JP)**
• **WAKASUGI, Kensuke
  Osaka-shi, Osaka 540-6207 (JP)**
• **MORIKAWA, Koji
  Osaka-shi, Osaka 540-6207 (JP)**

(74) Representative: **Eisenführ Speiser
Patentanwälte Rechtsanwälte PartGmbB
Postfach 31 02 60
80102 München (DE)**

(54)  **PROPERTY DISPLAY METHOD, PROPERTY DISPLAY DEVICE, INFORMATION PROCESSING
DEVICE, AND PROGRAM**

(57)　A property display method includes obtaining variables and pieces of option data (S101), determining compositions of the compounds by selecting one of the pieces of option data for each of the variables, obtaining predicted property values corresponding to the compositions (S1 01), obtaining experimental property values corresponding to the compositions (S101), generating a map indicating the predicted property values at positions corresponding to the compositions, generating a first image in which the experimental property values are superimposed upon the map, outputting the first image to a display unit (S102 and S103), obtaining a piece of compound identification information corresponding to a selected one of the experimental property values on the map (S104), obtaining a piece of compound information data relating to the experimental property value (S105), generating a second image including a compound data image indicating the compound information data and the first image, and outputting the second image to the display unit (S106 to S1 10).

FIG. 8

**Description**

Technical Field

The present disclosure relates to a technique for displaying properties of materials.

Background Art

In material development, experiments have been conducted with different compound ratios of raw materials, and composition formulae or ranges of processing conditions that produce excellent properties have been identified in order to find composition formulae or processing conditions that produce desired properties. Property display methods for displaying experiment values or predicted values of material properties have been proposed (e.g., refer to PTL 1 and NPL 1).

Citation List

Patent Literature

PTL 1: Japanese Patent No. 4009670

Non Patent Literature

NPL 1: Kei Terayama, Koji Tsuda, Ryo Tamura. Efficient recommendation tool of materials by an executable file based on machine learning. Japanese Journal of Applied Physics, 58 (098001) 2019.

Summary of Invention

With the techniques in the examples of the related art, however, it is difficult to appropriately support material development.

The present disclosure provides a property display method, a property display apparatus, an information processing apparatus, and a program for performing the property display method capable of appropriately supporting material development.

A property display method according to an aspect of the present disclosure includes a first step of obtaining variables for determining compositions of compounds and pieces of option data indicating possible values or elements of each of the variables, a second step of determining the compositions of the compounds by selecting one of the pieces of option data for each of the variables and obtaining predicted property values of the compounds corresponding to the determined compositions of the compounds, a third step of obtaining experimental property values of the compounds corresponding to the determined compositions of the compounds, a fourth step of generating a map indicating, at positions corresponding to the determined compositions of the compounds, the predicted property values of the compounds corresponding to the compositions, generating a first image by superimposing the experimental property values upon the map at positions corresponding to the compositions of the compounds according to the experimental property values, and outputting the first image to a display unit, a fifth step of obtaining a piece of compound identification information corresponding to a selected one of the experimental property values on the map, a sixth step of obtaining a piece of compound information data associated with the piece of compound identification information and relating to the selected experimental property value, and a seventh step of generating a second image including a compound data image indicating the piece of compound information data and the first image and outputting the second image to the display unit.

A property display apparatus according to another aspect of the present disclosure includes a first obtaining unit that obtains variables for determining compositions of compounds and pieces of option data indicating possible values or elements of each of the variables, a second obtaining unit that determines the compositions of the compounds by selecting one of the pieces of option data for each of the variables and that obtains predicted property values of the compounds corresponding to the determined compositions of the compounds, a third obtaining unit that obtains experimental property values of the compounds corresponding to the determined compositions of the compounds, a first image processing unit that generates a map indicating, at positions corresponding to the determined compositions of the compounds, the predicted property values of the compounds corresponding to the compositions, that generates a first image by superimposing the experimental property values upon the map at positions corresponding to the compositions of the compounds according to the experimental property values, and that outputs the first image to a display unit, a fourth obtaining unit that obtains a piece of compound identification information corresponding to a selected one of the experimental property values on the map, a fifth obtaining unit that obtains a piece of compound information data associated with the piece of compound identification information and relating to the selected experimental property value, and a second image processing unit that generates a second image including a compound data image indicating the piece of compound information data and the first image and that outputs the second image to the display unit.

A program according to another aspect of the present disclosure causes a computer to perform a process including a first step of obtaining variables for determining compositions of compounds and pieces of option data indicating possible values or elements of each of the variables, a second step of determining the compositions of the compounds by selecting one of the pieces of option data for each of the variables and obtaining predicted property values of the compounds corresponding to the determined compositions of the compounds, a third step of obtaining experimental property values of the compounds corresponding to the determined compositions of the compounds, a fourth step of generating a map indicating, at positions corresponding to the determined compositions of the compounds, the predicted property values of the compounds corresponding to the compositions, generating a first image by superimposing the experimental property values upon the map at positions corresponding to the compositions of the compounds according to the experimental property values, and outputting the first image to a display unit, a fifth step of obtaining a piece of compound identification information corresponding to a selected one of the experimental property values on the map, a sixth step of obtaining a piece of compound information data associated with the piece of compound identification information and relating to the selected experimental property value, and a seventh step of generating a second image including a compound data image indicating the piece of compound information data and the first image in one screen and outputting the second image to the display unit.

It should be noted that these general or specific aspects may be implemented as a system, an integrated circuit, a computer-readable storage medium, or any selective combination of a method, an apparatus, a system, a method, an integrated circuit, a computer program, and a storage medium. The computer-readable storage medium includes, for example, a nonvolatile storage medium such as a CD-ROM (compact disc-read only memory).

According to the present disclosure, material development can be appropriately supported.

Additional benefits and advantages of the disclosed embodiments will become apparent from the specification and drawings. The benefits and/or advantages may be individually obtained by the various embodiments and features of the specification and drawings, which need not all be provided in order to obtain one or more of such benefits and/or advantages.

Brief Description of Drawings

[Fig. 1] Fig. 1 is a block diagram illustrating a functional configuration of a display system according to embodiments 1-1 and 1-2.

[Fig. 2] Fig. 2 is a diagram illustrating an example of a search area.

[Fig. 3] Fig. 3 is a diagram illustrating an example of experimental data.

[Fig. 4A] Fig. 4A is a diagram illustrating an example of a first image.

[Fig. 4B] Fig. 4B is a diagram illustrating another example of the first image.

[Fig. 4C] Fig. 4C is a diagram illustrating a legend and the like of the first images illustrated in Figs. 4B and 5B.

[Fig. 5A] Fig. 5A is a diagram illustrating an example of highlighting of experimental property values.

[Fig. 5B] Fig. 5B is a diagram illustrating another example of the highlighting of experimental property values.

[Fig. 6] Fig. 6 is a diagram illustrating an example of compound composition data and compound graph data.

[Fig. 7] Fig. 7 is a diagram illustrating an example of a compound composition data image and a compound graph data image.

[Fig. 8] Fig. 8 is a diagram illustrating an example of a second image.

[Fig. 9] Fig. 9 is a diagram illustrating an example of highlighting of a selected experimental property value on the second image.

[Fig. 10A] Fig. 10A is a diagram illustrating an example of transition of a display screen according to embodiment 1-1.

[Fig. 10B] Fig. 10B is a diagram illustrating another example of the transition of the display screen according to embodiment 1-1.

[Fig. 10C] Fig. 10C is a diagram illustrating another example of the transition of the display screen according to embodiment 1-1.

[Fig. 11] Fig. 11 is a diagram illustrating another example of the transition of the display screen according to embodiment 1-1.

[Fig. 12] Fig. 12 is a diagram illustrating an example of a compound data image at a time when experimental property values have been selected.

[Fig. 13] Fig. 13 is a diagram illustrating another example of the compound data image at a time when experimental property values have been selected.

[Fig. 14] Fig. 14 is a diagram illustrating an example of transition of a display screen according to embodiment 1-2.

[Fig. 15] Fig. 15 is a diagram illustrating another example of the transition of the display screen according to embodiment 1-2.

[Fig. 16] Fig. 16 is a flowchart illustrating an example of an overall procedure of a process performed by a property display apparatus according to embodiments 1-1 and 1-2.

[Fig. 17] Fig. 17 is a block diagram illustrating a functional configuration of a display system according to embodiment 2.

[Fig. 18] Fig. 18 is a diagram illustrating an example of input edit information.

[Fig. 19] Fig. 19 is a diagram illustrating an example of an edit image.

[Fig. 20] Fig. 20 is a flowchart illustrating an example of an overall procedure of a process performed by a property display apparatus according to embodiment 2.

[Fig. 21] Fig. 21 is a block diagram illustrating a functional configuration of a display system according to embodiment 3.

[Fig. 22] Fig. 22 is a diagram illustrating an example of a first image including a read data button and a save data button.

[Fig. 23] Fig. 23 is a flowchart illustrating an example of an overall procedure of a process performed by a property display apparatus according to embodiment 3.

[Fig. 24] Fig. 24 is a block diagram illustrating a functional configuration of a display system according to embodiment 4.

[Fig. 25] Fig. 25 is a diagram illustrating an example of a second image including a correct data button according to embodiment 4.

[Fig. 26A] Fig. 26A is a diagram illustrating an example of transition from the first image to the second image according to embodiment 4.

[Fig. 26B] Fig. 26B is a diagram illustrating an example of transition from the second image to a fifth image as a result of correction according to embodiment 4.

[Fig. 27] Fig. 27 is a flowchart illustrating an example of an overall procedure of a process performed by a property display apparatus according to embodiment 4.

[Fig. 28] Fig. 28 is a block diagram illustrating a functional configuration of a display system according to embodiment 5.

[Fig. 29] Fig. 29 is a diagram illustrating an example of a first image including a display prediction model button according to embodiment 5.

[Fig. 30] Fig. 30 is a diagram illustrating an example of transition of a display screen according to embodiment 5.

[Fig. 31] Fig. 31 is a flowchart illustrating an example of an overall procedure of a process performed by a property display apparatus according to embodiment 5.

[Fig. 32] Fig. 32 is a block diagram illustrating a functional configuration of a display system according to embodiment 6.

[Fig. 33] Fig. 33 is a diagram illustrating an example of a compound graph data image according to embodiment 6.

[Fig. 34] Fig. 34 is a diagram illustrating another example of the compound graph data image according to embodiment 6.

[Fig. 35] Fig. 35 is a diagram illustrating an example of transition of a display screen according to embodiment 6.

[Fig. 36] Fig. 36 is a flowchart illustrating an example of an overall procedure of a process performed by a property display apparatus according to embodiment 6.

Description of Embodiments

(Underlying Knowledge Forming Basis of Present Disclosure)

In recent years, recognition and identification methods based on machine learning and the like have made remarkable progress in fields such as image recognition and natural language processing, and are beginning to be applied to prediction of material properties, including materials informatics (MI). NPL 1, for example, discloses a method for optimizing material properties and creating phase diagrams using Bayesian optimization. As a demonstrative example, experiment values that are results of a low melting point composition search or a phase diagram search for a NaF-KF-LiF system are superimposed upon a triangular phase diagram along with predicted values. A function of displaying compound information data associated with the experiment values, however, is not incorporated.

PTL 1 handles a huge number of compound ratios, and a compound ratio with which a target physicochemical property can be produced is output. PTL 1, however, does not disclose a method for arranging compound composition data or graph data regarding experiment values with the experiment values and displaying and tracking the data.

The inventors have therefore found that centralized management becomes possible by tracking information regarding experimental data corresponding to experiment values or predicted values and displaying compound information data using an experiment database that stores experimental data to which compound identification information is added or an experiment database that automatically adds compound identification information to experimental data, and have come up with the present disclosure.

That is, a property display method according to an aspect of the present disclosure includes a first step of obtaining variables for determining compositions of compounds and pieces of option data indicating possible values or elements of each of the variables, a second step of determining the compositions of the compounds by selecting one of the pieces of option data for each of the variables and obtaining predicted property values of the compounds corresponding to the determined compositions of the compounds, a third step of obtaining experimental property values of the compounds corresponding to the determined compositions of the compounds, a fourth step of generating a map indicating, at positions corresponding to the determined compositions of the compounds, the predicted property values of the compounds corresponding to the compositions, generating a first image by superimposing the experimental property values upon the map at positions corresponding to the compositions of the compounds according to the experimental property values, and outputting the first image to a display unit, a fifth step of obtaining a piece of compound identification information corresponding to a selected one of the experimental property values on the map, a sixth step of obtaining a piece of compound information data associated with the piece of compound identification information and relating to the selected experimental property value, and a seventh step of generating a second image including a compound data image indicating the piece of compound information data and the first image and outputting the second image to the display unit.

Here, a composition of a compound is determined for each of combinations of pieces of option data obtained by selecting one of the pieces of option data for each of variables. That is, a predicted property value of a compound is obtained for each of the combinations of the pieces of option data. An experimental property value of a compound corresponding to each of at least one combination of the pieces of option data is obtained.

As a result, a first image in which an experimental property value is superimposed upon a map indicating a predicted property value for each of the combinations of the pieces of option data, that is, a predicted property value of each of compositions of compounds, is generated and displayed. When a user of that computer, for example, inputs many variables and many pieces of option data to be substituted for each of the many variables to the computer, therefore, a map indicating a huge amount of information can be displayed. That is, the map associates a predicted property value of each of many possible compositions (i.e., composition formulae or chemical formulae) of compounds with the composition, and an experimental property value of each of at least one of the compositions with the composition. The user, therefore, can easily understand the predicted property values and the experimental property values of all the compositions of the compounds on the map.

Furthermore, in the property display method according to the aspect of the present disclosure, when the user selects, through an input operation, one of the experimental property values superimposed upon the map, a second image is generated and displayed. That is, a compound data image indicating compound information data relating to the selected experimental property value is displayed in the same screen as the first image. The user can thus display, through a simple input operation, a compound data image indicating compound information data relating to an experimental property value that the user is interested in. The user can easily visually understand the compound information data shown in the compound data image, a predicted property value of each of compositions shown in the first image, and the selected experimental property value without switching a screen.

Although an experiment value or a predicted value of a material property is displayed for a search area of a composition formula or a processing condition in PTL 1 or NPL 1, on the other hand, compound information data associated with the experiment value cannot be displayed. Since such compound information data can be displayed with the property display method according to the aspect of the present disclosure, that is, since compound information data can be tracked and displayed as information regarding the above-described experimental data, therefore, an experimental property value, a predicted property value, and compound information data can be managed in a centralized manner. As a result, material development by the user can be appropriately supported.

The property display method may further include a step of generating a reduced first image, which is obtained by reducing the first image, before the second image is generated in the seventh step. In the seventh step, the second image including the compound data image and the reduced first image may be generated and output to the display unit.

Since the first image is reduced in order to generate the second image before the second image is generated, therefore, the first image can be displayed widely in a screen in the fourth step. As a result, visibility of the first image improves.

A screen in which the first image and the second image are displayed in the fourth and seventh steps, respectively, may include a first area and a second area. In the fourth step, the first image disposed in the first area may be output to the display unit. In the seventh step, the second image may be generated by disposing the first image in the first area and the compound data image in the second area and output to the display unit.

As a result, since arrangement and size of the first image displayed in the fourth and seventh steps are the same, a processing load for the first image is reduced and processing speed improves. Furthermore, since the arrangement and the size of the first image remain the same even after a displayed image switches from the first image to the second image, the user's discomfort caused by the switching can be reduced.

The piece of compound information data may include a piece of compound composition data and a piece of compound graph data relating to the experimental property value.

For example, the compound composition data is tabular data indicating information regarding a compound, and the compound graph data is graph data indicating information regarding a compound. Since an image indicating these pieces of data is displayed as the compound data image, therefore, the user can easily understand, from various angles, various pieces of information regarding a compound relating to the selected experimental property value. When the compound composition data and the compound graph data are data used to obtain an experimental property value, raw data of the experimental property value can be easily understood.

In the fifth step, two or more of the experimental property values superimposed upon the map may be selected and a piece of the compound identification information corresponding to each of the two or more experimental property values may be obtained. In the sixth step, a piece of the compound information data associated with each of the pieces of compound identification information may be obtained. In the seventh step, the compound data image may be generated by superimposing a piece of the compound graph data corresponding to each of the obtained pieces of compound information data.

As a result, pieces of compound graph data relating to experimental property values are displayed while being superimposed upon each other, and the user can easily tell differences between the pieces of compound graph data.

The compound data image in the seventh step may include a graph superimposition image in which the piece of compound graph data corresponding to each of the obtained pieces of compound information data is superimposed and a list image in which the piece of compound identification information associated with each of the obtained pieces of compound information data is shown on a list.

As a result, since a list image is displayed, the user can easily understand compound identification information regarding each of the superimposed pieces of compound graph data.

The compound data image in the seventh step may include a graph superimposition image in which the piece of compound graph data corresponding to each of the obtained pieces of compound information data is superimposed and a tab switch image including tabs for switching and displaying the piece of compound composition data corresponding to each of the pieces of the compound graph data.

As a result, even when experimental property values are selected, the user can easily visually understand compound composition data relating to the experimental property values by using the tabs.

After the seventh step, at least one of the superimposed pieces of compound graph data shown in the graph superimposition image may be edited. A third image may be generated on a basis of the edit and output to the display unit. For example, the edit may include switching a state of, among the superimposed pieces of compound graph data, a selected one of the pieces of compound graph data and the piece of compound identification information associated with the selected pieces of the compound graph data between a display state and a non-display state. Alternatively, the edit may include selecting an area in the graph superimposition image and enlarging and displaying the selected area.

As a result, visibility of the superimposed pieces of compound graph data further improves.

After the seventh step, an area in an image that indicates the piece of compound graph data and that is included in the compound data image may be selected and the selected area may be enlarged and displayed.

As a result, even when a compound data image shows one piece of compound graph data, visibility of the compound graph data further improves.

After at least one of the first image or the second image is output to the display unit, the output image may be converted into a file in a certain format and saved. In this case, the saved file may be obtained, and a fourth image may be generated by reconfiguring the obtained file as an image and output to the display unit.

As a result, when files are saved to a cloud server or the like, for example, computers can save files and read and reconfigure files through the server. Consequently, users of the computers can view the same images even from distant locations and vigorously discuss material properties. That is, images can be shared.

In the seventh step, if information included in the piece of compound information data shown in the second image is corrected after the second image is output, the corrected information may be output to a compound information database storing the piece of compound information data before the correction, and a fifth image may be generated by correcting the second image on a basis of the corrected information and output to the display unit.

There is a case, for example, where an experimental property value superimposed upon a first image included in the second image has been calculated on the basis of information included in compound information data. If there is an error in the information included in the compound information data in this case, the experimental property value superimposed upon the first image is a value deviating from a predicted property value. Since the second image is corrected by correcting the information included in the compound information data in the property display method according to the aspect of the present disclosure, therefore, the experimental property value in the second image, for example, can be corrected. Even if there is an error in the compound information data, therefore, the second image can be appropriately corrected easily.

In the fourth step, a prediction model image based on the predicted property values and at least one of the experimental property values shown in the first image may be generated after the first image is output. A sixth image including the first image and the prediction model image may be generated and output to the display unit. In a case where one of the at least one experimental property value shown in the prediction model image is selected, a piece of the compound identification information corresponding to the selected experimental property value may be obtained. A piece of the compound information data associated with the piece of compound identification information may be obtained. A seventh image including an image indicating the piece of compound information data and the sixth image may be generated and output to the display unit.

As a result, not only when one of the experimental property values in the first image is selected but also when one of the at least one experimental property value shown in the prediction model image included in the sixth image is selected, an image indicating compound information data relating to the selected experimental property value is displayed. The user, therefore, can easily select an experimental property value greatly different from a predicted property value in the prediction model image, which indicates a relationship between predicted property values and experimental property values, and refer to compound information data relating to the selected experimental property value. Since the seventh image includes the first image, the prediction model image, and the image indicating the compound information data in one screen, the user can view these images all at once without switching the screen.

In the sixth step, a piece of predicted property data corresponding to the piece of compound identification information may be calculated while obtaining the piece of compound information data associated with the piece of compound identification information, an eighth image may be generated on a basis of the piece of compound information data and the piece of predicted property data, and a ninth image including the eighth image and the first image may be generated and output to the display unit.

As a result, an eighth image indicating differences between compound information data and predicted property data, for example, can be generated and displayed in the same screen as the first image. Consequently, material development can be supported more appropriately.

A number of, among the variables, first variables for which the pieces of option data indicating chemical elements are substituted may be four or more.

As a result, since a map is generated for a compound composed by combining together four or more elements, a map indicating information regarding many composition formulae (or chemical formulae) can be generated by changing the combination. That is, a map indicating a predicted property value of each of compounds expressed by many composition formulae (or chemical formulae) can be generated. Material development, therefore, can be supported more effectively.

A number of, among the variables, second variables for which the pieces of option data indicating values are substituted may be four or more.

As a result, since a map is generated for a compound composed by combining together four or more values (i.e., coefficients of elements), a map indicating information regarding many composition formulae (or chemical formulae) can be generated by changing the combination. That is, a map indicating a predicted property value of each of compounds expressed by many composition formulae (or chemical formulae) can be generated. Material development, therefore, can be supported more effectively.

The map may include an image map defined by a first axis and a second axis corresponding to two of the four or more second variables. The image map may include image element maps arranged in a matrix along the first and second axes. Each of the image element maps may be defined by a third axis and a fourth axis corresponding to other two of the four or more second variables. The predicted property values of the compounds corresponding to the determined compositions of the compounds may be indicated by colors or shades of color on the image element maps at the positions corresponding to the compositions of the compounds.

As a result, even when the number of second variables is four or more, a map on which the user can easily recognize a predicted property value of a compound expressed by each of combinations, that is, each of composition formulae (or chemical formulae), can be generated.

In the second step, the predicted property values of the compounds corresponding to the determined compositions of the compounds may be obtained by inputting the compositions of the compounds to a machine learning model based on a certain calculation algorithm. The machine learning model may be a model subjected to machine learning such that the predicted property values of the compounds corresponding to the determined compositions of the compounds are output in response to an input of the compositions of the compounds.

By performing, on a machine learning model, machine learning using many combinations of pieces of option data and experimental property values corresponding to the combinations as training data, for example, a machine learning model with high prediction accuracy can be generated. Accurate predicted property values, therefore, can be obtained by using the machine learning model.

A property display method according to another aspect of the present disclosure includes a first step of obtaining a map indicating a position corresponding to each of compositions of compounds, a second step of obtaining an experimental property value of each of at least one of the compositions of the compounds, a third step of generating a first image by superimposing the obtained experimental property value upon the map at a position corresponding to the composition corresponding to the experimental property value and outputting the first image to a display unit, a fourth step of obtaining a piece of compound information data corresponding to a selected one of the at least one experimental property value on the map, and a fifth step of generating a second image including an image indicating the piece of compound information data and the first image and outputting the second image to the display unit.

As a result, experimental property values are displayed on a map at positions corresponding to compositions of compounds according to the experimental property values. The user, therefore, can easily understand the experimental property values and the compositions of the compounds according to the experimental property values on the map.

Furthermore, in the property display method according to the aspect of the present disclosure, when the user selects one of the experimental property values superimposed upon the map through an input operation, a second image is generated and displayed. That is, a compound data image indicating compound information data relating to the selected experimental property value is displayed in the same screen as the first image. The user can thus display, through a simple input operation, a compound data image indicating compound information data relating to an experimental property value that the user is interested in. The user can easily visually understand the compound information data shown in the image and the selected one of the experimental property values in the first image without switching the screen.

Although an experiment value or a predicted value of a material property is displayed for a search area of a composition formula or a processing condition in PTL 1 or NPL 1, on the other hand, compound information data associated with the experiment value cannot be displayed. Since such compound information data can be displayed with the property display method according to the aspect of the present disclosure, that is, since compound information data can be tracked and displayed as information regarding the above-described experimental data, therefore, an experimental property value and compound information data can be managed in a centralized manner. As a result, material development by the user can be appropriately supported.

The map obtained in the first step may indicate a predicted property value of each of the compositions of the compounds at a position corresponding to the composition. The property display method may further include a sixth step of obtaining a piece of compound identification information corresponding to the experimental property value selected on the map. In the fourth step, the piece of compound information data associated with the piece of compound identification information and relating to the selected experimental property value may be obtained. In the fifth step, the second image including the image indicating the piece of compound information data and the first image may be generated and output to the display unit.

As a result, a predicted property value of each of the compositions of the compounds is also indicated on the map at a position corresponding to the composition. Since experimental property values are superimposed upon such a map, the user can easily compare a predicted property value and an experimental property value of a composition of the same compound.

The property display method may further include a seventh step of obtaining a predicted property value of each of the compositions of the compounds. In the first step, the map may be obtained by generating the map using the obtained predicted property value.

As a result, even when a map indicating predicted property values cannot be obtained, a computer can obtain predicted property values and generate a map indicating the predicted property values. A map indicating predicted property values, therefore, need not be prepared in advance.

Embodiments of the present disclosure will be described hereinafter with reference to the drawings. The embodiments described hereinafter are specific examples of the present disclosure. Values, shapes, materials, components, arrangement positions and connection modes of the components, and the like mentioned in the following embodiments, therefore, are examples and not intended to limit the present disclosure. Among the components described in the following embodiments, ones not described in the independent claims, which define broadest concepts, will be described as optional components.

The drawings are schematic diagrams, and not necessarily strict illustrations. In each of the drawings, substantially the same components are given the same reference numerals, and redundant description thereof is omitted or simplified.

(Embodiment 1-1)

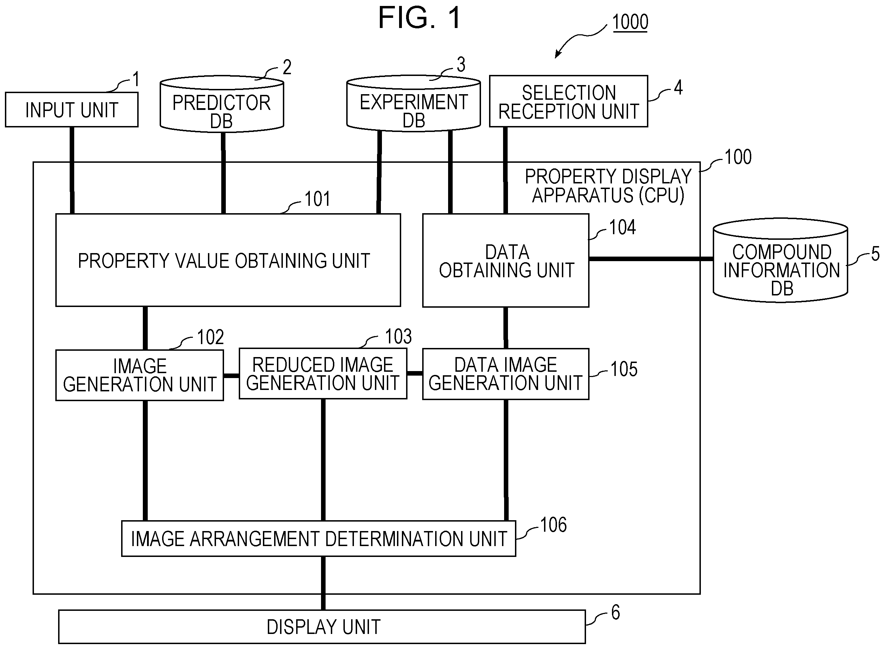

A property display method according to embodiment 1-1 will be described with reference to Figs. 1 to 11. Embodiment 1 includes embodiment 1-1 and embodiment 1-2.

A configuration according to embodiment 1-1 will be described. A display system 1000 according to embodiment 1 of the present disclosure will be described in detail hereinafter with reference to the drawings.

Fig. 1 is a block diagram illustrating the configuration of the display system 1000 according to embodiment 1-1 of the present disclosure. The display system 1000 illustrated in Fig. 1 includes a property display apparatus 100, an input unit 1, a predictor database (DB) 2, an experiment database (DB) 3, a selection reception unit 4, a compound information database (DB) 5, and a display unit 6.

The property display apparatus 100 includes a property value obtaining unit 101, an image generation unit 102, a reduced image generation unit 103, a data obtaining unit 104, a data image generation unit 105, and an image arrangement determination unit 106. The property display apparatus 100 may be achieved, for example, by a processor such as a central processing unit (CPU) and a memory. In this case, the processor functions as the property display apparatus 100 by, for example, executing a computer program stored in the memory. In Fig. 1, the predictor database 2, the experiment database 3, and the compound information database 5 are achieved, for example, by a nonvolatile memory.

Details of the components illustrated in Fig. 1 will be described hereinafter.

[Input Unit 1]

The input unit 1 obtains a search area to be displayed on the basis of a user input and outputs the search area to the property value obtaining unit 101. The input unit 1 may be, for example, a keyboard, a touch sensor, a touchpad, a mouse, or the like.

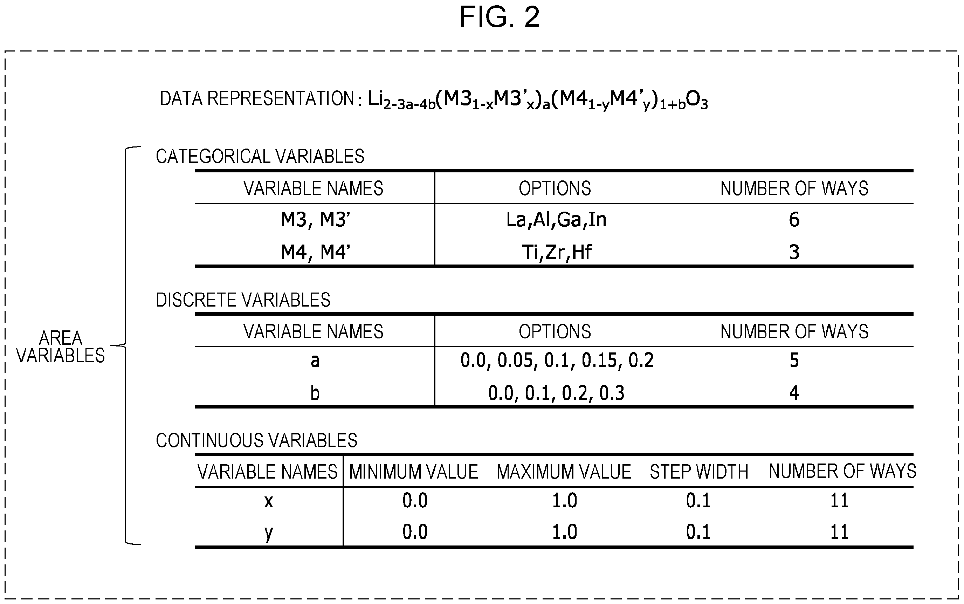

Fig. 2 is a diagram illustrating an example of the search area to be displayed input by the user. Data points included in the search area are expressed as predetermined values (e.g., elements Li and 0 and 3, which is a coefficient of the element 0) and area variables, and form a composition formula (or a chemical formula) when a value or an element is specified for every variable. That is, when a value or an element is specified for every area variable, a data representation indicated by these area variables forms a composition of a compound, that is, a composition formula (or a chemical formula). The data representation is, for example, $Li_{2-3a-4b}(M3_{1-x}M3'_x)_a(M4_{1-y}M4'_y)_{1+b}O_3$ illustrated in Fig. 2. The area variables are also simply called the variables. A compound is synonymous with a material.

Here, the area variables include categorical variables, discrete variables, and continuous variables. For example, the categorical variables include M3, M3', M4, and M4', which are variable names of elements, La, Al, Ga, and In, which are elements that can be selected for each of the variable names M3 and M3', 6 ($_4C_2 = 6$), which is the number of possible combinations of the four elements selected as the variable names M3 and M3', Ti, Zr, and Hf, which are elements that can be selected for each of the variable names M4 and M4', and 3 ($_3C_2 = 3$), which is the number of possible combinations of the three elements selected as the variable names M4 and M4'.

The discrete variables include a and b, which are variable names to which coefficients of the elements are set, 0.0, 0.05, 0.1, 0.15, and 0.2, which are coefficients that can be selected as the variable name a, 5 ($_5C_1 = 5$), which is the number of possible combinations of the five options selected as the variable name a, 0.0, 0.1, 0.2, and 0.3, which are coefficients that can be selected as the variable name b, and 4 ($_4C_1 = 4$), which is the number of possible combinations of the four options selected as the variable name b.

The continuous variables include x and y, which are variable names to which coefficients of the elements are set. When the variable name x and the variable name y each vary from a minimum value 0.0 to a maximum value 1.0 with a step width of 0.1, the number of possible combinations is 11 ($_{11}C_1 = 11$). Here, the continuous variables may be expressed in a format in which all possible options when the variable name x and the variable name y each vary from the minimum value 0.0 to the maximum value 1.0 with the step width of 0.1 are enumerated.

When data is generated while changing a value or an element specified for each of these area variables, various pieces of data are generated and indicate a search area to be displayed. The variables may also include processing conditions (calcination temperature and time, a type of synthesis method, etc.).

That is, the variables in the present embodiment are M3, M3', M4, and M4', which are categorical variables, a and b, which are discrete variables, and x and y, which are continuous variables. With respect to the categorical variables M3 and M3', each of La, Al, Ga, and In is option data indicating an element that can be M3 or M3'. With respect to the categorical variables M4 and M4', each of Ti, Zr, and Hf is option data indicating an element that can be M4 or M4'. With respect to the discrete variable a, each of 0.0, 0.05, 0.1, 0.15, and 0.2 is option data indicating a value that can be a. With respect to the discrete variable b, each of 0.0, 0.1, 0.2, and 0.3 is option data indicating a value that can be b. With respect to the continuous variable x, 0.0, 0.1, 0.2, 0.3, ..., 0.9, and 1.0 are option data indicating values that can be x. With respect to the continuous variable y, 0.0, 0.1, 0.2, 0.3, ..., 0.9, and 1.0 are option data indicating values that can be y. In other words, the variables M3, M3', M4, M4', a, b, x, and y are expressed as follows using the option data.

$M3 \in$ {La, Al, Ga, In}
$M3' \in$ {La, Al, Ga, In}
$M4 \in$ {Ti, Zr, Hf}
$M4' \in$ {Ti, Zr, Hf}
$a \in$ {0.0, 0.05, 0.1, 0.15, 0.2}

be{0.0, 0.1, 0.2, 0.3}

$x \in$ {0.0, 0.1, 0.2, 0.3, 0.4, 0.5, 0.6, 0.7, 0.8, 0.9, 1.0}

ye{0.0, 0.1, 0.2, 0.3, 0.4, 0.5, 0.6, 0.7, 0.8, 0.9, 1.0}

A combination of the option data is then obtained by selecting one of pieces of the option data corresponding to each of M3, M3', M4, M4', a, b, x, and y. The combination is (M3, M3', M4, M4', a, b, x, y) = (La, Al, Ti, Zr, 0.1, 0.1, 0.1, 0.1), (La, Al, Ti, Zr, 0.1, 0.1, 0.1, 0.2), (La, Al, Ti, Zr, 0.1, 0.1, 0.1, 0.3), or the like. That is, many combinations exist. Such a combination of option data uniquely expresses a composition formula (or a chemical formula), which indicates a composition of a compound. The above-described search area includes all these combinations, and the above-described data points can be regarded as indicating one combination or one composition of a compound.

[Predictor Database (DB) 2]

The predictor database 2 stores and manages at least one predictor for predicting composition formulae (or chemical formulae) of materials and property values of the materials. The predictor database 2 is, for example, a nonvolatile memory. The predictor is a program based on a certain calculation algorithm including machine learning and outputs a predicted value (hereinafter referred to as a "predicted property value") of a property value of a material having each of compositions in a search area determined on the basis of an input of variables and option data. The predictor database 2 may be capable of outputting, with one predictor, various predicted property values such as values of an electrochemical property, values of a thermochemical property, and X-ray diffraction patterns or may store and manage predictors and change a predictor to be used depending on a property to be predicted. For example, a predictor A that predicts values of an electrochemical property, a predictor B that predicts values of a thermochemical property, and a predictor C that predicts X-ray diffraction patterns are provided. In this case, complication of calculation algorithms of predictors can be suppressed, and prediction accuracy and processing speed for each of properties improve. This is just an example, and any predictor(s) may be used insofar as a desired predicted property value can be output for a composition of a material corresponding to input variables and option data.

The predictor may be an arithmetic device that performs arithmetic operations in accordance with a calculation model simulating neural networks of living things. The arithmetic device may include an input layer, hidden layers, and an output layer. The input layer may include units Ui1, Ui2, and so on. The hidden layers may each include units. The output layer may include units Uo1, Uo2, and so on.

When input data X = [x1, x2, ...] is input to the input layer, the units included in the hidden layers and the output layer perform arithmetic operations using weights, and the output layer outputs output data Y = [y1, y2, ...].

The Ui1, Ui2, and so on included in the input layer and the input data x1, x2, and so on are in one-to-one correspondence. The units Uo1, Uo2, and so on included in the output layer and the output data y1, y2, and so on are in one-to-one correspondence. The output layer may include only one unit, instead.

The input data X = [x1, x2, ...] for the predictor may be [a chemical symbol substituted for M3, a chemical symbol substituted for M3', a chemical symbol substituted for M4, a chemical symbol substituted for M4', a value substituted for a, a value substituted for b, a value substituted for x, a value substituted for y], and the output data Y = [y1, y2, ...] for the predictor may be [predetermined property values of a compound having a chemical formula defined by input data]. The predetermined property values may be band gap values. The input data may also include one or more pieces of information other than the chemical symbol substituted for M3, the chemical symbol substituted for M3', the chemical symbol substituted for M4, the chemical symbol substituted for M4', the value substituted for a, the value substituted for b, the value substituted for x, and the value substituted for y. The one or more pieces of information included in the input data may be, for example, descriptors generated by quantifying atomic radii and electronegativity or crystal structure of constituent elements of a material.

The predictor may optimize the weights through backpropagation.

Training data used for the backpropagation may be generated using chemical symbols and values based on chemical formulae of compounds to be produced and predetermined property values of produced compounds.

When a chemical formula (may also be referred to as a "target composition") of a compound to be produced is $Li_{1.7}Al_{0.04}Ga_{0.06}Ti_{0.2}Zr_{0.8}O_3$ and a measured band gap of a produced compound is eV, training data may be:

$$[X01, Y01] = [x1, x2, ..., y1, y2, ...] = [3, 13, 22, 40, 0.1, 0.0, 0.6, 0.8, e],$$

where 3, 13, 22, and 40 are atomic numbers of Li, Al, Ti, and Zr, respectively.

That is, the predictor of the predictor database 2 predicts a property value corresponding to a composition of a compound expressed by each of the above combinations. The prediction of a property value can also be regarded as a search for a property value of a material.

[Experiment Database 3]

The experiment database 3 stores and manages composition formulae (or chemical formulae) of materials, compound identification information, and experiment values (hereinafter referred to as "experimental property values") of properties to be searched for. When search targets include processing conditions, the experiment database 3 also stores in advance experiment process information indicating processing conditions used in experiments.

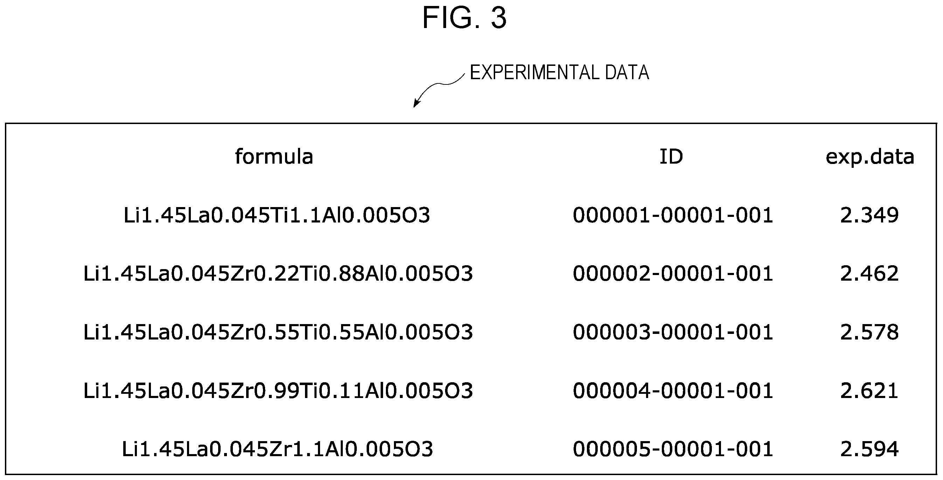

Fig. 3 illustrates an example of experimental data stored in the experiment database 3. The compound identification information is so-called IDs and may be information of any type with which compounds can be identified, such as names, signs, or numbers. In Fig. 3, for example, a material expressed by a chemical formula (formula) $Li_{1.45}La_{0.045}Ti_{1.1}Al_{0.005}O_3$ is registered with an ID of 000001-00001-001, and an experimental property value (exp.data) is 2.349. The chemical formula is defined by (M3, M3', M4, M4', a, b, x, y) = (La, Al, Ti, Zr, 0.05, 0.1, 0.1, 0). The ID of 000001-00001-001 illustrated in Fig. 3 includes three levels of numbers (000001, 00001, and 001). For example, IDs are registered such that first-level numbers "000001" to "000005" are assigned to five chemical formulae whose composition ranges of the elements Zr and Ti included in a material are different from each other. By giving an ID with which a composition formula (or a chemical formula) of a material can be identified, users can easily manage the experiment database 3. A type of experimental property value illustrated in Fig. 3 is selected as desired in accordance with a material. For example, an experimental property value for a battery material is a degree of conductivity, and an experimental property value for a thermoelectric conversion material is a thermoelectric conversion performance index.

[Property Value Obtaining Unit 101]

The property value obtaining unit 101 obtains, from the predictor database 2, predicted property values corresponding to a search area obtained from the input unit 1, obtains experimental property values from the experiment database 3, and outputs the predicted property values and the experimental property values to the image generation unit 102.

[Image Generation Unit 102]

The image generation unit 102 generates a first image in which experimental property values are superimposed upon predicted property values obtained from the property value obtaining unit 101 and outputs the first image to the image arrangement determination unit 106. When the first image is to be reduced, the image generation unit 102 also outputs the first image to the reduced image generation unit 103.

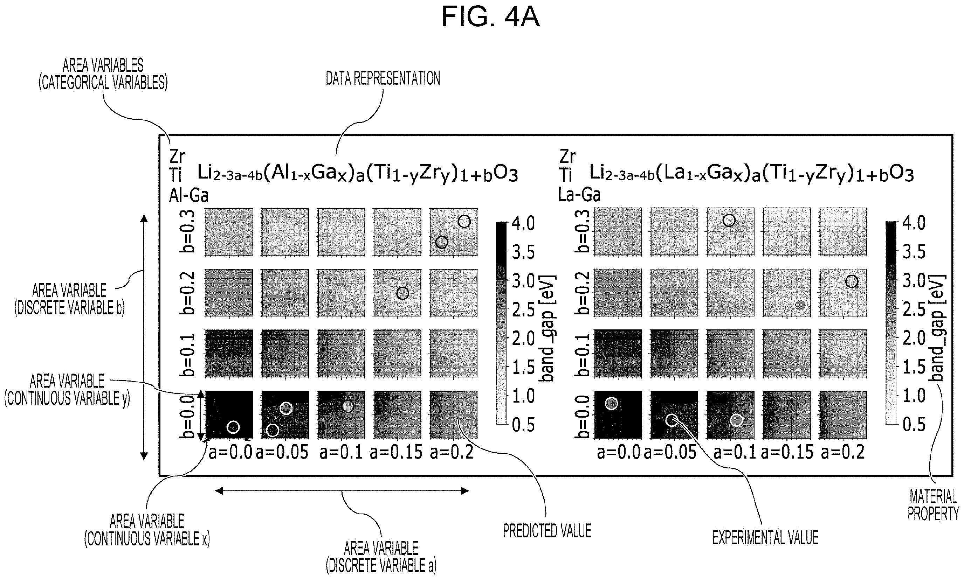

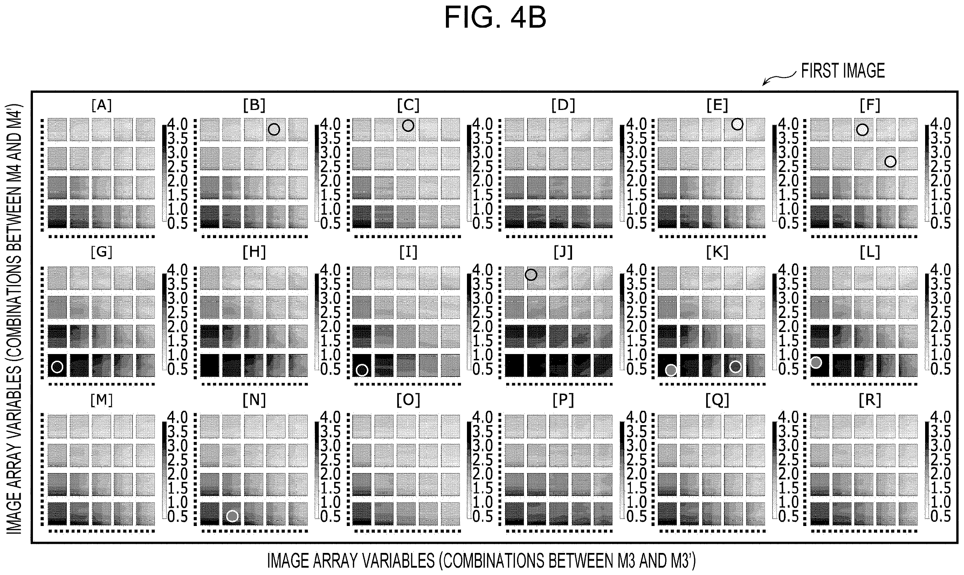

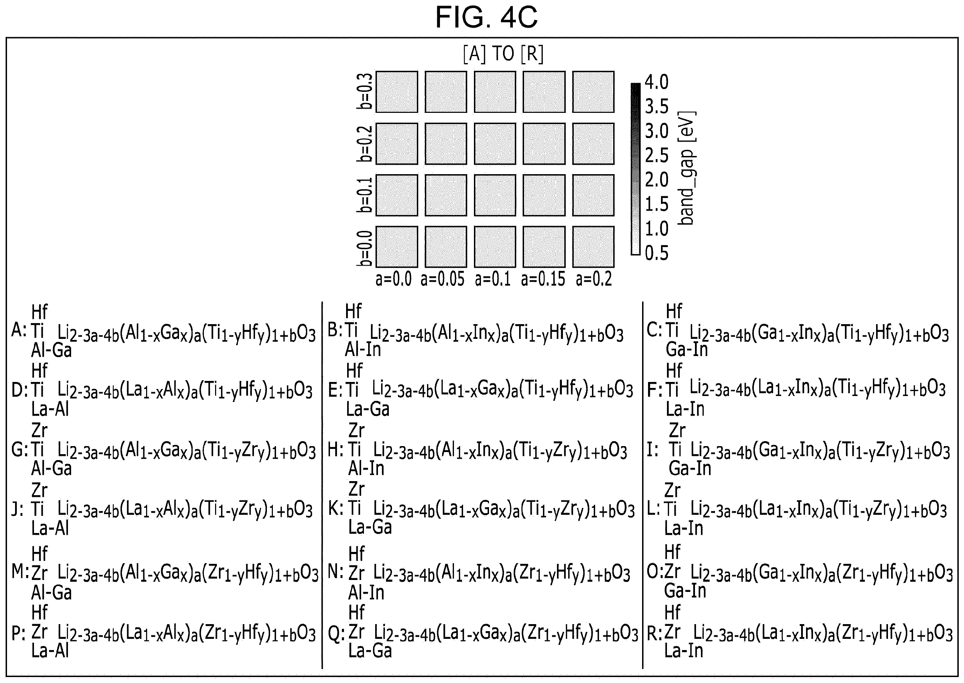

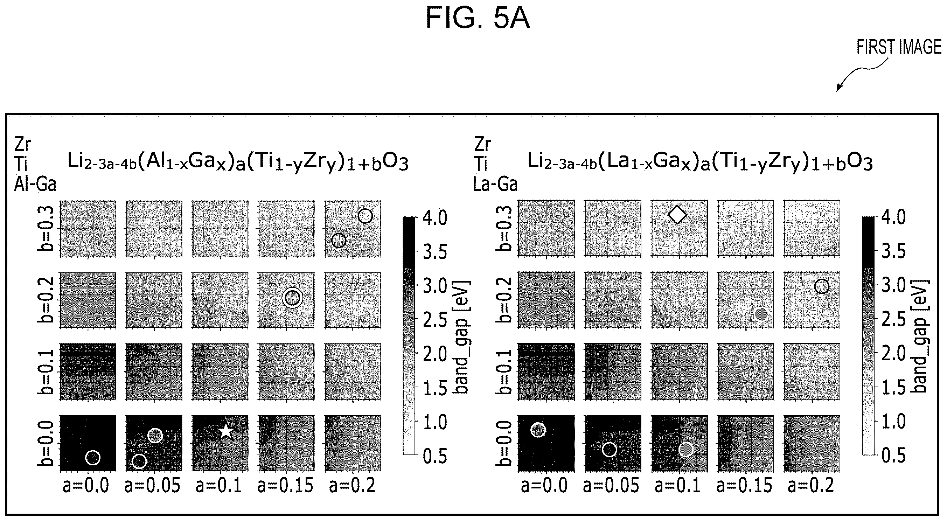

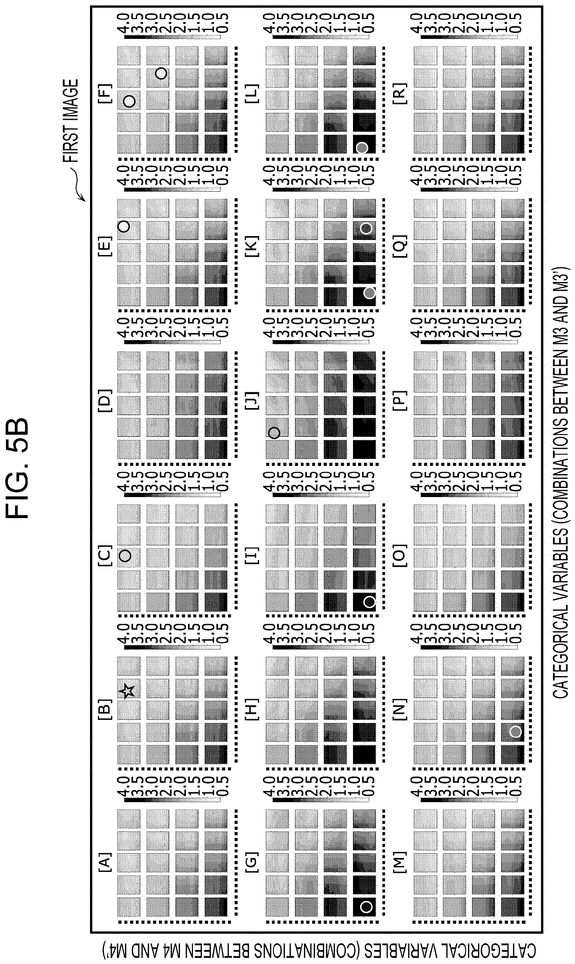

Figs. 4A, 4B, 5A, and 5B are examples of a generated first image. Fig. 4C illustrates a legend and the like of the first images illustrated in Figs. 4B and 5B. That is, [A] to [R] in Figs. 4B and 5B are chemical formulae associated with A to R, respectively, in Fig. 4C. Fig. 4C also illustrates a legend of each of image maps, which will be described later, illustrated in Figs. 4B and 5B.

The chemical formula corresponding to A in Fig. 4C may be indicated in a part A in Figs. 4B and 5B, ..., and the chemical formula corresponding to R in Fig. 4C may be indicated in a part R in Figs. 4B and 5B. Because the chemical formulae become too small to recognize if the chemical formulae are written in the part A in Figs. 4B and 5B, ..., and the part R in Figs. 4B and 5B, Figs. 4B, 5B, and 4C are provided as shown.

Figs. 4A and 4B illustrate an image map for each of combinations of option data regarding the four categorical variables (M3, M3', M4, and M4'). The combinations will also be referred to as combinations of the categorical variables hereinafter. A set of image maps corresponding to all the combinations of the categorical variables will also be referred to as a map. Each image map includes an array (or a matrix) of image element maps. Two axes of each image element map represent the continuous variables x and y, and two axes of the array (i.e., the image map) of the image element maps represent the discrete variables a and b. How the variables are assigned is not limited to this. The predicted property values may be displayed as they are for each of all the obtained combinations of option data or may be interpolated and displayed for combinations that have not been obtained (i.e., data points that have not been obtained). The predicted property values are indicated by colors or shades of color.

Here, Fig. 4A illustrates image maps corresponding to two combinations of the categorical variables, and Fig. 4B illustrates image maps corresponding to 18 combinations of the categorical variables. The number of image element maps included in a map in the present disclosure is determined on the basis of the number of combinations of option data regarding each of the two variables assigned to the two axes of the array of the image element maps and the number of combinations of option data regarding each of the four categorical variables (i.e., elements). That is, the number of image element maps increases as a composition range of materials stored in and managed by the predictor database 2 increases and the number of combinations of constituent elements of materials increases. It is therefore important for the user to display image element maps such that the user can easily understand the image element maps visually.

A case where the user searches for predicted property values for the composition expressed by the chemical formula $Li_{2-3a-4b}(M3_{1-x}M3'_x)_a(M4_{1-y}M4'_y)_{1+b}O_3$ illustrated in Fig. 2 will be described with reference to Fig. 4A as an example of display. First, the image generation unit 102 assigns the continuous variables x and y to the two axes of image element maps. The continuous variables x and y are variables including option data that varies from a minimum value 0.0 to a maximum value 1.0 with a step width of 0.1, and the number of combinations of each of x and y is 11. Although the image element maps in the present disclosure, therefore, are squares, shapes of image element maps depend on how axes of the image element maps are assigned, and may be triangles or other shapes, instead. Predicted property values of compounds defined by the variable x and the variable y are indicated on the image element maps by colors or shades of color at positions indicated by the variable x and the variable y. An example of the predicted property values in the present disclosure is band gaps, but the predicted property values are not limited to this.

The image generation unit 102 then assigns the discrete variables a and b to the two axes of the array of the image element maps. The discrete variable a includes five pieces of option data, namely 0.0, 0.05, 0.1, 0.15, and 0.2, and the discrete variable b includes four pieces of option data, namely 0.0, 0.1, 0.2, and 0.3. The number of image element maps for each of the combinations of the categorical variables, therefore, is 5 x 4 = 20 in Fig. 4A. In the above example, the continuous variables x and y and the discrete variables a and b are compared with each other, and the continuous variables x and y, which have more combinations, are assigned to the axes of the image element maps and the discrete variables a and b, which have fewer combinations, are assigned to the axes of the array of the image element maps in order to facilitate visual understanding by the user. The user may assign the variables as desired, or the variables may be automatically assigned after the number of combinations is calculated.

Furthermore, with respect to the categorical variables, there are six combinations of option data, namely the elements La, Al, Ga, and In, selected for the variable names M3 and M3', and there are three combinations of option data, namely the elements Ti, Zr, and Hf, selected for the variable names M4 and M4'. As a result, a total of 18 image maps, that is, six in a horizontal direction and three in a vertical direction, each of which includes 20 image element maps corresponding to the combinations of the categorical variables (a total of $20 \times 18 = 360$ image element maps), are illustrated in Fig. 4B. The above map consists of the 18 image maps. Fig. 4A illustrates two of the 18 image maps.

As illustrated in Figs. 4A and 4B, the image generation unit 102 displays experimental property values as, for example, marks such as circles. Colors or shades of color of the marks such as circles, for example, indicate the experimental property values. That is, the image generation unit 102 superimposes marks having colors or shades of color corresponding to experimental property values of compounds produced as a result of experiments on the map at positions corresponding to the above-described combinations indicating composition of the compounds. The superimposition of the marks is also called superimposition of the experimental property values. An example of experimental property values in the present disclosure is band gaps of compounds obtained as a result of experiments, but is not limited to this. An experimental property value may be data written on a document or a book, instead. On the map according to the present embodiment, a relationship between experimental property values or predicted property values and colors or shades of color may be unified. As a result, when an experimental property value deviates from a predicted property value, a difference between a color or a shade of color of a mark corresponding to the experimental property value and a surrounding color or shade of color stands out. Consequently, it becomes easy to visually understand the deviation of the experimental property value from the predicted property value. In other words, when a color or a shade of color of a mark corresponding to an experimental property value and a color or a shade of color corresponding to a predicted property value are visually the same, the user can immediately understand visually that the experimental property value and the predicted property value are substantially the same.

As for a method for displaying experimental property values, too, measures for making it easier for the user to visually understand the experimental property values may be taken. As illustrated in Figs. 5A and 5B, for example, the image generation unit 102 may highlight newly added experimental property values, experimental property values whose properties meet a certain criterion, experimental property values selected by the user, or the like. For example, the image generation unit 102 may highlight an experimental property value by adding a border to a dot, making a dot flash, or changing a dot to a diamond or a star. When newly added experimental property values are highlighted and the experiment database 3 is shared by different users, the users can keep track of progress of daily experiments because tens or hundreds of pieces of experimental data are expected to be newly added every day.

The experimental property values whose properties meet a certain criterion, the experimental property values selected by the user, and the like may be specified by the user or specified in advance.

By highlighting experimental property values (i.e., marks indicating experimental property values) on the basis of certain specifications especially when the number of image element maps over which the experimental property values are scattered exceeds 100 as illustrated in Fig. 5B, the user can easily understand the experimental property values visually. As additional measures for making it easier the user to understand experimental property values visually, an image indicating a data representation, an image of a color bar, an image indicating values of area variables, or the like may be displayed above the image maps.

The first image in the present disclosure is thus a map including the above-described image maps upon which marks indicating experimental property values, such as dots, stars, or circles, are superimposed.

[Reduced Image Generation Unit 103]

The reduced image generation unit 103 generates a reduced image of a first image (hereinafter referred to as a reduced first image) generated by the image generation unit 102 and outputs the reduced first image to the image arrangement determination unit 106.

[Image Arrangement Determination Unit 106]

The image arrangement determination unit 106 obtains a first image from the image generation unit 102, determines arrangement, and outputs the first image to the display unit 6. When the image arrangement determination unit 106 obtains a compound data image from the data image generation unit 105, which will be described later, the image arrangement determination unit 106 generates a second image by determining arrangement such that the obtained first image and the obtained compound data image are displayed in one screen and outputs the second image to the display unit 6. When a first image is reduced, the image arrangement determination unit 106 obtains a reduced first image from the reduced image generation unit 103, generates a second image by determining arrangement such that the obtained reduced first image and the obtained compound data image are displayed in one screen, and outputs the second image to the display unit 6.

[Display Unit 6]

The display unit 6 is a liquid crystal display, a plasma display, or an organic EL (electroluminescence) display, for example, but is not limited to this. The display unit 6 displays a result (i.e., a first image or a second image) obtained from the image arrangement determination unit 106.
The display unit 6 is, for example, a display unit included in an information terminal, an information terminal into which an electronic experiment note is introduced, or the like. Alternatively, the display unit 6 may be one included in a synthesis apparatus used to synthesize materials or compounds, a reaction apparatus used to react materials or compounds, an analysis apparatus used to analyze compounds, an evaluation apparatus used to evaluate compounds, or the like.
Details of components used in an operation after the display unit 6 displays a first image and the user selects an experimental property value included in the first image will be described hereinafter.

[Selection Reception Unit 4]

When the user selects an experimental property value included in a first image displayed on a screen of the display unit 6, the selection reception unit 4 receives the user input and outputs the user input to the data obtaining unit 104. The user input may be a click on an experiment point, which is a mark indicating the experimental property value, or a drag on a map.
That is, the selection reception unit 4 selects an experimental property value, for example, from one or more experimental property values included in a first image in accordance with an input operation performed by the user and outputs the selected experimental property value to the data obtaining unit 104. The selection reception unit 4 may be, for example, a keyboard, a touch sensor, a touchpad, a mouse, or the like.

[Data Obtaining Unit 104]

The data obtaining unit 104 obtains, from the experiment database 3, compound identification information given to an experimental property value selected by the user using the selection reception unit 4. The data obtaining unit 104 also obtains, from the compound information database 5, compound information data including at least one of compound composition data or compound graph data corresponding to the compound identification information and outputs the compound information data to the data image generation unit 105. The expression "at least one of A or B" in the specification, the claims, the abstract, and the drawings of the present application may mean "A, B, or both A and B".

[Compound Information Database 5]

The compound information database 5 stores and manages compound information data corresponding to experimental property values managed by the experiment database 3.

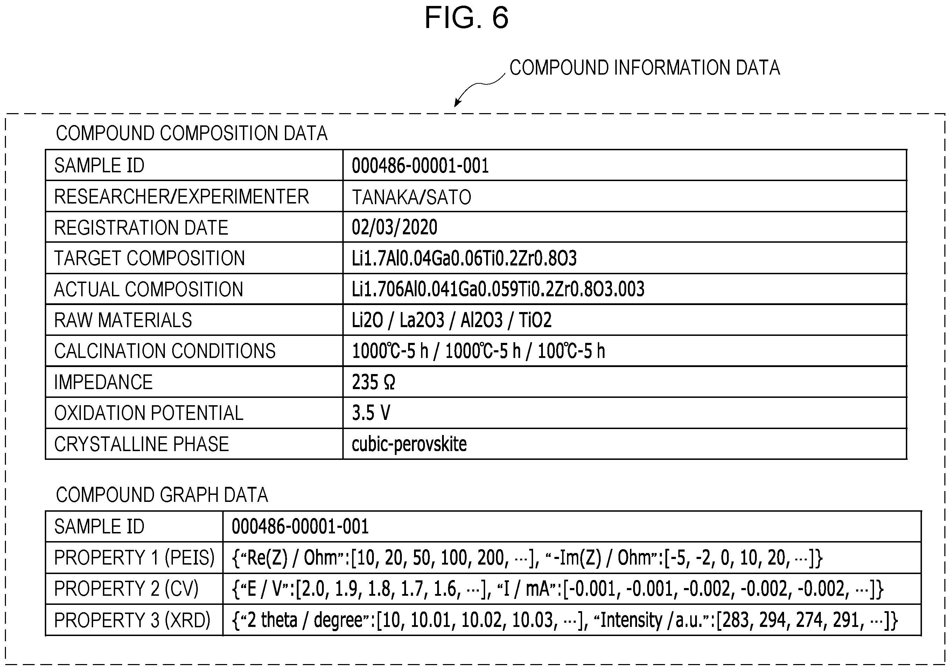

Fig. 6 illustrates an example of the compound information data. The compound information data illustrated in Fig. 6 includes compound composition data and compound graph data. The compound composition data includes compound identification information (also called a sample ID or a compound ID) and supplementary information regarding a compound. The supplementary information is so-called metadata and includes, for example, names of a researcher and an experimenter, a data registration date, composition information, processing conditions, and property value information. The composition information indicates a target composition. The target composition is also called a target composition. The processing conditions include an actual composition, raw materials, an experiment procedure, calcination conditions, a shape and a size of a crucible, a type of apparatus, and a serial number of an apparatus. The property value information includes impedance, an oxidation potential, and a crystalline phase. That is, the compound composition data indicates that a compound having an actual composition has been produced by producing a compound under processing conditions in order to produce a compound having a target composition and property value information obtained through an experiment conducted on the produced compound. The actual composition is evaluated, for example, using energy dispersive X-ray spectroscopy, X-ray photoelectron spectroscopy, inductively coupled plasma atomic emission spectroscopy, or the like. The experimental property values and the predicted property values indicated on the maps illustrated in Figs. 4A, 4B, 5A, and 5B are associated with target compositions, but may be associated with actual compositions, instead.

The experimental property values indicated by the experimental data stored in the experiment database 3 are values obtained or calculated on the basis of, for example, the property value information included in the compound information data. A specific example of an experimental property value is a band gap obtained using impedance and an oxidation potential of property value information or the like.

The compound composition data illustrated in Fig. 6 is an example, and compound composition data may include at least compound identification information (sample ID) and a target composition. Furthermore, when the compound composition data includes names of a researcher and an experimenter and a registration date, credibility of experimental data improves. When the compound composition data includes processing conditions and property value information, a more specific search for material properties and improvement of a value of experimental data are expected.

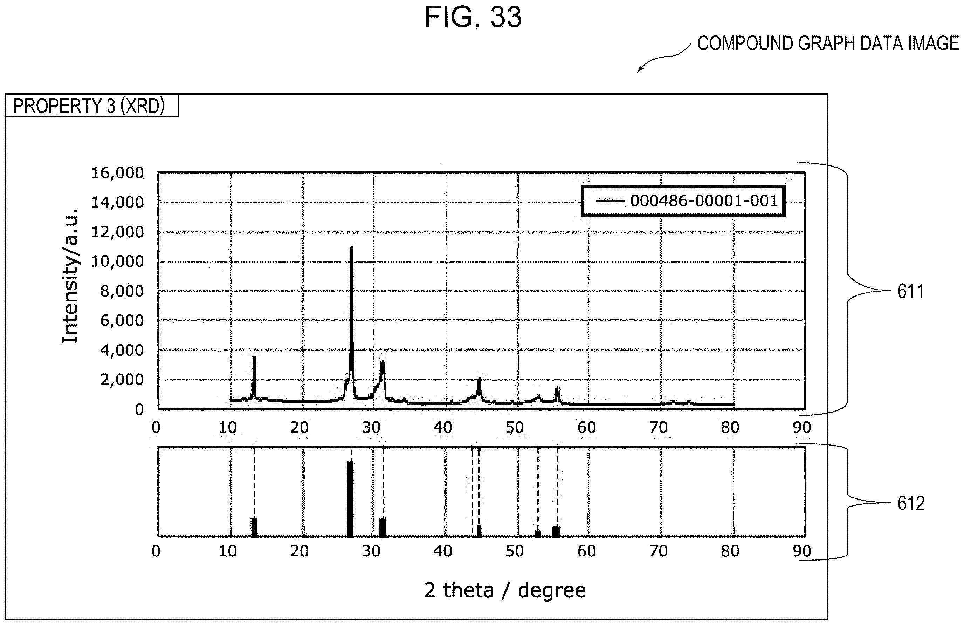

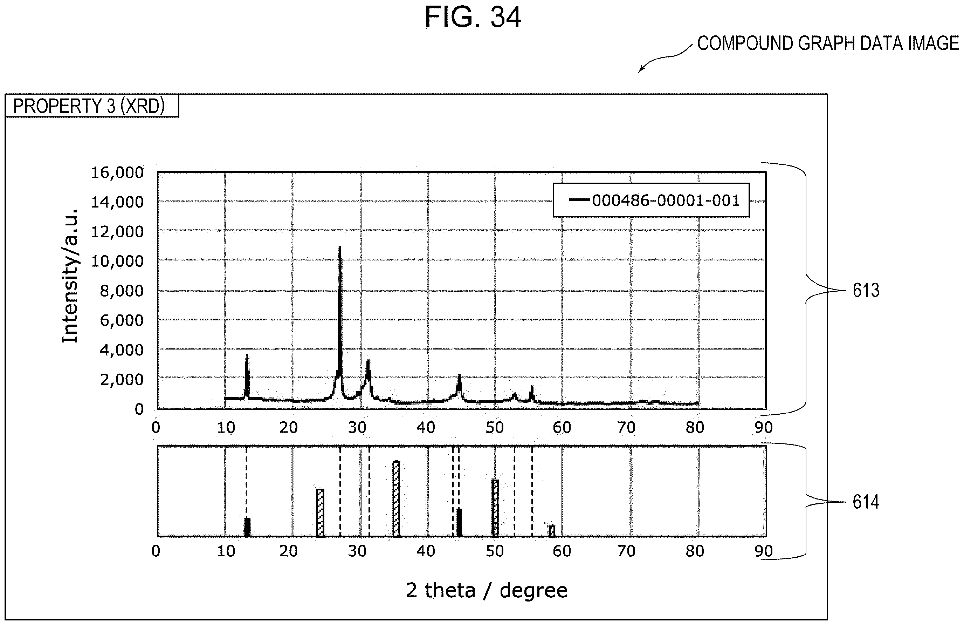

The compound graph data includes compound identification information and measurement data regarding a compound. The measurement data indicates, for example, impedance obtained through potentio electrochemical impedance spectroscopy (PEIS), a voltammogram obtained through cyclic voltammetry (CV), a diffraction pattern obtained through X-ray diffraction (XRD), and the like. X-ray diffraction (XRD) will be described later (refer to description with reference to Figs. 33 and 34). The number of properties indicated by the measurement data is not limited to one, but may be two or more, instead. In the present disclosure, PEIS, CV, and XRD are denoted by property 1, property 2, and property 3, respectively. The compound composition data may include experimental property values such as band gaps obtained from impedance or the like.

[Data Image Generation Unit 105]

The data image generation unit 105 generates a compound data image on the basis of compound information data obtained from the data obtaining unit 104 and outputs the compound data image to the image arrangement determination unit 106. The compound data image indicates the compound information data as an image.

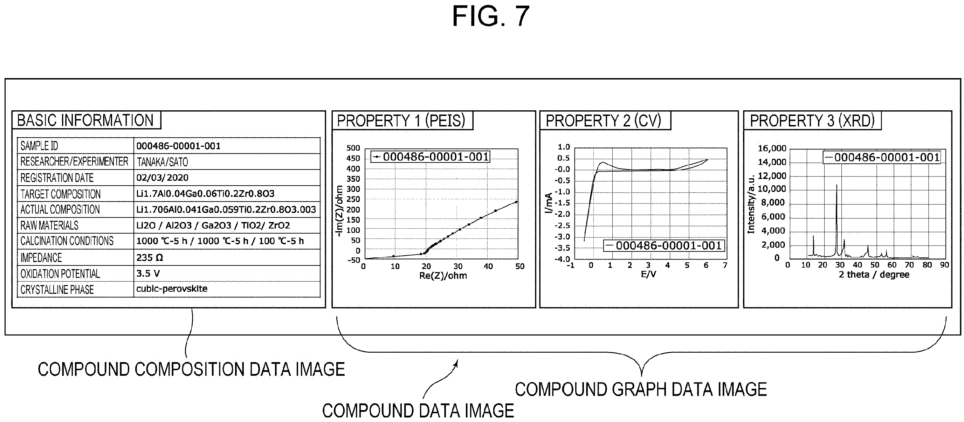

Fig. 7 illustrates an example of a generated compound data image. The compound data image includes a compound composition data image and compound graph data images corresponding to an experimental property value selected by the user.

Impedance, an oxidation potential, and a crystalline phase included in the compound composition data may be information read by a person from the compound graph data. For example, an experimenter may read, from a graph of property 1 (i.e., PEIS) of the compound graph data, a value on a horizontal axis corresponding to a value of 0 on a vertical axis as impedance and register the impedance to the compound composition data. The experimenter may read, from a graph of property 2 (i.e., CV) of the compound graph data, a potential on a horizontal axis at which a current value on a vertical axis rises from 0 as an oxidation potential and register the oxidation potential to the compound composition data. The experimenter may read, from a pattern in a graph of property 3 (i.e., XRD) of the compound graph data, a crystalline phase of a compound and register the crystalline phase to the compound composition data.

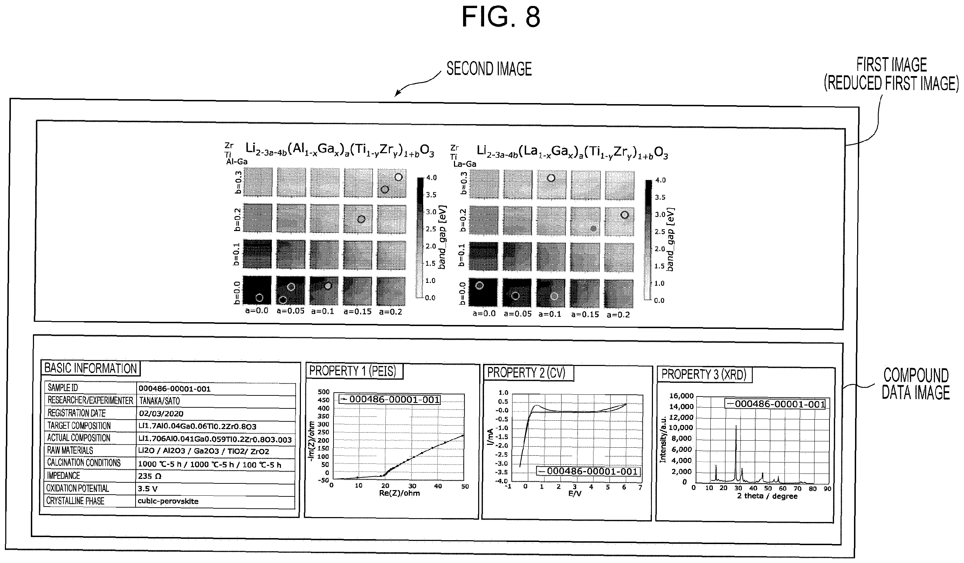

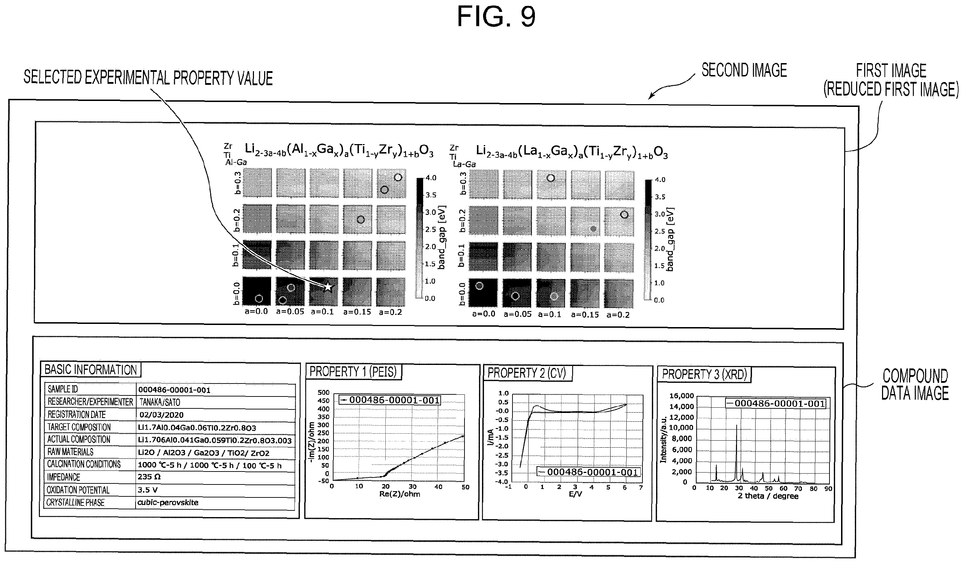

Figs. 8 and 9 illustrate examples of a displayed second image. Although a first image (or a reduced first image) is displayed above a compound data image in Figs. 8 and 9, the first image and the compound data image may be displayed in any manner insofar as the first image and the compound data image are displayed in one screen. As illustrated in Fig. 9, a selected experimental property value may be highlighted in the first image or the reduced first image. In Fig. 9, the experimental property value is displayed as a star as an example of highlighting. When highlighting is performed, unspecified users can easily understand a correspondence between displayed compound information data and a selected experimental property value visually. That is, even when a user who has not selected an experimental property value views a second image, the user can immediately find an experimental property value corresponding to compound information data displayed in a second image.

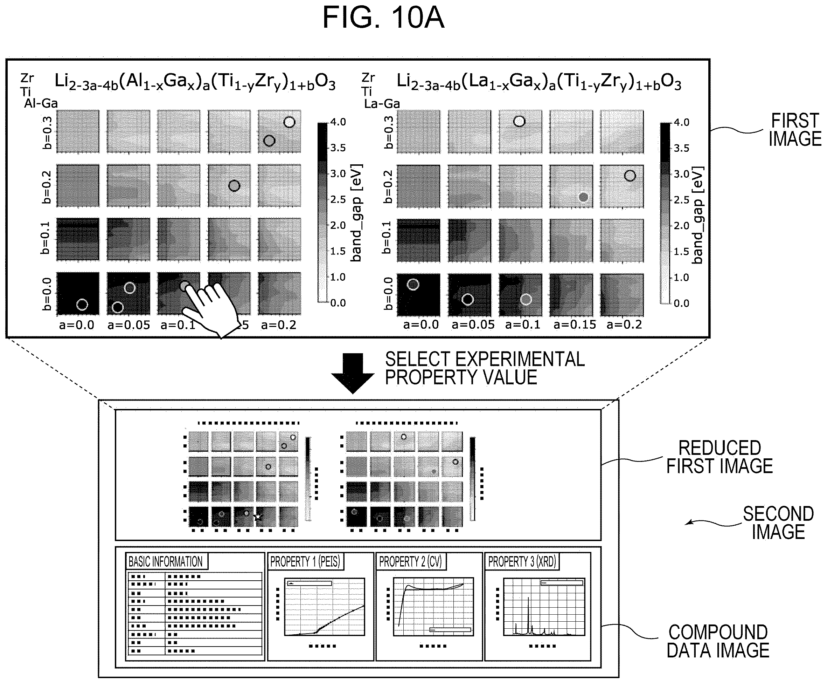

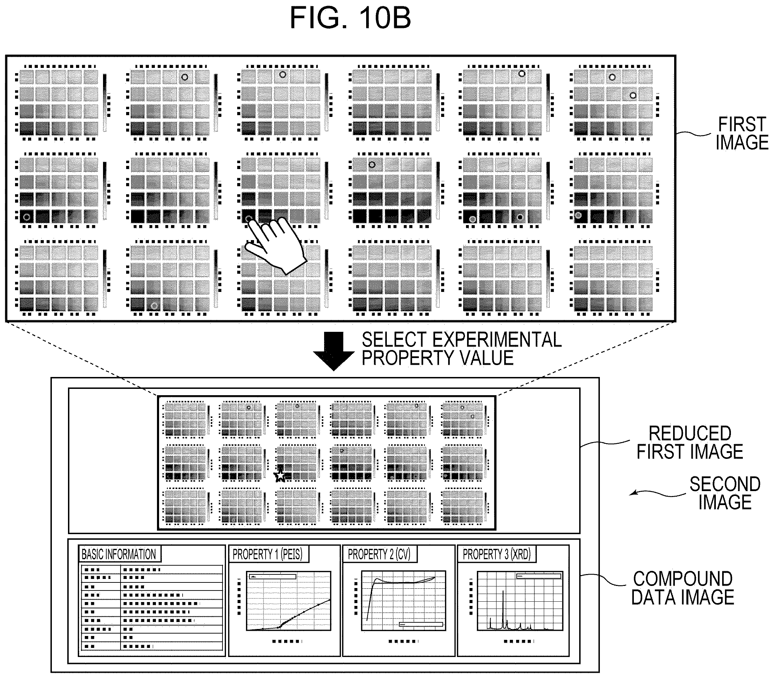

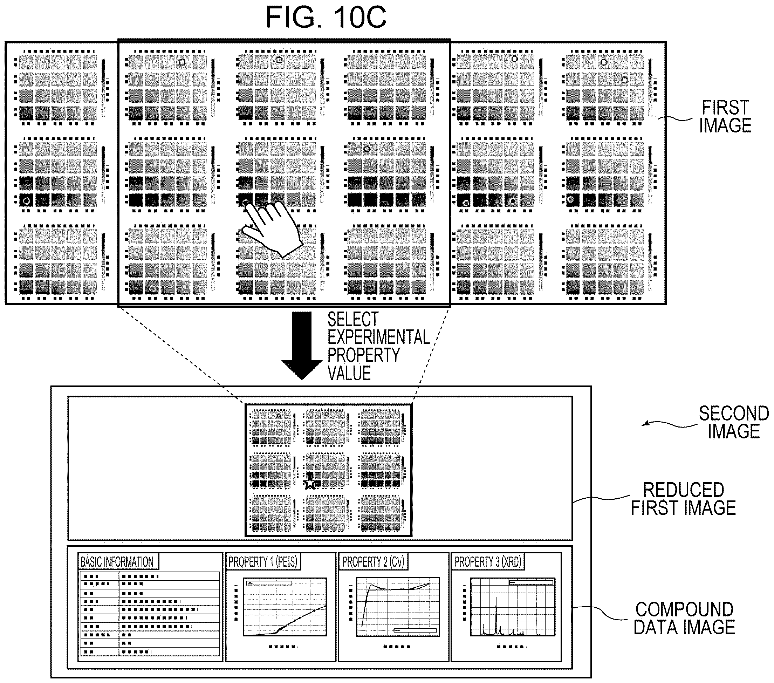

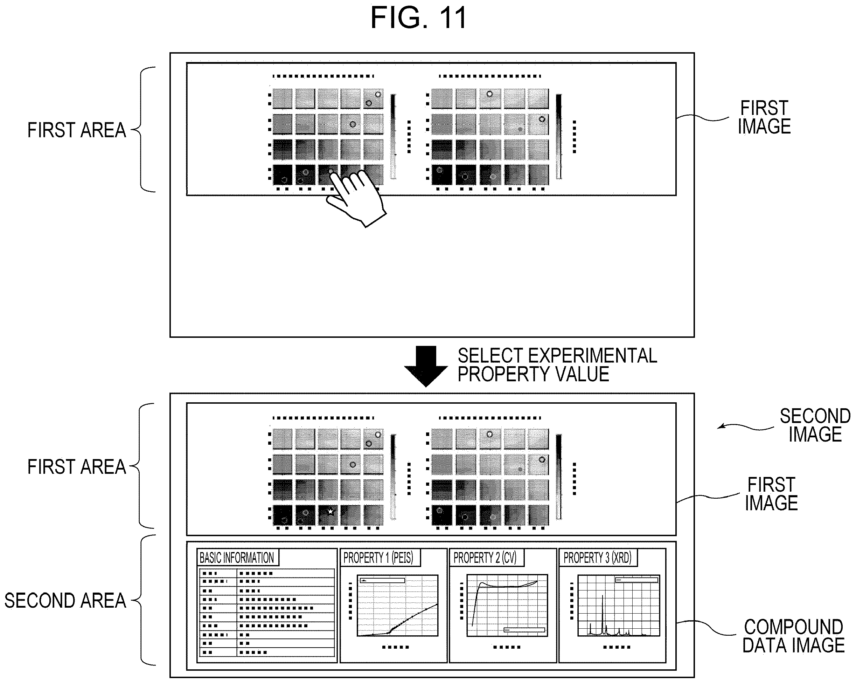

Figs. 10A, 10B, 10C, and 11 illustrate examples of an image indicating transition of a result displayed on the display unit 6.

In Figs. 10A, 10B, and 10C, a first image is displayed first, and after the user selects an experimental property value, a second image in which a reduced first image, which is obtained by reducing the first image, and a compound data image are displayed in one screen, is displayed. A second image including a compound data image is thus dynamically displayed by selecting an experimental property value in a first image. As illustrated in Figs. 10A and 10B, by reducing a first image after the user selects an experimental property value, operability for the user at a time when the user selects an experimental property value is expected to improve. Especially when the first image is that in the example illustrated in Fig. 4B, that is, when the number of image element maps exceeds 100, it becomes easier for the user to discuss while checking surrounding image maps when selecting an experimental property value as illustrated in Fig. 10B.

When the first image is that in the example illustrated in Fig. 4B, on the other hand, a reduced first image, which is obtained by reducing the entirety of a first image, is expected to be difficult for the user to view. As illustrated in Fig. 10C, therefore, an area around an image map of categorical variables including an experimental property value selected by the user may be selected and reduced. That is, a part of a map is selected and reduced. In this case, the user can easily understand a displayed second image visually compared to the case illustrated in Fig. 10B.

In Fig. 11, a first image is displayed in an upper part (i.e., a first area) of a screen displayed by the display unit 6 first, and after the user selects an experimental property value, a second image is displayed by displaying a compound data image in a lower part (i.e., a second area) of the screen. That is, when the first image does not occupy the entirety of the screen of the display unit 6 from a beginning, the first image may be displayed without being reduced. Display size of the first image may be determined on the basis of screen size of the display unit 6 or the number of image maps included in the first image. Since a step of reducing the first image can be omitted in Fig. 11, processing speed is expected to improve. A change in a result displayed on the display unit 6 when the user selects an experimental property value and the second image is displayed is reduced to a minimum, and it is also expected to make it easier for the user to understand the result dynamically.

<Summary of Embodiment 1-1>

As described above, a property display method according to an aspect of embodiment 1-1 includes processing in the following first to seventh steps. In the first step, a first obtaining unit obtains variables for determining composition of compounds and pieces of option data indicating possible values or elements of each of the variables. The first obtaining unit is a component included in the property value obtaining unit 101 of the property display apparatus 100, for example, and obtains the variables and the pieces of option data from the input unit 1 as a search area.

In the second step, a second obtaining unit determines the compositions of the compounds by selecting one of the pieces of option data for each of the variables and obtains predicted property values of the compounds corresponding to the determined compositions of the compounds. The second obtaining unit is a component included in the property value obtaining unit 101 of the property display apparatus 100, for example, and obtains the predicted property values by inputting combinations of the piece of option data to the predictor database 2 and obtaining a result output from the predictor database 2.

In the third step, a third obtaining unit obtains experimental property values of the compounds corresponding to the determined compositions of the compounds. The third obtaining unit is a component included in the property value obtaining unit 101 of the property display apparatus 100, for example, and obtains, from the experiment database 3, experimental property values held in association with the combinations.

In the fourth step, a first image processing unit generates a map indicating the predicted property values of the compounds corresponding to the determined compositions at positions corresponding to the compositions of the compounds, generates a first image by superimposing the obtained experimental property values upon the map at positions corresponding to the compositions of the compounds according to the experimental property values, and outputs the first image to the display unit 6. The first image processing unit is, for example, a component including the image generation unit 102 and the image arrangement determination unit 106 of the property display apparatus 100.

In the fifth step, a fourth obtaining unit obtains compound identification information corresponding to a selected one of the experimental property values on the map. The fourth obtaining unit is a component included in the data obtaining unit 104 of the property display apparatus 100, for example, and obtains, from the experiment database 3, compound identification information held in association with the experimental property value.

In the sixth step, a fifth obtaining unit obtains compound information data associated with the compound identification information and relating to the selected experimental property value. The fifth obtaining unit is a component included in the data obtaining unit 104 of the property display apparatus 100 and obtains, from the compound information database 5, compound information data held in association with the compound identification information.

In the seventh step, a second image processing unit generates a second image including a compound data image indicating the compound information data and the first image and outputs the second image to the display unit 6. The second image processing unit is, for example, a component including the data image generation unit 105 and the image arrangement determination unit 106 of the property display apparatus 100.

As a result, the first image in which the experimental property values are superimposed upon the map indicating the predicted property value of each of the combinations of the pieces of option data, that is, the predicted property value of each of the compositions of the compounds is generated and displayed. When the user of the property display apparatus 100 inputs, for example, many variables and many pieces of option data to be substituted for each of the variables to the property display apparatus 100 using the input unit 1, therefore, a map indicating a huge amount of information can be displayed. That is, on the map, a predicted property value of each of many possible compositions (i.e., composition formulae or chemical formulae) of compounds is associated with the composition, and an experimental property value of each of at least one composition is associated with the composition. The user, therefore, can easily understand the predicted property values and the experimental property values of all the compositions of the compounds on the map.

Furthermore, in the property display method according to the aspect of the present embodiment, when the user selects one of the experimental property values superimposed upon the map, the second image is generated and displayed. That is, a compound data image indicating compound information data relating to the selected experimental property value is displayed in the same screen as the first image. As a result, the user can display, through a simple input operation, a compound data image indicating compound information data relating to an experimental property value that the user is interested in. The user can easily visually understand the compound information data shown in the compound data image, the predicted property value of each of the compositions shown in the first image, and the selected experimental property value without switching the screen. Since the compound information data can be tracked and displayed with the property display method according to the aspect of the present embodiment, therefore, the experimental property value, the predicted property value, and the compound information data can be managed in a centralized manner. As a result, material development by the user can be appropriately supported.

The property display method according to the aspect of the present embodiment may further include a step in which the reduced image generation unit 103 generates a reduced first image, which is obtained by reducing the first image, before the second image is generated in the seventh step. In this case, in the seventh step, the image arrangement determination unit 106 generates a second image including the compound data image and the reduced first image and outputs the second image to the display unit 6.

Since the first image is reduced in order to generate the second image before the second image is generated, therefore, the first image can be displayed widely in the screen in the fourth step. As a result, visibility of the first image improves.

A screen in which the first image and the second image are displayed in the fourth and seventh steps, respectively, may include a first area and a second area. In the fourth step, the image arrangement determination unit 106 may output the first image disposed in the first area to the display unit. In the seventh step, the image arrangement determination unit 106 may generate the second image by disposing the first image in the first area and the compound data image in the second area and output the second image to the display unit 6.

As a result, since arrangement and size of the first image displayed in the fourth and seventh steps are the same, a processing load for the first image is reduced and processing speed improves. Furthermore, since the arrangement and the size of the first image remain the same even after a displayed image switches from the first image to the second image, the user's discomfort caused by the switching can be reduced.

The compound information data includes compound composition data and compound graph data relating to the experimental property value.

Since an image indicating these pieces of data is displayed as the compound data image, therefore, the user can easily understand, from various angles, various pieces of information regarding a compound relating to the selected experimental property value. When the compound composition data and the compound graph data are data used to obtain the experimental property value, raw data of the experimental property value can be easily understood.

A property display method according to another aspect of embodiment 1-1 includes step 1 of obtaining a map indicating a position corresponding to each of compositions of compounds, step 2 of obtaining an experimental property value of each of at least one composition of the compounds, step 3 of generating a first image by superimposing the experimental property values on the map at positions corresponding to the compositions corresponding to the obtained experimental property values and outputting the first image to a display unit, step 4 of obtaining compound information data corresponding to a selected one of the experimental property values on the map, and step 5 of generating a second image including an image indicating the compound information data and the first image and outputting the second image to the display. That is, in the property display method, predicted property values need not be obtained, and a map indicating predicted property values need not be generated. A so-called blank map may be obtained.

As a result, the experimental property values are displayed on the map at the positions corresponding to the compositions of the compounds according to the experimental property values. The user, therefore, can easily understand the experimental property values and the compositions of the compounds according to the experimental property values by viewing the map.

Furthermore, in the property display method according to the other aspect of the present embodiment, too, the second image is generated and displayed when the user selects, through an input operation, one of the experimental property values superimposed upon the map. That is, a compound data image indicating compound information data relating to the selected experimental property value is displayed in the same screen as the first image. As a result, the user can display, through a simple input operation, an image indicating compound information data relating to an experimental property value that the user is interested in. The user can easily visually recognize the compound information data shown in the image and the selected experimental property value shown in the first image without switching the screen. Furthermore, since the compound information data can be tracked and displayed, the experimental property value and the compound information data can be managed in a centralized manner. Consequently, material development by the user can be appropriately supported.

The map obtained in the step 1 may indicate a predicted property value of each of the compositions of the compounds at a position corresponding to the composition. In this case, the property display method according to the other aspect of the present embodiment further includes step 6 of obtaining compound identification information corresponding to the experimental property value selected on the map. In step 4, compound information data associated with the compound identification information and relating to the selected experimental property value is obtained. In step 5, a second image including an image indicating the compound information data and the first image is generated and output to the display unit. That is, in the property display method, predicted property values need not be obtained, and a map indicating predicted property values may be obtained.

As a result, the predicted property value of each of the compositions of the compounds is also indicated on the map at the position corresponding to the composition. Since the experimental property values are superimposed upon such a map, the user can easily compare a predicted property value and an experimental property value corresponding to a composition of the same compound.

The property display method according to the other aspect of the present embodiment may further include step 7 of obtaining a predicted property value of each of the compositions of the compounds. In this case, in step 1, the map is obtained by generating a map using the obtained predicted property values. That is, in the property display method, predicted property values need not be calculated using a calculation algorithm.

As a result, since the property display apparatus 100 obtains predicted property values and generates a map indicating the predicted property values even when a map indicating predicted property values cannot be obtained, a map indicating predicted property values need not be prepared in advance. Since a calculation algorithm is not used, a processing load can be reduced.

(Embodiment 1-2)

Embodiment 1-2 applies to a case where experimental property values have been selected. A display system according to the present embodiment has the same configuration as the display system 1000 according to embodiment 1-1, but performs processing different from that in embodiment 1-1 when experimental property values are selected. The data image generation unit 105 will be described in detail hereinafter.

[Data Image Generation Unit 105]

The data image generation unit 105 generates an image on the basis of compound information data obtained and selected by the data obtaining unit 104. Because pieces of compound information data are selected in the present embodiment, the data image generation unit 105 generates an image in which the pieces of compound information data are superimposed upon each other.

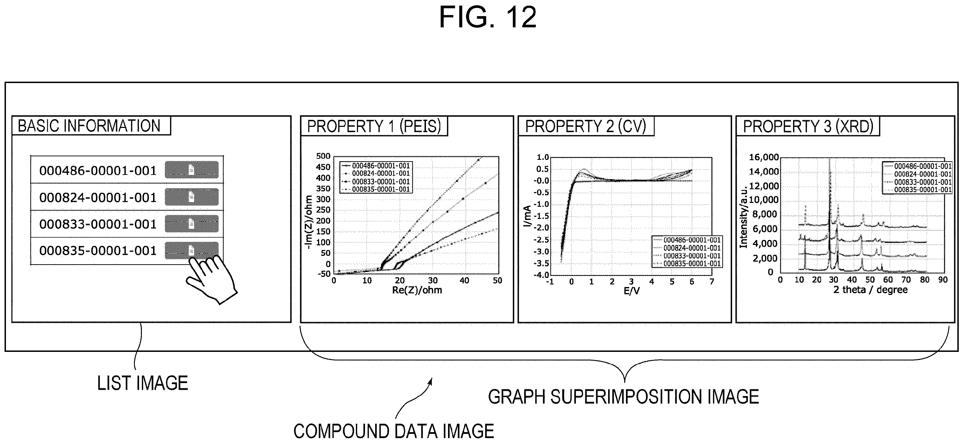

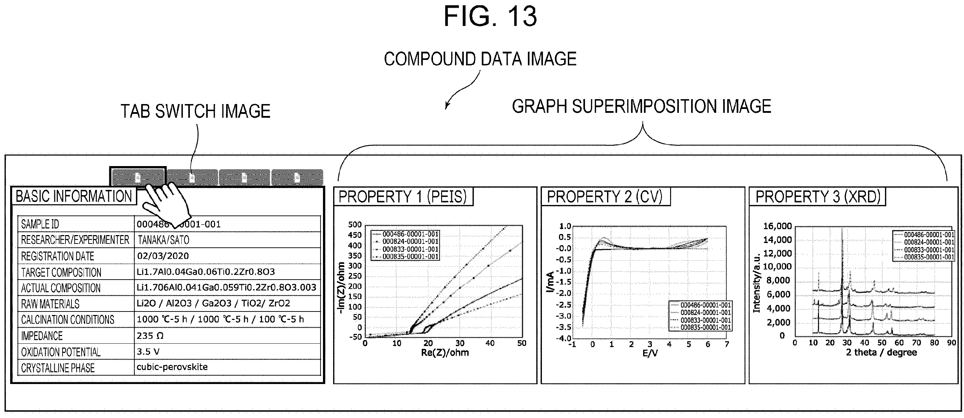

Figs. 12 and 13 illustrate examples of a compound data image at a time when experimental property values have been selected. As illustrated in Fig. 12, a compound data image includes a list image indicating compound identification information corresponding to each of the selected experimental property values instead of a compound composition data image. When the user selects a piece of the compound identification information in the list image, a compound composition data image corresponding to the selected piece of compound identification information is displayed in place of the list image. When such a list image is employed, that is, when compound identification information is displayed as a list, it is more likely that the user can visually understand selected experimental property values as the number of experimental property values selected increases.

The data image generation unit 105, for example, generates and displays a list image and switches the displayed list image to a compound composition data image. When experimental property values are selected, the data image generation unit 105 generates, as a compound graph data image as illustrated in Fig. 12, a graph superimposition image in which compound graph data corresponding to each of the selected experimental property values is superimposed upon each other. That is, the graph superimposition image is a compound graph data image indicating pieces of compound graph data. When the user selects one of pieces of compound identification information included in the list image, the data image generation unit 105 may switch the displayed graph superimposition image to a compound graph data image indicating compound graph data corresponding to the selected piece of compound identification information. Compound graph data corresponding to compound identification information is thus displayed by selecting the compound identification information. Alternatively, when one of pieces of compound identification information is selected, the data image generation unit 105 may change a color of a piece of compound graph data corresponding to the selected piece of compound identification information among pieces of compound graph data shown in a graph superimposition image to one different from that of the other pieces of compound graph data.

In Fig. 13, on the other hand, compound composition data is displayed in a tab format so that the compound composition data can be switched. That is, the data image generation unit 105 generates a tab switch image instead of a compound composition data image. The tab switch image includes tabs for switching between pieces of compound composition data. The tabs are associated with pieces of compound identification information, respectively, and the pieces of compound identification information correspond to the experimental property values selected by the user, respectively. When the user selects one of the tabs included in the tab switch image, therefore, the data image generation unit 105 switches the displayed tab switch image to a compound composition data image indicating compound composition data corresponding to the selected tab. Furthermore, as in the example illustrated in Fig. 12, the data image generation unit 105 may switch a graph superimposition image to a compound graph data image indicating compound graph data corresponding to the selected tab. Alternatively, as in the example illustrated in Fig. 12, when the user selects one of the tabs, the data image generation unit 105 may change a color of a piece of compound graph data corresponding to the selected tab among pieces of compound graph data shown in the graph superimposition image to one different from that of the other pieces of compound graph data. When such a tab format is employed, it is more likely that the user can visually understand selected experimental property values as the number of experimental property values selected decreases. In an example, therefore, a display format may be changed in accordance with the number of experimental property values selected, that is, for example, the tab format illustrated in Fig. 13 may be employed when the number of experimental property values selected is smaller than or equal to five, and the list format illustrated in Fig. 12 may be employed when the number of experimental property values selected is larger than or equal to six.

A method for displaying compound composition data is not limited to those illustrated in Figs. 12 and 13, and any format may be used insofar as pieces of information can be displayed in one screen.

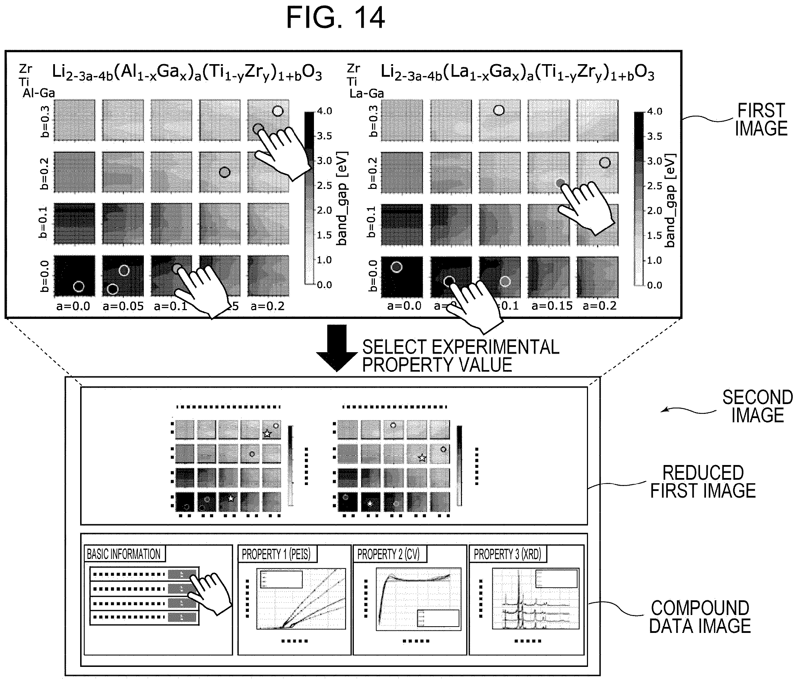

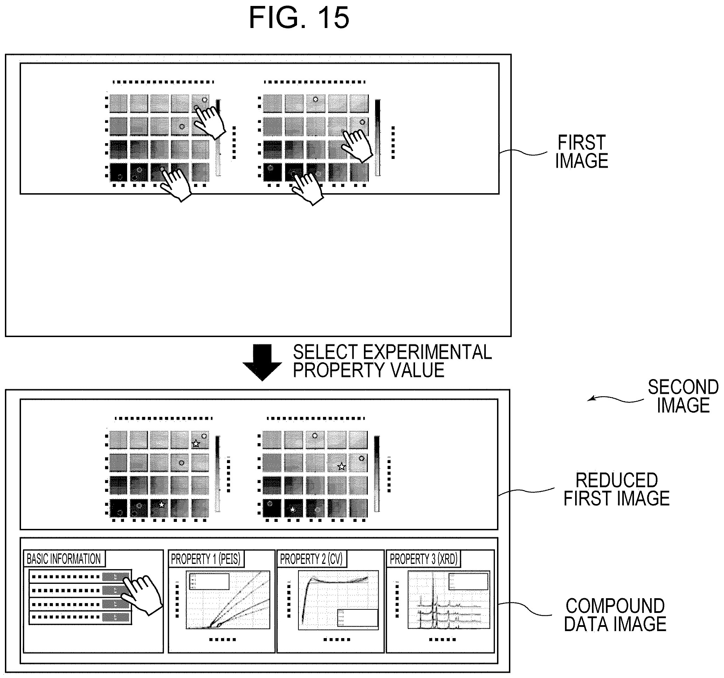

Figs. 14 and 15 illustrate examples of an image indicating transition of an output result of the display unit 6 at a time when experimental property values have been selected. Fig. 14 illustrates an example of an image indicating transition of a displayed result of the display unit 6 at a time when a first image similar to those illustrated in Figs. 10A to 10C is reduced and the compound data image illustrated in Fig. 12 is used. Fig. 15 illustrates an example of an image indicating transition of an output result of the display unit 6 at a time when a first image similar to that illustrated in Fig. 11 is not reduced and experimental property values are selected. As illustrated in Figs. 14 and 15, the selected experimental property values are highlighted as stars in the reduced first image or the first image displayed in a second image. When the user clicks one of the stars in the second image, therefore, the selection of the experimental property values is canceled. It is assumed, for example, that experimental property values have been selected and pieces of compound information data corresponding to the experimental property values are superimposed upon each other. In this case, the selection of an experimental property value found to be unnecessary can be canceled by clicking a corresponding star as described above. Experimental property values can thus be selected or removed and compound information data can be reorganized after a second image is displayed.

<Summary of Embodiment 1-2>

As described above, in a property display method according to embodiment 1-2, in the fifth step, experimental property values superimposed upon the map are selected, and the data obtaining unit 104 obtains a piece of compound identification information corresponding to each of the experimental property values. In the sixth step, the data obtaining unit 104 obtains a piece of compound information data associated with each of the pieces of compound identification information. In the seventh step, the data image generation unit 105 generates a compound data image by superimposing a piece of compound graph data corresponding to each of the obtained pieces of compound information data upon each other.

As a result, since the pieces of compound graph data relating to the experimental property values are displayed while being superimposed upon each other, the user can easily tell differences between the pieces of compound graph data.

The compound data image in the seventh step includes a graph superimposition image in which the piece of compound graph data corresponding to each of the obtained pieces of compound information data is superimposed upon each other and a list image in which the piece of compound identification information associated with each of the obtained pieces of compound information data is shown on a list.

As a result, since the list image is displayed, the user can easily understand the piece of compound identification information corresponding to each of the superimposed pieces of compound graph data. More specifically, since pieces of compound identification information are shown as a list, when the number of experimental property values selected is large, the pieces of compound identification information can be displayed in one screen in an easy-to-see manner and selected as desired.

Alternatively, the compound data image in the seventh step includes a graph superimposition image in which a piece of compound graph data corresponding to each of the obtained pieces of compound information data is superimposed upon each other and a tab switch image including tabs for switching and displaying the piece of compound composition data corresponding to each of the pieces of compound graph data.

As a result, even when the user selects experimental property values, the user can easily visually understand compound composition data relating to the experimental property values by using the tabs. More specifically, when the number of experimental property values selected is small, pieces of compound identification information can be displayed in one screen in an easy-to-see manner and selected as desired by using the tabs.

(Embodiment 1: Description of Operations)

Next, operations performed by the property display apparatus 100 will be described.

(Flowchart)

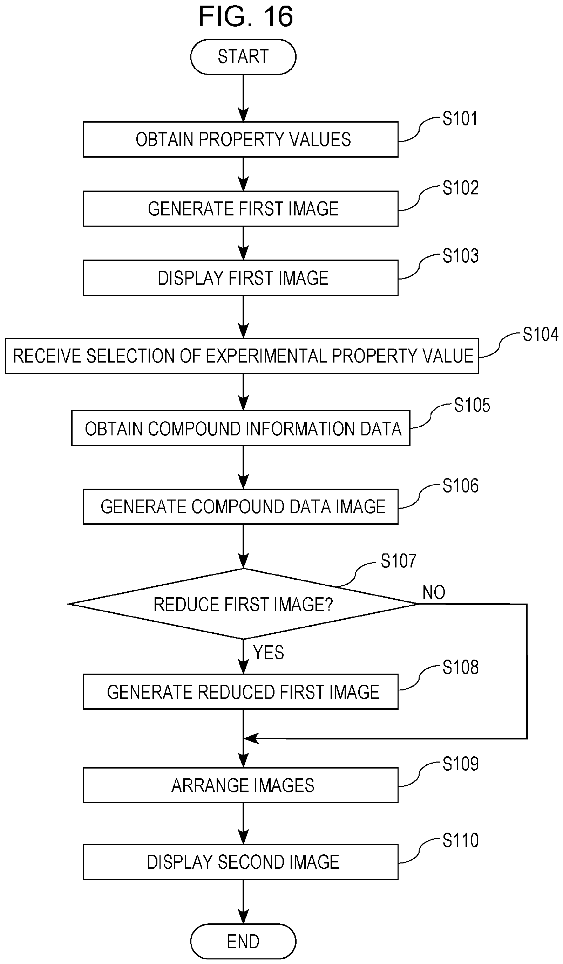

Fig. 16 is a flowchart indicating an overall procedure of a process performed by the property display apparatus 100 according to embodiments 1-1 and 1-2 of the present disclosure.

(Step S101)

The property value obtaining unit 101 obtains, from the predictor database 2 and the experiment database 3, predicted property values and experimental property values, respectively, corresponding to a search area obtained from the input unit 1 and outputs the predicted property values and the experimental property values to the image generation unit 102.

(Step S102)

The image generation unit 102 generates a first image in which the experimental property values are superimposed upon the predicted property values obtained from the property value obtaining unit 101 and outputs the first image to the image arrangement determination unit 106.

(Step S103)

The image arrangement determination unit 106 obtains the first image from the image generation unit 102, determines arrangement of the first image, and outputs the first image to the display unit 6. As a result, the display unit 6 displays the first image.

(Step S104)

When the user selects one of the experimental property value included in the first image displayed on the screen of the display unit 6, the selection reception unit 4 receives the user input and outputs the user input to the data obtaining unit 104. That is, the selection reception unit 4 outputs the experimental property value selected by the user to the data obtaining unit 104.

(Step S105)

The data obtaining unit 104 obtains, from the experiment database 3, compound identification information corresponding to the experimental property value output from the selection reception unit 4 and then obtains, from the compound information database 5, compound information data corresponding to the compound identification information. The data obtaining unit 104 outputs the compound information data to the data image generation unit 105.

(Step S106)

The data image generation unit 105 generates a compound data image on the basis of the compound information data obtained from the data obtaining unit 104 and outputs the compound data image to the image arrangement determination unit 106.

(Step S107)

The image arrangement determination unit 106 determines, on the basis of the compound data image output from the data image generation unit 105, for example, whether to reduce the first image displayed on the display unit 6 in step S103.
Here, if determining that the displayed first image is to be reduced (YES in step S107), the image arrangement determination unit 106 causes the reduced image generation unit 103 to reduce the first image. At this time, the reduced image generation unit 103 obtains the first image from the image generation unit 102. If determining that the first image is not to be reduced (NO in step S107), on the other hand, the image arrangement determination unit 106 uses the first image displayed in step S103.
When the first image has been displayed in full screen in step S103, or when, even if the first image has not been displayed in full screen, the amount of information of the compound information data is large and accordingly the compound data image will be large, for example, the image arrangement determination unit 106 determines that the first image is to be reduced. That is, the image arrangement determination unit 106 makes the determination in consideration of sizes of the first image and the compound data image. When the first image has not been displayed in full screen in step S103, and when the amount of information of the compound information data is small and accordingly the compound data image will be small, the image arrangement determination unit 106 determines that the first image is not to be reduced.

(Step S108)

The reduced image generation unit 103 generates a reduced first image by reducing the first image obtained from the image generation unit 102 and outputs the reduced first image to the image arrangement determination unit 106.

(Step S109)

If determining that the first image is not to be reduced, the image arrangement determination unit 106 generates a second image while determining arrangement such that the unreduced first image and the compound data image obtained from the data image generation unit 105 are displayed in one screen and outputs the second image to the display unit 6. If determining that the first image is to be reduced, the image arrangement determination unit 106 obtains the reduced first image from the reduced image generation unit 103, generates a second image while determining arrangement such that the obtained reduced first image and the compound data image are displayed in one screen, and outputs the second image to the display unit 6.

(Step S110)

The display unit 6 displays the second image obtained from the image arrangement determination unit 106.
The first to third steps in embodiments 1-1 and 1-2 correspond to step S101 illustrated in Fig. 16. The fourth step corresponds to steps S102 and S103, the fifth and sixth steps correspond to steps S104 and S105, and the seventh step corresponds to steps S106 to S110.

<Modification of Embodiment 1>

In description of this modification, numerical information regarding a data representation is input to a predictor, and the predictor outputs property values. Content disclosed in the modification and other embodiments may be combined together to alter the modification such that numerical information regarding a data representation and chemical symbols are input to a predictor and the predictor outputs property values, instead.
First, the property display apparatus 100 causes the display unit 6 to display a first image including first graphs (refer to Fig. 5A). The first graphs may be 20 graphs arranged in four rows and five columns relating to a data representation $Li_{2-3a-4b}(Al_{1-x}Ga_x)_a(Ti_{1-y}Zr_y)_{1+b}O_3$ illustrated in Fig. 5A. The display unit may also be called a display.
Next, the user selects first data included in one of the first graphs (refer to Fig. 10A). That is, when the property display apparatus 100 detects selection of first data, the property display apparatus 100 causes the display unit 6 to display a second image including a reduced first image, which is obtained by reducing a first screen, and a compound data image, which is an image including information relating to the first data (refer to Fig. 10A).

Fig. 10A illustrates an example of the selection of first data. The first graph is a graph that is arranged in a first row and a third column of 20 graphs arranged in four rows and five columns relating to the data representation $Li_{2-3a-4b}(Al_{1-x}Ga_x)_a(Ti_{1-y}Zr_y)_{1+b}O_3$ illustrated in Fig. 10A. The first graph indicates, for a chemical formula $Li_{1.7}(Al_{1-x}Ga_x)_{0.1}(Ti_{1-y}Zr_y)_1O_3$, a value of a band gap with $(x, y) = (0.0, 0.0)$, ..., and a value of a band gap with $(x, y) = (1.0, 1.0)$ (refer to Fig. 4A).

The first graphs are a $graph_{11}$, ..., a $graph_{pq}$, ..., and a $graph_{rs}$ ($1 \leq p \leq r$, $1 \leq q \leq s$, p is a natural number, q is a natural number, r is a natural number larger than or equal to 2, and s is a natural number larger than or equal to 2).

Values $e_{pq(1, 1)}$ to $e_{pq(n, m)}$ are shown in the $graph_{pq}$ (n is a natural number larger than or equal to 2, m is a natural number larger than or equal to 2, i is a natural number, and j is a natural number).

As an example of the first graphs, Figs. 10A and 5A illustrate 20 graphs arranged in four rows and five columns relating to the data representation $Li_{2-3a-4b}(Al_{1-x}Ga_x)_a(Ti_{1-y}Zr_y)_{1+b}O_3$, namely the $graph_{11}$ to a graph4s ($r = 4$, $s = 5$). A relationship between the 20 graphs and the $graph_{11}$ to the $graph_{45}$ is as follows.

| $Graph_{41}$ is graph where b = 0.3 and a = 0.0 | $Graph_{42}$ is graph where b = 0.3 and a = 0.05 | $Grapt_{43}$ is graph where b = 0.3 and a = 0.1 | $Graph_{44}$ is graph where b = 0.3 and a = 0.15 | Graptus is graph where b = 0.3 and a = 0.2 |
|---|---|---|---|---|
| $Graph_{31}$ is graph where b = 0.2 and a = 0.0 | $Graph_{32}$ is graph where b = 0.2 and a = 0.05 | $Graph_{33}$ is graph where b = 0.2 and a = 0.1 | $Graph_{32}$ is graph where b = 0.2 and a = 0.15 | $Graph_{35}$ is graph where b = 0.2 and a = 0.2 |
| $Graph_{21}$ is graph where b = 0.1 and a = 0.0 | $Graph_{32}$ is graph where b = 0.1 and a = 0.05 | $Grapt_{23}$ is graph where b = 0.1 and a = 0.1 | $Graph_{32}$ is graph where b = 0.1 and a = 0.15 | $Graph_{32}$ is graph where b = 0.1 and a = 0.2 |
| $Graph_{11}$ is graph where b = 0.0 and a = 0.0 | $Graph_{32}$ is graph where b = 0.0 and a = 0.05 | $Graph_{42}$ is graph where b = 0.0 and a = 0.1 | $Graph_{14}$ is graph where b = 0.0 and a = 0.15 | $Graph_{32}$ is graph where b = 0.0 and a = 0.2 |

The $graph_{11}$ indicates values $e_{11(1, 1)}$ to $e_{11(n, m)}$, ..., the $graph_{pq}$ indicates values $e_{pq(1, 1)}$ to $e_{pq(n, m)}$, ..., and the $graph_{rs}$ indicates values $e_{rs(1, 1)}$ to $e_{rs(n, m)}$. Values $e_{13(1, 1)}$ to $e_{13(n, m)}$ shown in a $graph_{13}$ will be described hereinafter.

The $graph_{13}$ is a graph arranged in a first row and a third column of the 20 graphs arranged in four rows and five columns relating to the data representation $Li_{2-3a-4b}(Al_{1-x}Ga_x)_a(Ti_{1-y}Zr_y)_{1+b}O_3$ illustrated in Fig. 10A.

The $graph_{13}$ indicates values of 121 band gaps of compounds indicated by 121 chemical formulae obtained by varying x and y with a = 0.1 and b = 0.0 in the data representation $Li_{2-3a-4b}(Al_{1-x}Ga_x)_a(Ti_{1-y}Zr_y)_{1+b}O_3$. $(x, y)$ included in chemical formula 1 and $(x, y)$ included in chemical formula 2, which are any two chemical formulae included in the 121 chemical formulae, are different from each other.

That is, the $graph_{13}$ indicates a value $e_{13(1, 1)}$ ($= e_{13((x, y) = (0.1, 0.0))}$) of a band gap of a substance identified by a chemical formula $Li_{1.7}(Al_1Ga_0)_{0.1}(Ti_1Zr_0)_1O_3$, ..., a value $e_{13(1, 11)}$ ($= e_{13((x, y) = (0.0, 1.0))}$) of a band gap of a substance identified by a chemical formula $Li_{1.7}(Al_1Ga_0)_{0.1}(Ti_0Zr_1)_1O_3$, ..., a value $e_{13(6, 1)}$ ($= e_{13((x, y) = (0.5, 0.0))}$) of a band gap of a substance identified by a chemical formula $Li_{1.7}(Al_{0.5}Ga_{0.5})_{0.1}(Ti_1Zr_0)_1O_3$, ..., a value $e_{13(6, 11)}$ ($= e_{13((x, y) = (0.5, 1.0))}$) of a band gap of a substance identified by a chemical formula $Li_{1.7}(Al_{0.5}Ga_{0.5})_{0.1}(Ti_0Zr_1)_1O_3$, ..., a value $e_{13(11, 1)}$ ($= e_{13((x, y) = (1.0, 0.0))}$) of a band gap of a substance identified by a chemical formula $Li_{1.7}(Al_0Ga_1)_{0.1}(Ti_1Zr_0)_1O_3$, ..., a value $e_{13(11, 11)}$ ($= e_{13((x, y) = (1.0, 1.0))}$) of a band gap of a substance identified by a chemical formula $Li_{1.7}(Al_0Ga_1)_{0.1}(Ti_0Zr_1)_1O_3$. A minimum value and a maximum value of x are 0.0 and 1.0, respectively, and x varies in 0.1 increments. A minimum value and a maximum value of y are 0.0 and 1.0, respectively, and y varies in 0.1 increments.

For example, an expression $e_{13(5, 6)}$ ($= e_{13((x, y) = (0.4, 0.5))}$) may be interpreted to mean a value of a band gap of a substance identified by a chemical formula $Li_{2-3\times0.1-4\times0.0}(Al_{1-0.4}Ga_{0.4})_{0.1}(Ti_{1-0.5}Zr_{0.5})_{1+0.0}O_3$ ($=$ a chemical formula $Li_{1.7}(Al_{0.6}Ga_{0.4})_{0.1}(Ti_{0.5}Zr_{0.5})_{1.0}O_3$) obtained by substituting $(b, a) = 0.0, 0.1$ (based on e13, refer to the above table) and $(x, y) = (0.4, 0.5)$ for the data representation $Li_{2-3a-4b}(Al_{1-x}Ga_x)_a(Ti_{1-y}Zr_y)_{1+b}O_3$.

A first value is a = 0.1, a second value is 1.0 based on b = 0.0 ($= 1 + b = 1 + 0.0$), a third value is x = 0.4, and a fourth value is y = 0.5. A fact that the first to fourth values are these values can be understood by comparing the chemical formula $Li_{2-3\times0.1-4\times0.0}(Al_{1-0.4}Ga_{0.4})_{0.1}(Ti_{1-0.5}Zr_{0.5})_{1+0.0}O_3$ and the data representation $Li_{2-3a-4b}(Al_{1-x}Ga_x)_a(Ti_{1-y}Zr_y)_{1+b}O_3$.

The chemical formula $Li_{2-3\times0.1-4\times0.0}(Al_{1-0.4}Ga_{0.4})_{0.1}(Ti_{1-0.5}Zr_{0.5})_{1+0.0}O_3$ ($=$ the chemical formula $Li_{1.7}(Al_{0.6}Ga_{0.4})_{0.1}(Ti_{0.5}Zr_{0.5})_{1.0}O_3$) includes a first chemical formula $(Al_{0.6}Ga_{0.4})$ including a first chemical symbol Ga and a second chemical formula (Tio.sZro.s) including a second chemical symbol Zr.

0.1 that indicates the first value is a subscript value added to the first chemical formula ($Al_{0.6}Ga_{0.4}$), 1.0 that indicates the second value is a subscript value added to the second chemical formula (Tio.sZro.s), 0.4 that indicates the third value is a subscript value added to the first chemical symbol Ga, and 0.5 that indicates the fourth value is a subscript value added to the second chemical symbol Zr.

The predictor is tuned using pieces of training data including first training data.

The first training data may include a fifth value, a sixth value, a seventh value, and an eighth value based on a chemical formula of a compound to be produced, a value of a certain property of a produced compound, namely, for example, a band gap. An example of the first training data will be described hereinafter.

It is assumed that raw materials $Li_2O$, $La_2O_3$, $Al_2O_3$, and TiOz are processed under certain conditions in order to produce compound A whose chemical formula is $Li_{1.7}(Al_{0.4}Ga_{0.6})_{0.1}(Ti_{0.2}Zr_{0.8})_{1.0}O_3$ and a chemical formula of an actually obtained compound B is $Li_{1.706}Al_{0.041}Ga_{0.059}Ti_{0.2}Zr_{0.8}O_{3.003}$.

A chemical formula (here, $Li_{1.7}Al_{0.04}Ga_{0.06}Ti_{0.2}Zr_{0.8}O_3$ (= $Li_{1.7}(Al_{0.4}Ga_{0.6})_{0.1}(Ti_{0.2}Zr_{0.8})_{1+0.0}O_3$)) of a compound to be produced may also be called a target composition, a target composition formula, or a target chemical formula.

A chemical formula (here, $Li_{1.706}Al_{0.041}Ga_{0.059}Ti_{0.2}Zr_{0.8}O_{3.003}$ of an actually obtained compound may also be called an actual composition, an actual composition formula, or an actual chemical formula.

A value of a band gap obtained by measuring compound B is denoted by eoo.

It can be seen by comparing the chemical formula $Li_{1.7}(Al_{0.4}Ga_{0.6})_{0.1}(Ti_{0.2}Zr_{0.8})_{1+0.0}O_3$ of compound A and the data representation $Li_{2-3a-4b}(Al_{1-x}Ga_x)_a(Ti_{1-y}Zr_y)_{1+b}O_3$ that the fifth value, the sixth value, the seventh value, and the eighth value included in chemical formula A are a = 0.1, 1 + b = 1.0, x = 0.6, and y = 0.8, respectively.

The training data, therefore, is (a, b, x, y, and value of band gap) = (0.1, 0.0, 0.6, 0.8, eoo). The training data may also include information other than a, b, x, y, and the value of the band gap. An example of the information is information for identifying Li, Al, Ga, Ti, Zr, and/or O.

First data may be eoo.

A ninth value, a tenth value, an eleventh value, and a twelfth value are a = 0.1, b = 0.0, x = 0.6, and y = 0.8, respectively. A value to be input to a node of the predictor may be determined by adding an arithmetic operation to a value included in a chemical formula. Here, the sixth value (= 1 + b) is converted into the tenth value (= b).

Since the first value and the fifth value indicate the value of a in the data representation, the first value and the fifth value correspond to each other. Since the second value and the sixth value indicate the value of 1 + b in the data representation, the second value and the sixth value correspond to each other. Since the third value and the seventh value indicate the value of y in the data representation, the third value and the seventh value correspond to each other. Since the fourth value and the eighth value indicate x in the data representation, the fourth value and the eighth value correspond to each other.

When information for identifying a value corresponding to p, a value corresponding to q, a value corresponding to i, and a value corresponding to j is input to the tuned predictor, the predictor outputs a value $e_{pq(i, j)}$.

For example, an output $e_{13(5, 6)}$ (= $e_{13((x, y) = (0.4, 0.5))}$) refers to a value of a band gap of a substance identified by a chemical formula $Li_{2-3\times0.1-4\times0.0}(Al_{1-0.4}Ga_{0.4})_{0.1}(Ti_{1-0.5}Zr_{0.5})_{1+0.0}O_3$ (= a chemical formula $Li_{1.7}(Al_{0.6}Ga_{0.4})_{0.1}(Ti_{0.5}Zr_{0.5})_{1.0}O_3$)) for the data representation $Li_{2-3a-4b}(Al_{1-x}Ga_x)_a(Ti_{1-y}Zr_y)_{1+b}O_3$. $e_{13(5, 6)}$, therefore, is a value output from the predictor when (a, b, x, y) = (0.1, 0.0, 0.4, 0.5) is input to the predictor.

Information relating to the first data is the graph of identification 1 and the graph of property 2 illustrated in Fig. 10A. The graph of property 1 indicates an impedance of 235 ohms, and the graph of property 2 indicates an oxidation potential of 3.5.

(Embodiment 2)

In embodiment 2, a second image is generated and displayed, and the displayed second image is edited and displayed on the basis of a user input. In the present embodiment, the same components as in embodiment 1 are given the same reference numerals, and description thereof is omitted.

A configuration according to embodiment 2 will be described. A display system 2000 according to embodiment 2 of the present disclosure will be described in detail hereinafter with reference to the drawings.

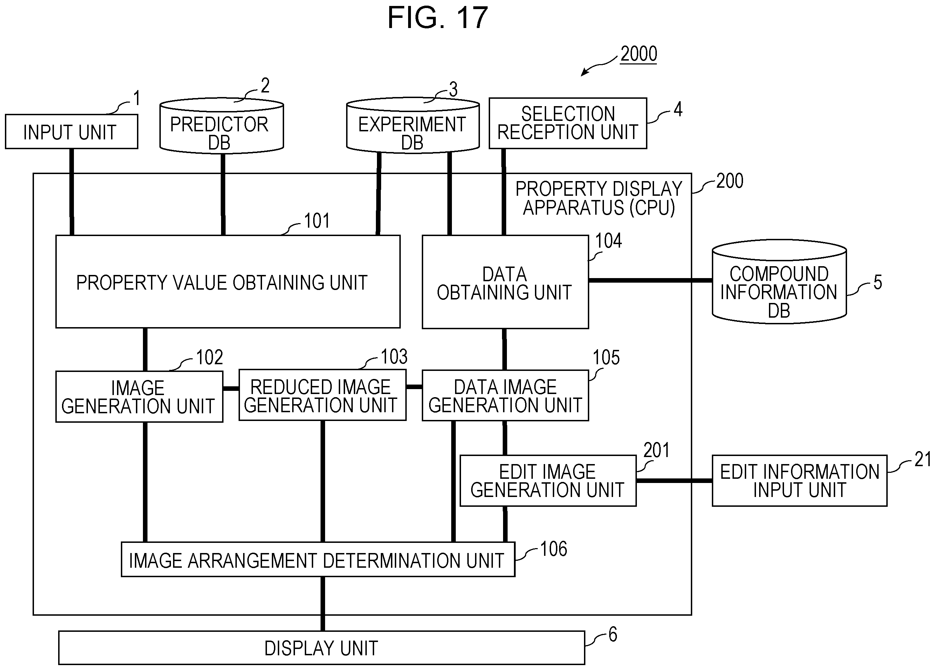

Fig. 17 is a block diagram illustrating the configuration of the display system 2000 according to embodiment 2 of the present disclosure. The display system 2000 illustrated in Fig. 17 includes a property display apparatus 200, an input unit 1, a predictor database (DB) 2, an experiment database (DB) 3, a selection reception unit 4, a compound information database (DB) 5, a display unit 6, and an edit information input unit 21.

The property display apparatus 200 includes a property value obtaining unit 101, an image generation unit 102, a reduced image generation unit 103, a data obtaining unit 104, a data image generation unit 105, an image arrangement determination unit 106, and an edit image generation unit 201. The property display apparatus 200 may be achieved, for example, by a processor such as a central processing unit (CPU) and a memory. In this case, the processor functions as the property display apparatus 200 by, for example, executing a computer program stored in the memory. In Fig. 17, the predictor database 2, the experiment database 3, and the compound information database 5 are achieved, for example, by a nonvolatile memory.

Details of the components illustrated in Fig. 17 will be described hereinafter.

The components other than the edit information input unit 21 and the edit image generation unit 201 are the same as those illustrated in Fig. 1, and description thereof is omitted. Operations performed after the display unit 6 displays a second image in embodiments 1-1 and 1-2 will be described.

[Edit Information Input Unit 21]

The edit information input unit 21 receives a user input and outputs the user input to the edit image generation unit 201. The user input may be a click or a drag. The edit information input unit 21 may be achieved, for example, by a keyboard, a touch sensor, a touchpad, a mouse, or the like.

[Edit Image Generation Unit 201]

The edit image generation unit 201 generates an edit image on the basis of an input obtained from the edit information input unit 21 and outputs the edit image to the image arrangement determination unit 106. The image arrangement determination unit 106 displays the generated edit image on the display unit 6.

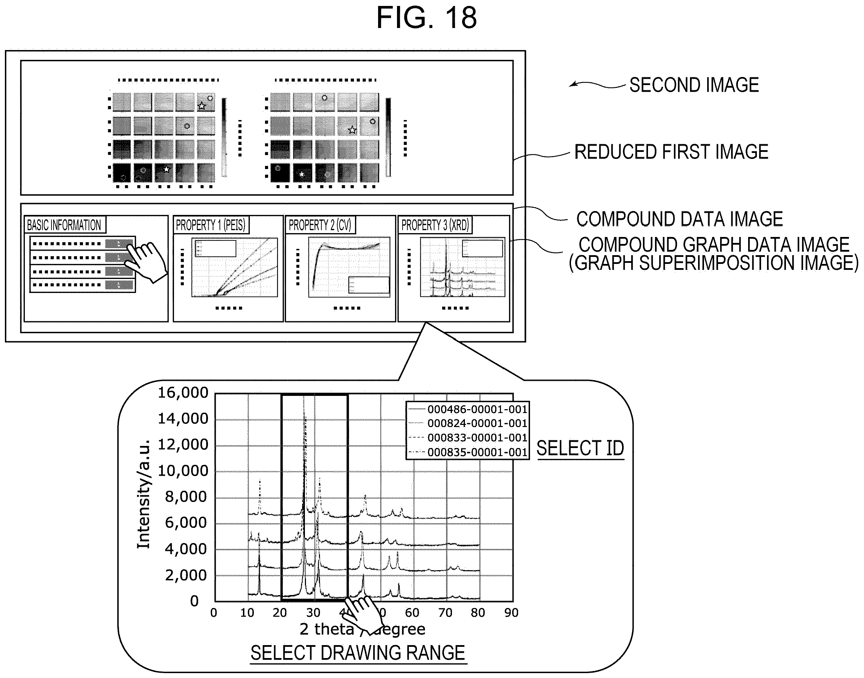

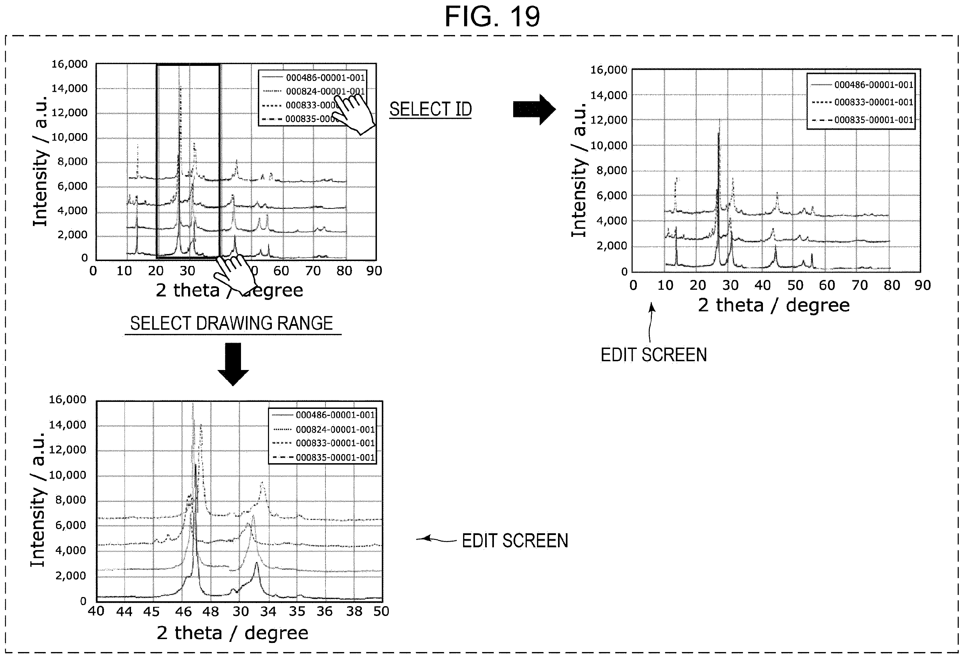

Fig. 18 illustrates an example of edit information input by the user after the display unit 6 displays a second image, and Fig. 19 illustrates an example of an edit image. The edit information is information specified through a user input received by the edit information input unit 21, that is, for example, compound identification information specified by a click or information regarding an area specified by a drag.

In Fig. 18, four pieces of compound graph data corresponding to four selected experimental property values are superimposed upon each other in a graph superimposition image included in the second image. In this case, as illustrated in Fig. 19, compound identification information displayed as a legend is selected by a click, or the compound graph data itself is selected by a click. As a result, the edit image generation unit 201 switches display and non-display of the compound graph data to generate an edit image. That is, the edit image generation unit 201 obtains a user input from the edit information input unit 21, that is, compound identification information or compound graph data selected by the user. The edit image generation unit 201 then switches display and non-display of compound graph data associated with the selected compound identification information or the directly selected compound graph data. An edit image is generated by switching display and non-display in this manner. In the example illustrated in Fig. 19, compound identification information is selected by a click, and compound graph data associated with the compound identification information is switched to non-display.

When a part of compound graph data included in a compound graph data image is enclosed by a drag and selected (i.e., selection of a drawing range), the edit image generation unit 201 generates an edit image by enlarging a selected drawing range. That is, the edit image generation unit 201 obtains a user input, that is, a drawing range selected by the user, from the edit information input unit 21. The edit image generation unit 201 generates an edit image by enlarging the selected drawing range.

When a user of the display system 2000 or the property display apparatus 200 is a researcher and developer, for example, the user is expected to select similar experimental property values from a first image in order to proceed with an experiment. At this time, it is often difficult for the user to visually tell differences between peak positions of compound graph data corresponding to the experimental property values, even though the differences are important for the experiment to proceed.

According to the present embodiment, therefore, the user understands an overall trend by superimposing selected pieces of compound graph data upon each other. Methods for comparing pieces of compound graph data with each other include a method in which the pieces of compound graph data are superimposed upon each other and minor differences between peaks are identified, a method in which the pieces of compound graph data are shifted and superimposed upon each other and major differences between peaks are identified, and a method in which a difference between two pieces of compound graph data is displayed. A method in which the predictor database 2 outputs waveform data and a degree of matching between compound graph data and the waveform data is displayed, a method in which a similarity calculation formula is saved in advance and compound graph data whose degree of similarity does not meet a certain criterion is highlighted, or the like may be used, instead. That is, the user may visually check differences, or differences may be calculated on the basis of machine learning or a calculation formula and compared with each other.

In order to further conduct a more detailed analysis, for example, display of compound graph data may be switched, and an area in which peaks are complex or hard to see due to an overlap may be enlarged. A detailed analysis can be conducted by checking specific points at which peaks match or peaks are different from each other (misalignment of peak positions etc.).

(Embodiment 2: Description of Operations)

Next, operations performed by the property display apparatus 200 will be described.

(Flowchart)

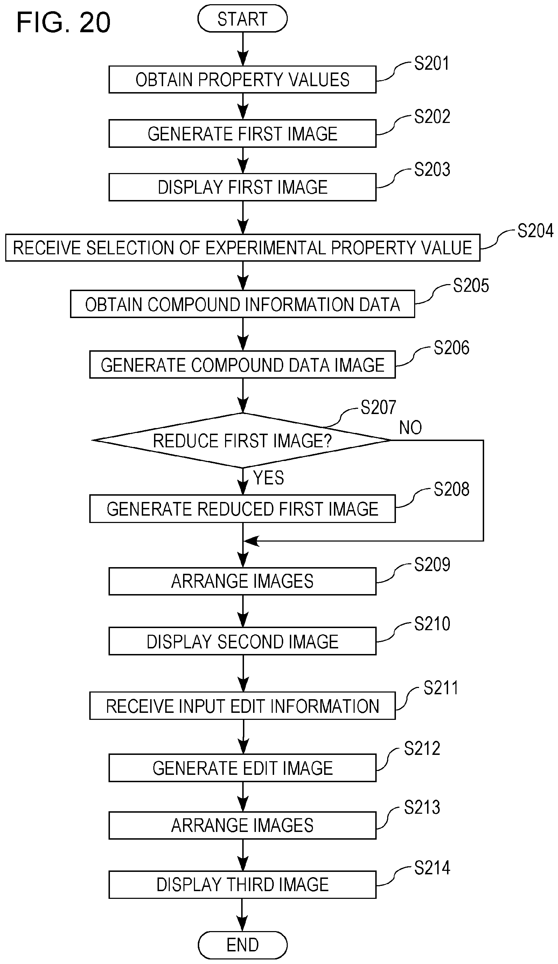

Fig. 20 is a flowchart illustrating an overall procedure of a process performed by the property display apparatus 200 according to embodiment 2 of the present disclosure. Processing in steps S201 to S210 is the same as that in steps S101 to S110 illustrated in Fig. 16, and description thereof is omitted. That is, operations performed after the second image according to embodiment 1 is generated and displayed will be described.

(Step S211)

The edit information input unit 21 receives a user input and outputs the user input to the edit image generation unit 201.

(Step S212)

The edit image generation unit 201 generates an edit image on the basis of the input obtained from the edit information input unit 21 and outputs the edit image to the image arrangement determination unit 106.

(Step S213)

The image arrangement determination unit 106 generates a third image including a first image or a reduced first image and the edit image in one screen and outputs the third image to the display unit 6.

(Step S214)

The display unit 6 displays the third image obtained from the image arrangement determination unit 106.

<Summary of Embodiment 2>

As described above, in the property display method according to embodiment 2, after the seventh step, the edit image generation unit 201 and the image arrangement determination unit 106 edit at least one of superimposed pieces of compound graph data shown in a graph superimposition image, generate a third image on the basis of the edit, and output the third image to the display unit 6. The edit includes switching a state of, among the superimposed pieces of compound graph data, a selected one of the pieces of compound graph data and compound identification information associated with the selected piece of compound graph data between a display state and a non-display state. Alternatively, the edit includes selecting an area in the graph superimposition image and enlarging and displaying the selected area.

As a result, visibility of the superimposed pieces of compound graph data further improves. More specifically, by displaying two of three or more pieces of compound graph data and not displaying other pieces of compound graph data, the two pieces of compound graph data can be compared with each other. By enlarging the area, details of pieces of compound graph data can be compared with each other.

In the property display method according to embodiment 2, a graph superimposition image in which pieces of compound graph data are superimposed upon each other is edited, but a compound graph data image indicating one piece of compound graph data may be edited in the same manner as above. That is, after the seventh step, the edit image generation unit 201 and the image arrangement determination unit 106 select an area in the compound graph data image included in the compound data image illustrated in Fig. 7, for example, and enlarge and display the selected area. As a result, even when compound graph data corresponding to one experimental property value or one piece of compound identification information is shown in a compound data image, visibility of the compound graph data further improves.

(Third Embodiment)

In embodiment 3, a first image in which experimental property values are superimposed upon predicted property values, a second image in which a first image or a reduced first image and a compound data image are displayed in one screen, or a third image in which a first image or a reduced first image and an edit image are displayed in one screen is generated and displayed, and then the displayed image is saved and saved data is read on the basis of user inputs. Data is read when saved data is reconfigured as a fourth image and displayed. In the present embodiment, the same components as in embodiment 1 are given the same reference numerals, and description thereof is omitted.

A configuration according to embodiment 3 will be described. A display system 3000 according to embodiment 3 of the present disclosure will be described in detail hereinafter with reference to the drawings.

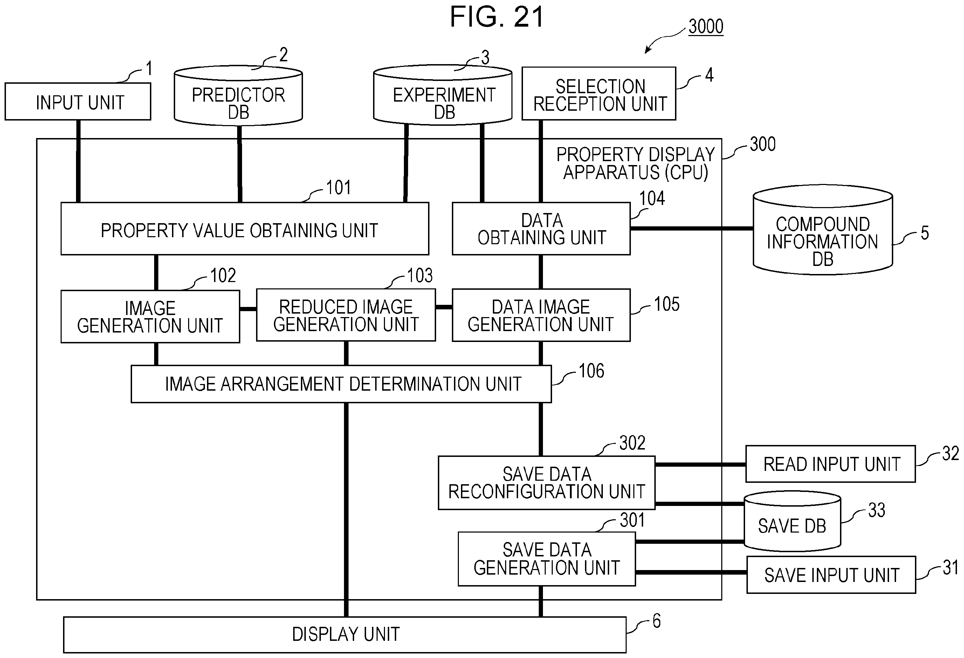

Fig. 21 is a block diagram illustrating the configuration of the display system 3000 according to embodiment 3 of the present disclosure. The display system 3000 illustrated in Fig. 21 includes a property display apparatus 300, an input unit 1, a predictor database (DB) 2, an experiment database (DB) 3, a selection reception unit 4, a compound information database (DB) 5, a display unit 6, a save input unit 31, a read input unit 32, and a save database (DB) 33.

The property display apparatus 300 includes a property value obtaining unit 101, an image generation unit 102, a reduced image generation unit 103, a data obtaining unit 104, a data image generation unit 105, an image arrangement determination unit 106, a save data generation unit 301, and a save data reconfiguration unit 302. The property display apparatus 300 may be achieved, for example, by a processor such as a central processing unit (CPU) and a memory. In this case, the processor functions as the property display apparatus 300 by, for example, executing a computer program stored in the memory. In Fig. 21, the predictor database 2, the experiment database 3, the compound information database 5, and the save database 33 are achieved, for example, by a nonvolatile memory.

Details of the components illustrated in Fig. 21 will be described hereinafter.

The components other than the save input unit 31, the read input unit 32, the save database 33, the save data generation unit 301, and the save data reconfiguration unit 302 are the same as those illustrated in Fig. 1, and description thereof is omitted. That is, operations performed after a first image in which experimental property values are superimposed upon predicted property values, a second image in which a first image or a reduced first image and a compound data image are displayed in one screen, or a third image in which a first image or a reduced first image and an edit image are displayed in one screen is generated and displayed will be described. At this time, the first image, the reduced first image, the second image, and the third image displayed on the display unit 6 in embodiment 3 each include a read data button and a save data button.

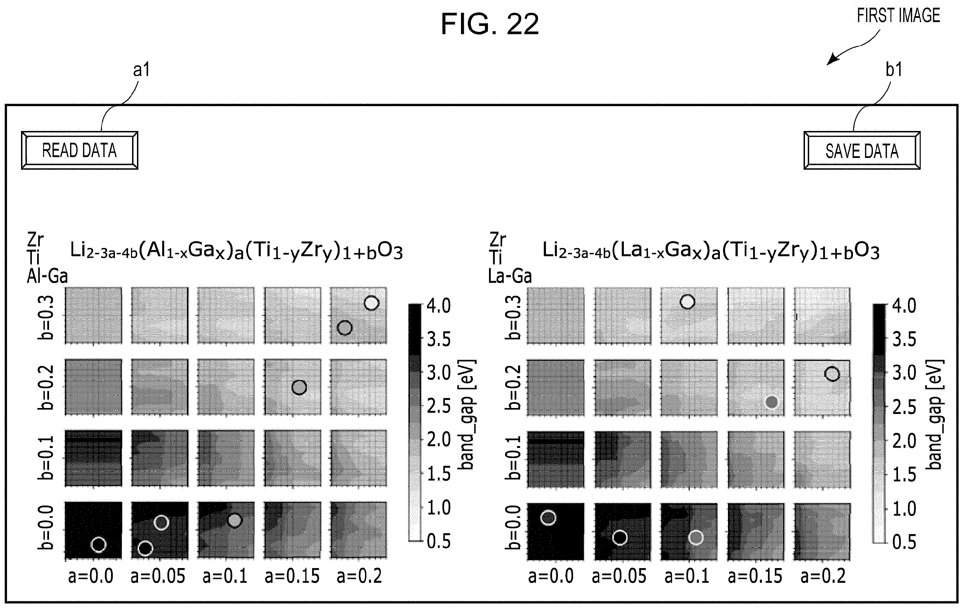

Fig. 22 illustrates an example of a first image including a read data button a1 and a save data button b1. Since data can be saved and read, the user, when he/she is a researcher and developer, can save and reconfirm information regarding properties that he/she has once investigated in the course of experiments in his/her research and development, which often take several years. When users are doing joint research, the users can proceed with experiments while sharing data with files attached to mails or shared in the cloud. That is, even when the users who are doing the joint research are based in distant locations, the users can share data in real-time and conduct the joint research through analyses and discussions.

[Save Input Unit 31]

The save input unit 31 receives a user input and outputs the user input to the save data generation unit 301. The user input may be made by specifying a save file name after clicking the save data button b1. The user may specify the save file name as desired or use a save file name automatically given on the basis of data to be saved.

[Save Data Generation Unit 301]

The save data generation unit 301 outputs a first image, a reduced first image, a second image, or a third image displayed, when a user input is received from the save input unit 31, on the display unit 6 to the save database 33 with a save file name specified by the user. For example, the save data generation unit 301 displays at least one of images including the first image and the second image, converts the displayed image into a file in a certain format, and saves the file.

[Save Database 33]

The save database 33 stores image data obtained from the save data generation unit 301. The data saved in the save database 33 is also called image data, files, or save data. The save database 33 may be a cloud server connected to the property display apparatus 300 over a communication network such as the Internet.

[Read Input Unit 32]

The read input unit 32 receives a user input and outputs save data selected by the user to the save data reconfiguration unit 302. The user input includes, for example, specifying a save file name to be read after clicking the read data button a1.

[Save Data Reconfiguration Unit 302]

The save data reconfiguration unit 302 obtains, from the save database 33, image data corresponding to a save file name specified by the user received from the read input unit 32, reconfigures the image data, and outputs the image data to the image arrangement determination unit 106. That is, a fourth image is output to the image arrangement determination unit 106. The image data saved in the save database 33 is a first image, a reduced first image, a second image, or a third image displayed on the display unit 6. That is, with the image data, a further operation such as selection or editing of experimental property values is not possible. In other words, each image such as the first image is saved while being converted into a file in a format in which a further operation such as selection or editing of experimental property values is not possible. Through reconfiguration, therefore, the images can be restored to states before saving, in which such an operation is possible. Since so-called data backup is possible, for example, an analysis of data shared with a file attached to a mail or shared in the cloud can be resumed immediately after reading, and smooth progression of discussions and more efficient research and development are expected.

The save input unit 31 and the read input unit 32 in the present embodiment may be achieved, for example, by a keyboard, a touch sensor, a touchpad, or the like.

(Embodiment 3: Description of Operations)

Next, operations performed by the property display apparatus 300 will be described.

(Flowchart)

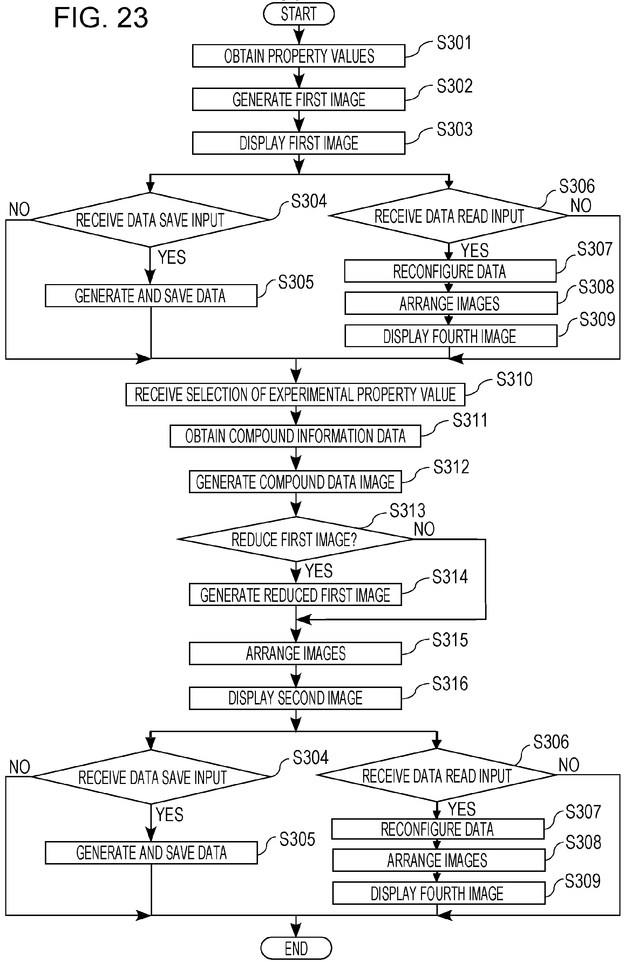

Fig. 23 is a flowchart illustrating an overall procedure of a process performed by the property display apparatus 300 according to embodiment 3 of the present disclosure. Processing in steps S301 to S303 and S310 to S316 is the same as that in steps S101 to S110 illustrated in Fig. 16, and description thereof is omitted. That is, operations performed after a first image in which experimental property values are superimposed upon predicted property values, a second image in which a first image or a reduced first image and a compound data image are displayed in one screen, or a third image in which a first image or a reduced first image and an edit image are displayed in one screen is generated and displayed on the display unit 6 will be described.

(Step S304)

The save input unit 31 receives a user input (YES in step S304) and outputs the user input to the save data generation unit 301.

(Step S305)

The save data generation unit 301 outputs content displayed on the display unit 6 when the user input is received from the save input unit 31 to the save database 33 with a save file name specified by the user. If the save input unit 31 does not receive a user input (NO in step S304), the processing in step S305 is skipped.

(Step S306)

The read input unit 32 receives the user input (YES in step S306) and outputs save data selected by the user to the save data reconfiguration unit 302.

(Step S307)

The save data reconfiguration unit 302 obtains, from the save database 33, the data regarding the save file name specified by the user received from the read input unit 32, reconfigures the data as a fourth image, and outputs the fourth image to the image arrangement determination unit 106.

(Step S308)

The image arrangement determination unit 106 determines arrangement of the fourth image obtained from the save data reconfiguration unit 302 and outputs the fourth image to the display unit 6.

(Step S309)

The display unit 6 displays the fourth image obtained from the image arrangement determination unit 106. If the read input unit 32 does not receive a user input (NO in step S306), the processing in steps S307 to S309 is skipped.

<Summary of Embodiment 3>

As described above, in a property display method according to embodiment 3, after at least one of a first image or a second image is output to the display unit, the save data generation unit 301 converts the output image into a file in a certain format and saves the file. The save data reconfiguration unit 302 and the image arrangement determination unit 106 then obtain the saved file, generate a fourth image by reconfiguring the obtained file as an image, and outputs the fourth image to the display unit 6.

As a result, when files are saved to a cloud server or the like, for example, property display apparatuses 300 can save files and read and reconfigure files through the server. Consequently, users of the property display apparatuses 300 can view the same images even from distant locations and vigorously discuss material properties. That is, the images can be shared.

(Embodiment 4)

In embodiment 4, a second image in which a first image or a reduced first image and a compound data image are displayed in one screen is corrected on the basis of a user input, and a fifth image is generated by correcting the second image and displayed. In the present embodiment, the same components as in embodiment 1 are given the same reference numerals, and description thereof is omitted.

A configuration according to embodiment 4 will be described. A display system 4000 according to embodiment 4 of the present disclosure will be described in detail hereinafter with reference to the drawings.

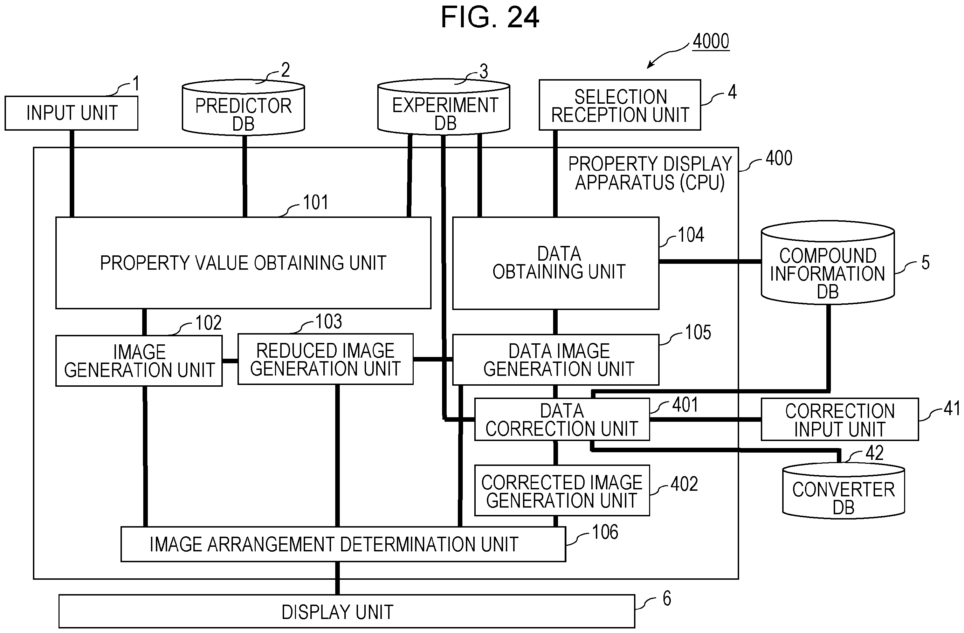

Fig. 24 is a block diagram illustrating the configuration of the display system 4000 according to embodiment 4 of the present disclosure. The display system 4000 illustrated in Fig. 24 includes a property display apparatus 400, an input unit 1, a predictor database (DB) 2, an experiment database (DB) 3, a selection reception unit 4, a compound information database (DB) 5, a display unit 6, a correction input unit 41, and a converter database (DB) 42.

The property display apparatus 400 includes a property value obtaining unit 101, an image generation unit 102, a reduced image generation unit 103, a data obtaining unit 104, a data image generation unit 105, an image arrangement determination unit 106, a data correction unit 401, and a corrected image generation unit 402. The property display apparatus 400 may be achieved, for example, by a processor such as a central processing unit (CPU) and a memory. In this case, the processor functions as the property display apparatus 400 by, for example, executing a computer program stored in the memory. In Fig. 24, the predictor database 2, the experiment database 3, the compound information database 5, and the converter database (DB) 42 are achieved, for example, by a nonvolatile memory.

Details of the components illustrated in Fig. 24 will be described hereinafter.

The components other than the correction input unit 41, the converter database 42, the data correction unit 401, and the corrected image generation unit 402 are the same as in Fig. 1, and description thereof is omitted. That is, operations performed after the display unit 6 displays a second image in which a first image or a reduced first image and a compound data image are displayed in one screen will be described.

At this time, a second image according to embodiment 4 includes a correct data button.

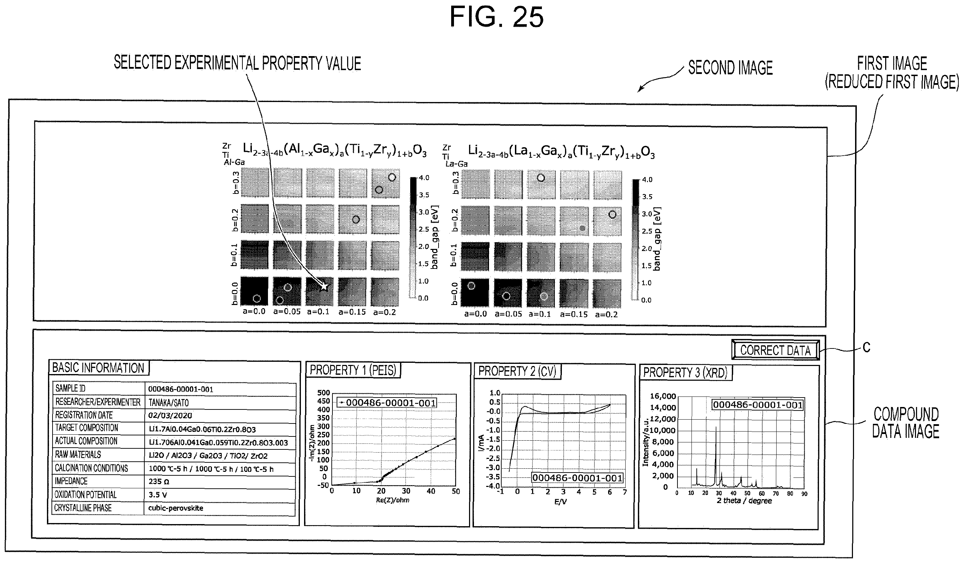

Fig. 25 illustrates an example of a second image including a correct data button c. In a research and development site, for example, (i) a case where an incorrect input has been made when an experimental property value is registered to the experiment database 3, (ii) a case where an experiment has been inappropriate and a correct experimental property value has been obtained later, and (iii) a case where an experimental property value is updated to a more accurate one as a result of acquisition of knowledge through experience, changes to experiment methods, changes overtime, or the like are expected. Since data can be corrected, experimental property values can be corrected or updated, thereby improving accuracy of the experimental property values.

[Correction Input Unit 41]

The correction input unit 41 receives a user input and outputs the user input to the data correction unit 401. The user input includes, for example, displaying a check screen after inputting a correction value to a compound composition data image and clicking the correct data button c. The correction input unit 41 may be achieved, for example, by a keyboard, a touch sensor, a touchpad, a mouse, or the like. The correction value is input to change a value included in compound composition data shown in the compound composition data image.

[Data Correction Unit 401]

The data correction unit 401 outputs a correction value obtained from the correction input unit 41 to the compound information database 5. The data correction unit 401 obtains a converter from the converter database 42. The data correction unit 401 then obtains a corrected experimental property value by inputting corrected compound composition data to the converter and outputs the corrected experimental property value to the experiment database 3. The corrected compound composition data includes the correction value. The data correction unit 401 outputs the correction value to the corrected image generation unit 402.

[Converter Database 42]

The converter database 42 stores in advance a converter that outputs an experimental property value from compound composition data. The converter is, for example, a calculation formula with which an experimental property value can be calculated from compound composition data. A converter may be prepared for each calculation formula, or related calculation formulae may be collectively saved in one converter. Any converter may be used insofar as the converter can use compound composition data as an input and output an experimental property value. For example, the converter may be a learning model generated through machine learning, and, more specifically, may be a neural network generated through machine learning such as deep learning.

[Corrected Image Generation Unit 402]

The corrected image generation unit 402 generates a corrected image on the basis of a correction value obtained from the correction input unit 41 and outputs the corrected image to the image arrangement determination unit 106. The corrected image is a corrected compound data image, that is, a compound data image in which a value indicated by a compound composition data image is replaced by a correction value.

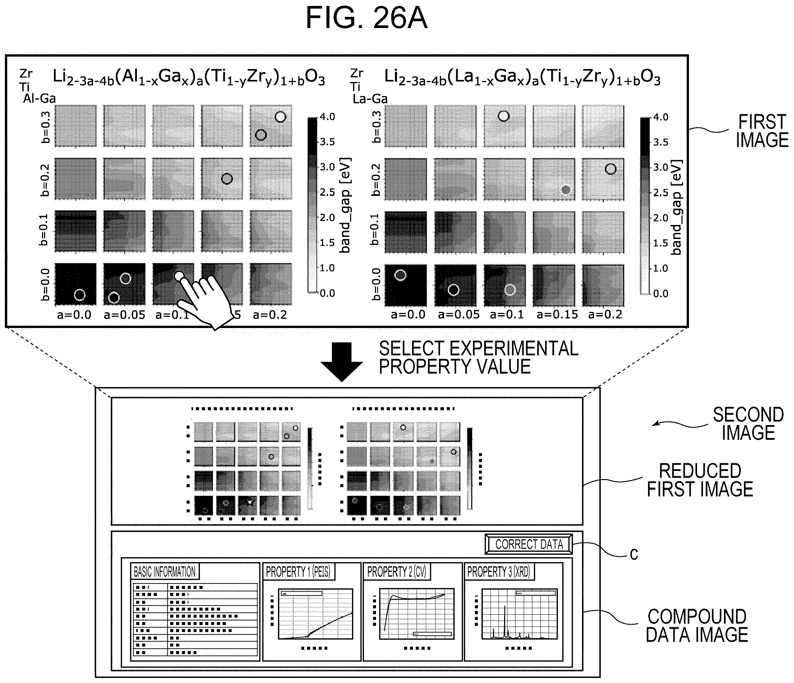

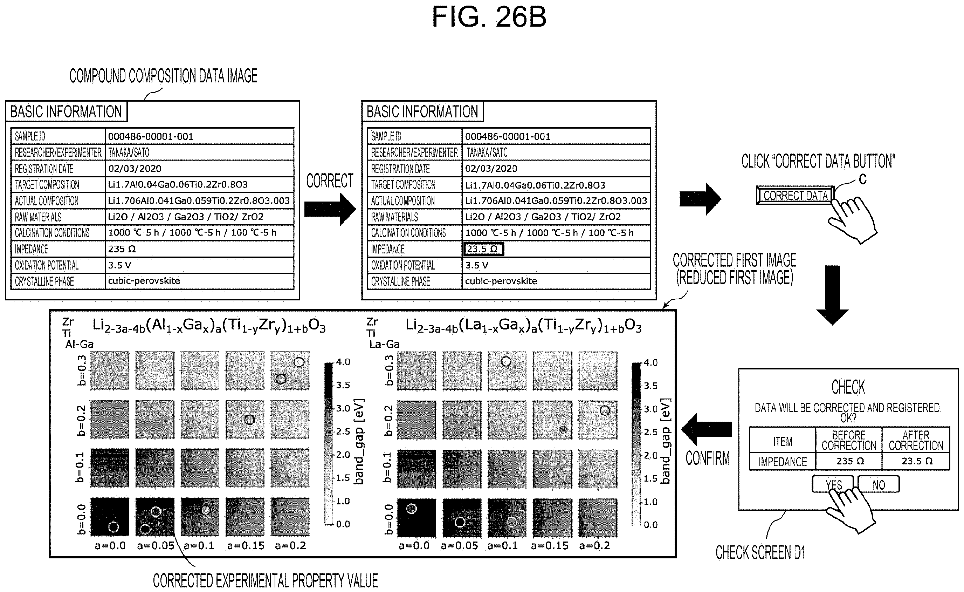

Figs. 26A and 26B illustrate an example of transition from a second image to a fifth image as a result of correction. As illustrated in Fig. 26A, when an experimental property value displayed in a first image is selected, the image arrangement determination unit 106 displays, on the display unit 6, a second image in which a compound data image corresponding to the selected experimental property value and a reduced first image are displayed in one screen. Here, an experimental property value considered to be a result of an incorrect input, that is, an experimental property value greatly different from a predicted property value, for example, is selected. Next, as illustrated in Fig. 26B, the user inputs a correction value for compound composition data shown in the compound composition data image and clicks the correct data button c. For example, a value of impedance "235 $\Omega$" is corrected to a correction value "23.5 $\Omega$". The image arrangement determination unit 106 displays a check screen D1 on the display unit 6. The image arrangement determination unit 106 then confirms correction input information (i.e., the correction value) on the displayed check screen D1 on the basis of a user input and displays a fifth image.

A fifth image is an image in which a corrected first image or a corrected reduced first image and a correction image, which is a corrected compound data image, are displayed in one screen. The first image or the reduced first image is corrected and included in the fifth image when the data correction unit 401 outputs a corrected experimental property value to the experiment database 3. That is, the property value obtaining unit 101 obtains a corrected experimental property value from the experiment database 3. The image generation unit 102 or the reduced image generation unit 103 then replaces a mark indicating the experimental property value before the correction included in the first image or the reduced image with a mark indicating the corrected experimental property value. The first image or the reduced first image is thus corrected. The first image or the reduced first image corrected in this manner is included in the fifth image.

It can be seen from Fig. 26B that the experimental property value has become closer to the predicted property value than in Fig. 26A, which illustrates the state before the correction. According to the present embodiment, a fifth image can be generated and displayed and states before and after correction can be compared with each other without using another apparatus or a program after a correction value is input, and the number of analysis steps can be reduced.

(Embodiment 4: Description of Operations)

Next, operations performed by the property display apparatus 400 will be described.

(Flowchart)

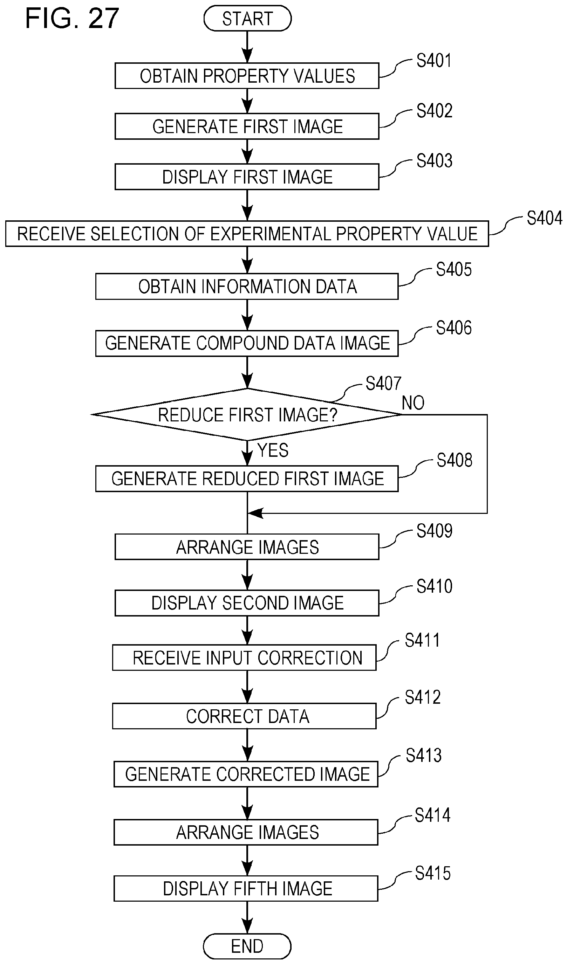

Fig. 27 is a flowchart illustrating an overall procedure of a process performed by the property display apparatus 400 according to embodiment 4 of the present disclosure. Processing in steps S401 to S410 is the same as that in steps S101 to S110 illustrated in Fig. 16, and description thereof is omitted. That is, operations performed after the display unit 6 displays a second image in which a first image or a reduced first image and a compound data image are displayed in one screen will be described.

(Step S411)

The correction input unit 41 receives a correction value as a user input and outputs the correction value to the data correction unit 401.

(Step S412)

The data correction unit 401 outputs the correction value obtained from the correction input unit 41 to the compound information database 5. As a result, a value of compound composition data included in the compound information data saved in the compound information database 5 is replaced by the correction value. The data correction unit 401 obtains a converter from the converter database 42. The data correction unit 401 then inputs the corrected compound composition data to the converter to obtain a corrected experimental property value and outputs the corrected experimental property value to the experiment database 3. The data correction unit 401 also outputs the correction value to the corrected image generation unit 402.

(Step S413)

The corrected image generation unit 402 generates a corrected image on the basis of the correction value obtained from the data correction unit 401 and outputs the corrected image to the image arrangement determination unit 106.

(Step S414)

The image arrangement determination unit 106 generates a fifth image in which the corrected first image or the corrected reduced first image and the corrected image are displayed in one screen and outputs the fifth image to the display unit 6.

(Step S415)

The display unit 6 displays the fifth image obtained from the image arrangement determination unit 106.

<Summary of Embodiment 4>

As described above, in a property display method according to embodiment 4, in the seventh step, if information included in compound information data shown in a second image is corrected after the image arrangement determination unit 106 outputs the second image to the display unit 6 in the seventh step, the data correction unit 401, the corrected image generation unit 402, and the image arrangement determination unit 106 output the corrected information to the compound information database 5 storing the compound information data before the correction, generate a fifth image by correcting the second image on the basis of the corrected information, and output the fifth image to the display unit 6.

As described above, there is a case, for example, where an experimental property value superimposed upon a first image included in the second image has been calculated on the basis of information included in compound information data. If there is an error in the information included in the compound information data in this case, the experimental property values superimposed upon the first image is a value deviating from a predicted property value. With the property display method according to the present embodiment, therefore, the experimental property value in the second image can be corrected since the second image is corrected by correcting the information included in the compound information data. Even if there is an error in compound information data, therefore, a second image can be appropriately corrected easily.

(Embodiment 5)

In embodiment 5, a first image and a display prediction model button are displayed, a prediction model image is generated on the basis of a user input, and a sixth image in which at least one of a first image or a reduced first image and at least one of the prediction model image or a reduced prediction model image are displayed in one screen is generated and displayed. A seventh image in which a compound data image corresponding to an experimental property value indicated by the prediction model image and selected by the user and at least one of the sixth image or a reduced sixth image are displayed in one screen is then generated and displayed. In the present embodiment, the same components as in embodiment 1 are given the same reference numerals, and description thereof is omitted.

A configuration according to embodiment 5 will be described. A display system 5000 according to embodiment 5 of the present disclosure will be described in detail hereinafter with reference to the drawings.

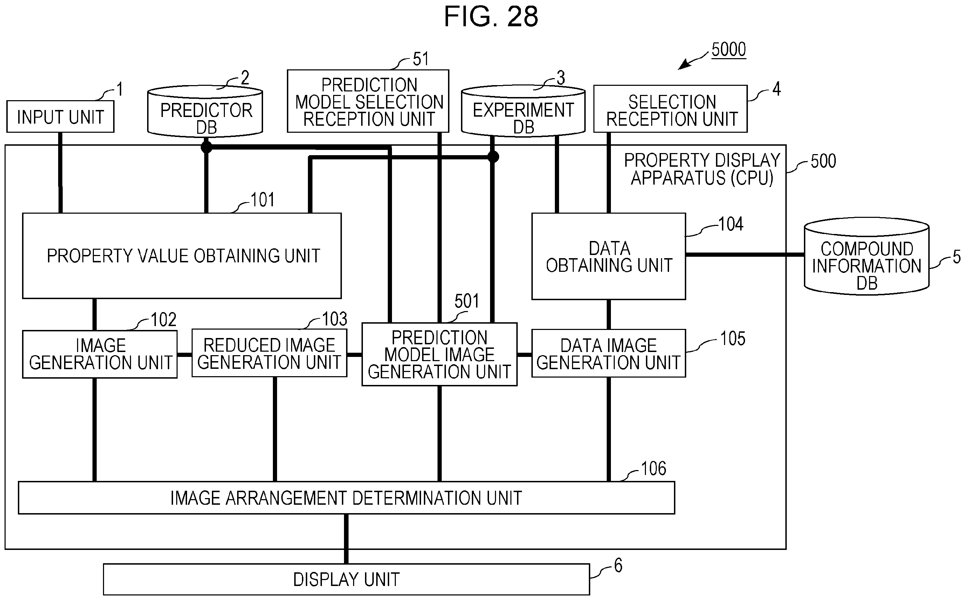

Fig. 28 is a block diagram illustrating the configuration of the display system 5000 according to embodiment 5 of the present disclosure. The display system 5000 illustrated in Fig. 28 includes a property display apparatus 500, an input unit 1, a predictor database (DB) 2, an experiment database (DB) 3, a selection reception unit 4, a compound information database (DB) 5, a display unit 6, and a prediction model selection reception unit 51.

The property display apparatus 500 includes a property value obtaining unit 101, an image generation unit 102, a reduced image generation unit 103, a data obtaining unit 104, a data image generation unit 105, an image arrangement determination unit 106, and a prediction model image generation unit 501. The property display apparatus 500 may be achieved, for example, by a processor such as a central processing unit (CPU) and a memory. In this case, the processor functions as the property display apparatus 500 by, for example, executing a computer program stored in the memory. In Fig. 28, the predictor database 2, the experiment database 3, and the compound information database 5 are achieved, for example, by a nonvolatile memory.

Details of the components illustrated in Fig. 28 will be described hereinafter.

The components other than the prediction model selection reception unit 51 and the prediction model image generation unit 501 are the same as in Fig. 1, and description thereof is omitted. That is, operations performed after a first image is displayed will be described.

At this time, a first image in embodiment 5 includes a display prediction model button.

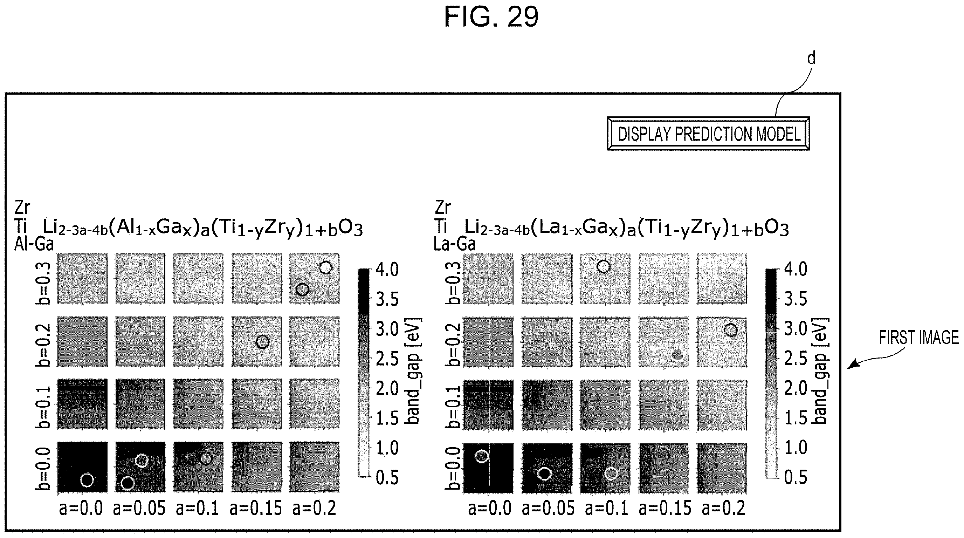

Fig. 29 illustrates an example of a first image including a display prediction model button d. Since the first image prioritizes improved visual understanding, it is impossible to know how much an experimental property value is actually different from a corresponding predicted property value. When there are experimental property values or the first image is as illustrated in Fig. 4B, that is, when there are 100 or more image element maps, it takes enormous time to examine experimental property values one by one. It becomes possible, therefore, to know how much an experimental property value is different from a corresponding predicted property value by displaying a prediction model indicating a correspondence between experimental property values and predicted property values using a linear graph or the like.

[Prediction Model Selection Reception Unit 51]

The prediction model selection reception unit 51 receives a user input and outputs the user input to the prediction model image generation unit 501. The user input may be clicking the display prediction model button d. The prediction model selection reception unit 51 may be achieved, for example, by a keyboard, a touch sensor, a touchpad, a mouse, or the like.

[Prediction Model Image Generation Unit 501]

The prediction model image generation unit 501 obtains, from the experiment database 3, experimental property values included in a first image displayed when the prediction model selection reception unit 51 receives an input and obtains, from the predictor database 2, predicted property values. The prediction model image generation unit 501 then generates a prediction model image on the basis of the obtained experimental property values and the obtained predicted property values and outputs the prediction model image to the image arrangement determination unit 106.

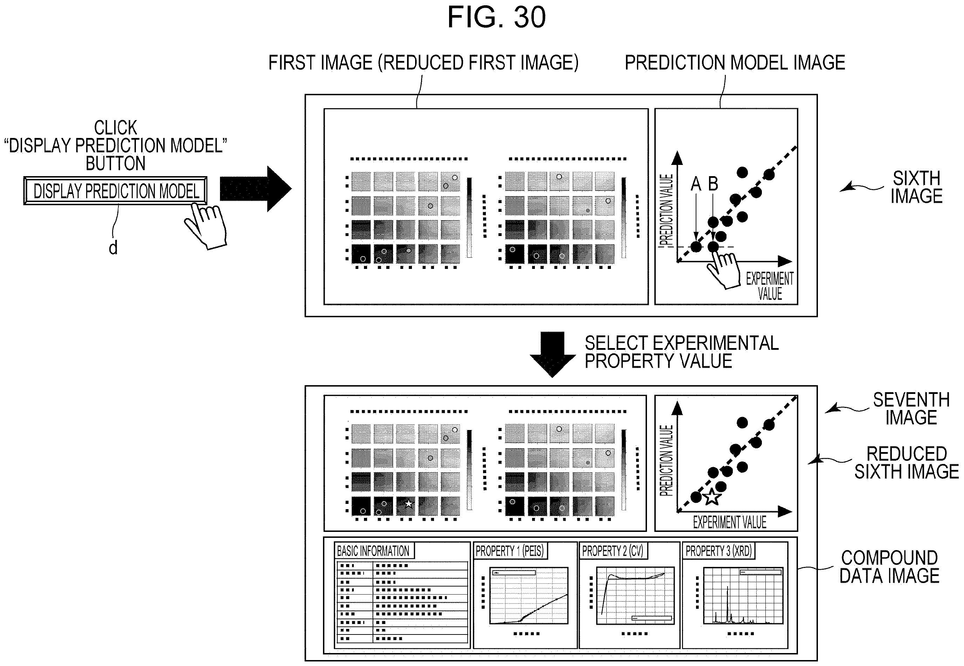

Fig. 30 illustrates an example of an image indicating transition of a display screen. After the user selects the display prediction model button d, the image arrangement determination unit 106 displays, on the display unit 6, a sixth image in which a reduced first image and a prediction model image are displayed in one screen. As illustrated in Fig. 30, the prediction model image is an image indicating a predicted property value and an experimental property value corresponding to the same compound identification information with one dot and is a graph in which values on a vertical axis and values on a horizontal axis indicate predicted property values and experimental property values, respectively. When the user selects one of the experimental property values included in the prediction model image in the sixth image, the selection reception unit 4 obtains the selected experimental property value and outputs the experimental property value to the data obtaining unit 104. The data image generation unit 105 generates a compound data image corresponding to the selected experimental property value and outputs the compound data image to the image arrangement determination unit 106. The image arrangement determination unit 106 displays, on the display unit 6, a seventh image in which the compound data image and at least one of the sixth image or a reduced sixth image are displayed in one screen. As a result, the user can select data while viewing the prediction model image, which improves analysis efficiency compared to when the user selects an experimental property value in first image. When there is an experimental property value greatly different from a predicted property value, for example, the user selects the experimental property value and checks corresponding compound information data. The experimental property value, for example, is a dot B slightly away from a broken line, on which the predicted property value and the experimental property value perfectly match, in the prediction model image illustrated in Fig. 30. When there is an experimental property value that substantially matches a predicted property value, on the other hand, the user selects the experimental property value and checks corresponding compound information data. In an example, the experimental property value that substantially matches the predicted property value is a dot A partly overlapping the broken line, on which the predicted property value and the experimental property value perfectly match, in the prediction model image illustrated in Fig. 30. The user can then compare compound information data and find a parameter or a processing condition that has caused the difference. It is easier for the user to determine, on the basis of the parameter or the processing condition that has caused the difference, whether the difference is due to accuracy of the predicted property value or the experimental property value and efficiently examine improvement measures to be taken.

(Embodiment 5: Description of Operations)

Next, operations performed by the property display apparatus 500 will be described.

(Flowchart)

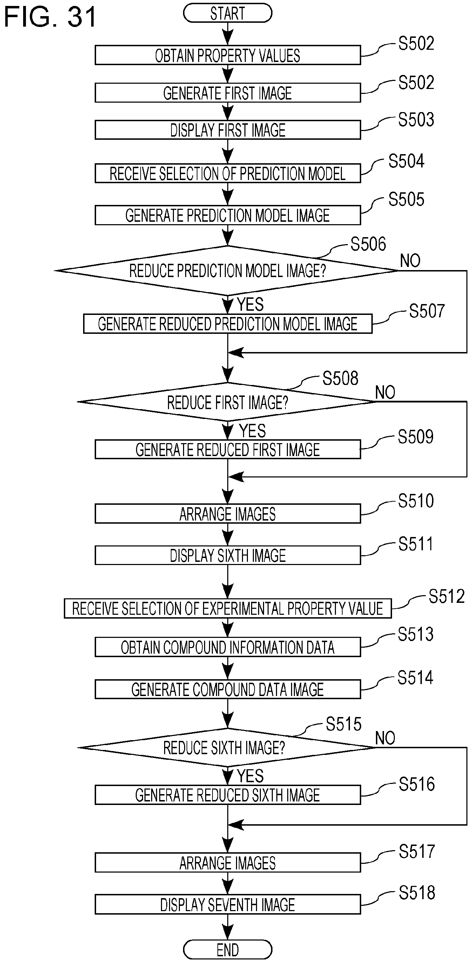

Fig. 31 is a flowchart illustrating an overall procedure of a process performed by the property display apparatus 500 according to embodiment 5 of the present disclosure. Processing in steps S501 to S503, steps S508 and S509, and steps S512 to S514 is the same as that in steps S101 to S108 illustrated in Fig. 16, and description thereof is omitted.

(Step S504)

The prediction model selection reception unit 51 receives a user input and outputs the user input to the prediction model image generation unit 501.

(Step S505)

The prediction model image generation unit 501 obtains, from the experiment database 3, experimental property values included in a first image displayed when the input is received from the prediction model selection reception unit 51 and obtains, from the predictor database 2, predicted property values. The prediction model image generation unit 501 then generates a prediction model image on the basis of the obtained experimental property values and predicted property values.

(Step S506)

The image arrangement determination unit 106 determines whether to reduce the prediction model image. If the prediction model image generated by the prediction model image generation unit 501 is to be reduced (YES in step S506), the image arrangement determination unit 106 causes the reduced image generation unit 103 to reduce the prediction model image. At this time, the prediction model image generation unit 501 outputs the prediction model image to the reduced image generation unit 103. The image arrangement determination unit 106 then obtains the reduced prediction model image from the reduced image generation unit 103. If the prediction model image is not to be reduced (NO in step S506), the image arrangement determination unit 106 obtains the prediction model image from the prediction model image generation unit 501.

(Step S507)

The reduced image generation unit 103 reduces the prediction model image obtained from the prediction model image generation unit 501 and outputs the reduced prediction model image to the image arrangement determination unit 106.

(Step S510)

The image arrangement determination unit 106 generates a sixth image in which at least one of the first image or a reduced first image and at least one of the prediction model image or the reduced prediction model image are displayed in one screen and outputs the sixth image to the display unit 6.

(Step S511)

The display unit 6 displays the sixth image obtained from the image arrangement determination unit 106.

(Step S515)

The image arrangement determination unit 106 determines whether to reduce the sixth image.
If the sixth image is to be reduced (YES in step S515), the image arrangement determination unit 106 causes the reduced image generation unit 103 to reduce the sixth image. At this time, the image arrangement determination unit 106 outputs the sixth image to the reduced image generation unit 103. If the sixth image is not to be reduced (NO in step S515), the process proceeds to step S517.

(Step S516)

The reduced image generation unit 103 reduces the sixth image obtained from the image arrangement determination unit 106 and outputs the reduced sixth image to the image arrangement determination unit 106.

(Step S517)

The image arrangement determination unit 106 generates a seventh image in which a compound data image and at least one of the sixth image or the reduced sixth image are displayed in one screen and outputs the seventh image to the display unit 6.

(Step S518)

The display unit 6 displays the seventh image obtained from the image arrangement determination unit 106.

<Summary of Embodiment 5>

As described above, in a property display method according to embodiment 5, in the fourth step, after the image arrangement determination unit 106 outputs a first image to the display unit 6, the prediction model image generation unit 501 generates a prediction model image based on predicted property values and at least one experimental property value shown in the first image. The image arrangement determination unit 106 then generates a sixth image including the first image and the prediction model image and outputs the sixth image to the display unit. Here, if one of the at least one experimental property value shown in the prediction model image is selected, the data obtaining unit 104 obtains compound identification information corresponding to the selected experimental property value and compound information data associated with the compound identification information. The image arrangement determination unit 106 generates a seventh image including an image indicating the compound information data and the sixth image and outputs the seventh image to the display unit 6.
As a result, not only when one of the experimental property values shown in the first image is selected but also when one of the at least one experimental property value shown in the prediction model image include in the sixth image is selected, an image indicating compound information data relating to the experimental property value is displayed. The user, therefore, can easily select an experimental property value greatly different from a predicted property value in a prediction model image indicating a relationship between predicted property values and experimental property values, for example, and refer to compound information data relating to the experimental property value. Since a seventh image includes a first image, a prediction model image, and an image indicating compound information data in one screen, the user can view these images all at once without switching a screen.

(Embodiment 6)

In embodiment 6, a first image is displayed, compound identification information corresponding to an experimental property value selected by the user is obtained, predicted property data corresponding to the compound identification information is obtained, and a compound data image according to a relationship with the predicted property data is generated and displayed. The predicted property data indicates, for example, features of compound graph data predicted for a compound having the compound identification information. In a specific example, the predicted property data indicates each of peaks of included the compound graph data as a feature. That is, the present embodiment is effective, for example, when compound graph data with which peak positions can be compared with ones in predicted property data is saved in the compound information database 5. In the present embodiment, the same components as in embodiment 1 are given the same reference numerals, and description thereof is omitted.

A configuration according to embodiment 6 will be described. A display system 6000 according to embodiment 6 of the present disclosure will be described in detail hereinafter with reference the drawings.

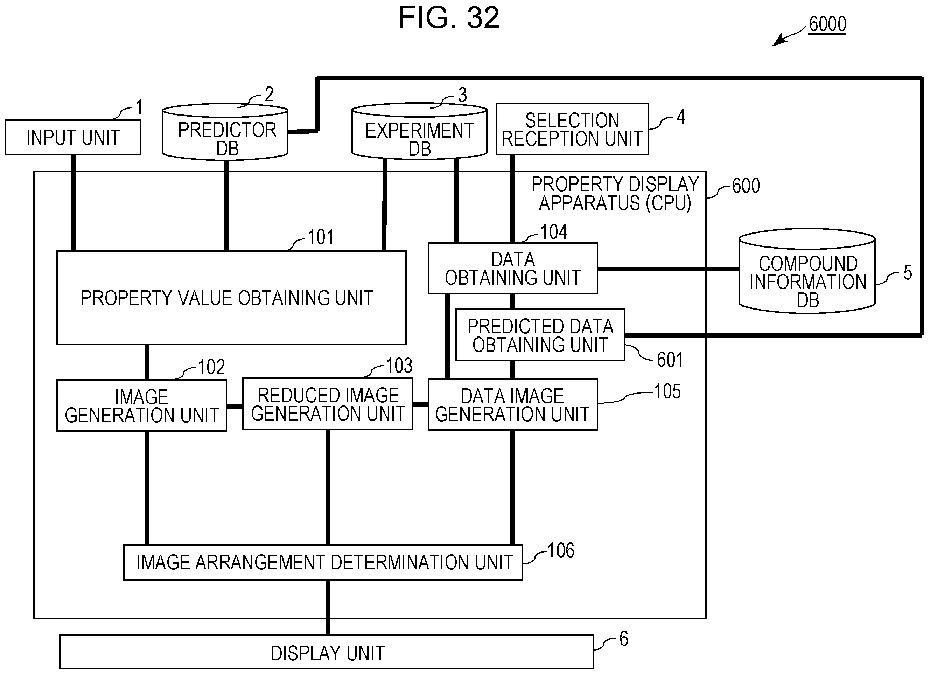

Fig. 32 is a block diagram illustrating the configuration of the display system 6000 according to embodiment 6 of the present disclosure. The display system 6000 illustrated in Fig. 32 includes a property display apparatus 600, an input unit 1, a predictor database (DB) 2, an experiment database (DB) 3, a selection reception unit 4, a compound information database (DB) 5, and a display unit 6.

The property display apparatus 600 includes a property value obtaining unit 101, an image generation unit 102, a reduced image generation unit 103, a data obtaining unit 104, a data image generation unit 105, an image arrangement determination unit 106, and a predicted data obtaining unit 601. The property display apparatus 600 may be achieved, for example, by a processor such as a central processing unit (CPU) and a memory. In this case, the processor functions as the property display apparatus 600 by, for example, executing a computer program stored in the memory. In Fig. 32, the predictor database 2, the experiment database 3, and the compound information database 5 are achieved, for example, by a nonvolatile memory.

Details of the components illustrated in Fig. 32 will be described hereinafter.

The components other than the predicted data obtaining unit 601 are the same as in Fig. 1, and description thereof is omitted. That is, operations performed after the first image is displayed will be described.

[Predicted Data Obtaining Unit 601]

The prediction data obtaining unit 601 obtains, from the predictor database 2, predicted property data corresponding to compound identification information obtained by the data obtaining unit 104 and outputs the predicted property data to the data image generation unit 105.

A predictor held in the predictor database 2 outputs predicted property data corresponding to a compound in response to an input of a composition of the compound, that is, a combination of option data. The predicted data obtaining unit 601, therefore, obtains predicted property data corresponding to a compound having compound identification information obtained by the data obtaining unit 104 by inputting a combination of option data indicating a composition of the compound to the predictor. In the present embodiment, any predictor may be used insofar as the predictor can output predicted property data in response to an input composition of a compound.

Alternatively, the predictor held in the predictor database 2 may output predicted property data corresponding to a compound in response to an input of a composition of the compound, that is, a combination of option data, and information included in compound information data regarding the compound. In this case, the predicted data obtaining unit 601 inputs, to the predictor, a combination of option data indicating a composition of a compound having compound identification information obtained by the data obtaining unit 104 and information included in compound information data regarding the compound obtained from the compound information database 5. As a result, the predicted data obtaining unit 601 obtains predicted property data corresponding to the compound.

Figs. 33 and 34 illustrate examples of a compound graph data image. Here, the compound graph data image indicates, for example, XRD (X-ray diffraction) data as compound graph data. Here, XRD refers to a type of analysis method for finding out a type of substance. XRD data is data indicating a result of XRD as, for example, a graph. A horizontal axis of the graph represents a diffraction angle, and a vertical axis of the graph represents diffracted X-ray intensity. In the following description, XRD data is data obtained as a result of an experiment conducted on a compound having compound identification information, and predicted property data indicates peak positions and intensity, for example, of the XRD data predicted for the compound having the compound identification information.

It is well known that there is a close relationship between a type of substance (i.e., a material or a compound) and properties of the substance, and it is extremely important in material development to identify a type of substance synthesized. Fig. 33 illustrates XRD data and predicted property data whose peak positions substantially match. An upper part 611 of Fig. 33 illustrates XRD data corresponding to an experimental property value. A lower part 612 illustrates a comparison between peak positions in the predicted property data and peak positions in the XRD data corresponding to the experimental property value. In the comparison of peaks between the predicted property data and the XRD data, broken lines indicate peak positions of the XRD data. When peak positions match between the XRD data and the predicted property data, at least parts of the broken lines are shown as solid lines. That is, the XRD data corresponding to the experimental property value illustrated in the upper part 611 of Fig. 33 includes seven high and low peaks, and the lower part 612 indicates that positions of six out of the seven peaks match peak positions in the predicted property data.

Fig. 34, on the other hand, illustrates XRD data and predicted property data whose peak positions do not match. XRD data corresponding to an experimental property value illustrated in an upper part 613 of Fig. 34 includes seven high and low peaks, and a lower part 614 indicates that positions of only two out of the seven peaks match peak positions in the predicted property data. In this case, for example, processing conditions might not be optimal. Alternatively, the type of substance has not been identified.

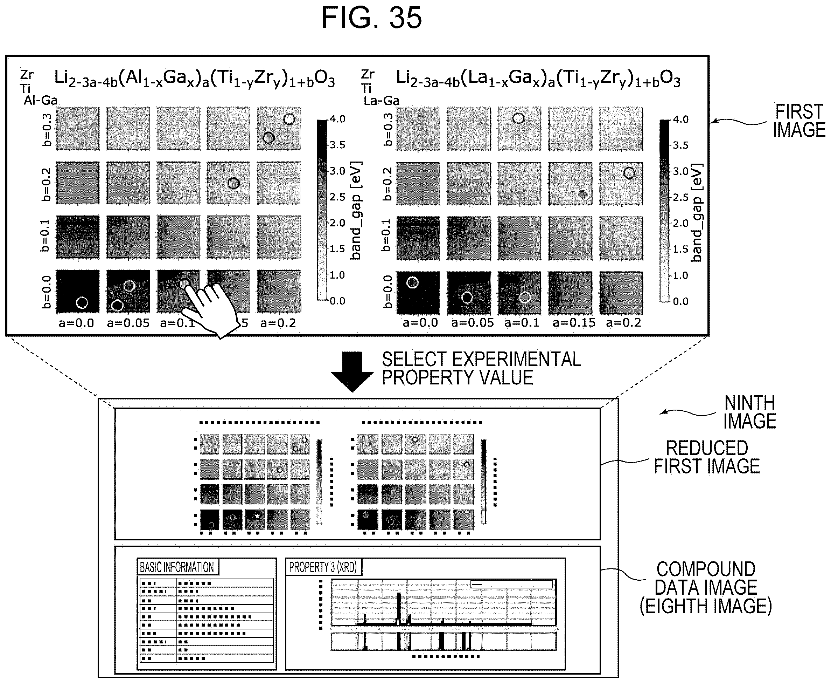

Fig. 35 illustrates an example of an image indicating transition of a display screen. As a result, since a displayed result changes in accordance with a degree of matching between predicted property data and XRD data, it becomes easier for the user to perform analyses and discussions, and the user can examine candidates for next experiment conditions. That is, efficiency of research also improves.

(Embodiment 6: Description of Operations)

Next, operations performed by the property display apparatus 600 will be described.

(Flowchart)

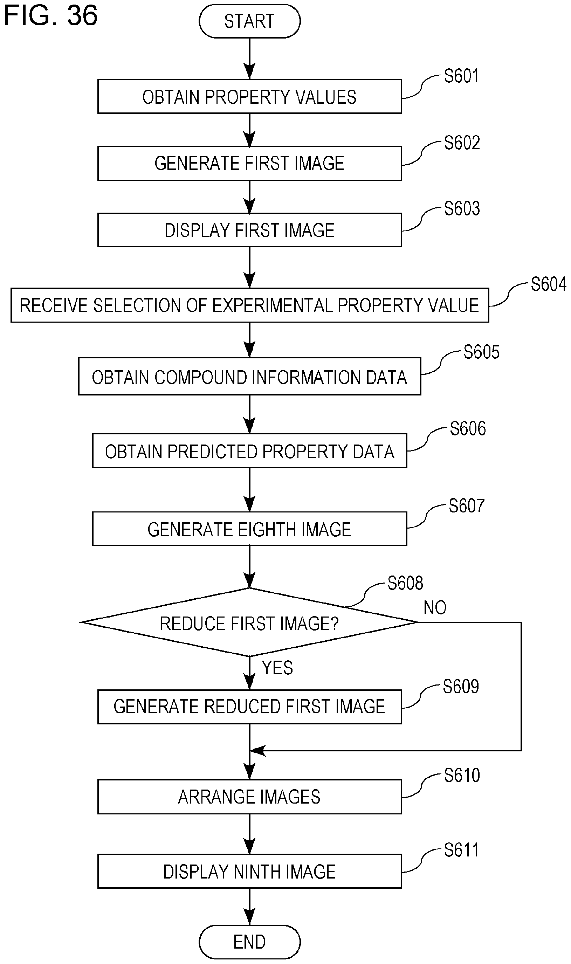

Fig. 36 is a flowchart illustrating an overall procedure of a process performed by the property display apparatus 600 according to embodiment 6 of the present disclosure. Processing in steps S601 to S605 and processing in steps S608 and S609 are the same as that in steps S101 to S105 and that in steps S107 and S108, respectively, illustrated in Fig. 16, and description thereof is omitted.

(Step S606)

The predicted data obtaining unit 601 obtains, from the predictor database 2, predicted property data corresponding to compound identification information obtained by the data obtaining unit 104 and outputs the predicted property data to the data image generation unit 105.

(Step S607)

The data image generation unit 105 generates an eighth image on the basis of compound information data obtained from the data obtaining unit 104 and the predicted property data obtained from the predicted data obtaining unit 601. The compound information data includes, for example, XRD data as compound graph data. The eighth image is, for example, a compound composition data image and the compound graph data image illustrated in Fig. 33 or 34.

(Step S610)

The image arrangement determination unit 106 generates a ninth image by determining arrangement such that at least one of a first image or a reduced first image and the eighth image are displayed in one screen and outputs the ninth image to the display unit 6.

(Step S611)

The display unit 6 displays the ninth image obtained from the image arrangement determination unit 106.

<Summary of Embodiment 6>

As described above, in a property display method according to embodiment 6, in the sixth step, the data obtaining unit 104 obtains compound information data associated with compound identification information, and the predicted data obtaining unit 601 calculates predicted property data corresponding to the compound identification information using the predictor database 2. The data image generation unit 105 and the image arrangement determination unit 106 then generate an eighth image on the basis of the compound information data and the predicted property data, generate a ninth image including the eighth image and the first image, and output the ninth image to the display unit 6.
As a result, an eighth image indicating differences between compound information data and predicted property data, for example, can be generated, and the eighth image can be displayed in the same screen as a first image. The differences are, for example, ones in peak positions. As a result, material development can be supported more appropriately.

<Appendix>

Although the number of categorical variables is four in each of the above embodiments, more than four categorical variables may be provided, instead. That is, among the variables, the number of categorical variables, that is, the number of first variables for which option data indicating chemical elements is substituted, may be four or more, instead.
As a result, since a map is generated for a compound composed by combining together four or more elements, a map indicating information for many composition formulae (or chemical formulae) can be generated by changing the combination. That is, a map indicating a predicted property value of each of compounds expressed by many composition formulae (or chemical formulae) can be generated. Material development, therefore, can be supported more effectively.

Although the total number of discrete variables and continuous variables is four in each of the above embodiments, more than four discrete variables and continuous variables may be used, instead. In other words, among the variables, the number of discrete variables and continuous variables, that is, the number of second variables for which option data indicating values is substituted, may be four or more, instead.

As a result, since a map is generated for a compound composed by combining together four or more values (i.e., coefficients of elements), a map indicating information for many composition formulae (or chemical formulae) can be generated by changing the combination. That is, a map indicating a predicted property value of each of compounds expressed by many composition formulae (or chemical formulae) can be generated. Material development, therefore, can be supported more effectively.

The map includes image maps defined by a first axis and a second axis corresponding to two of the four or more second variables. The image maps include image element maps each arranged in a matrix along the first and second axes. Each of the image element maps is defined by a third axis and a fourth axis corresponding to other two of the four or more second variables. Predicted property values of compounds corresponding to determined compositions are indicated by colors or shades of color at positions on the image element maps corresponding to the determined compositions.

As illustrated in Fig. 4A, for example, the first and second axes corresponding to the two second variables are axes corresponding to the discrete variable a and the discrete variable b, respectively. As illustrated in Fig. 4A, the third and fourth axes corresponding to the other two second variables are axes corresponding to the continuous variable x and the continuous variable y, respectively. In the example illustrated in Fig. 4A, the predicted property values are indicated by black and white shades.

As a result, even when the number of second variables is four or more, a map on which a predicted property value of a compound expressed by each combination, that is, each composition formula (or each chemical formula), is clearly shown can be generated.

The predictor stored in the predictor database 2 in each of the above embodiments may be a machine learning model, instead. That is, in the second step, the property value obtaining unit 101 obtains predicted property values of compounds corresponding to determined compositions of the compounds by inputting the compositions of the compounds to the machine learning model based on a certain calculation algorithm. The machine learning model is obtained by performing machine learning such that a predicted property value of a compound corresponding to a determined composition is output in response to an input of the composition of the compound.

By performing, on the machine learning model, machine learning in which many combinations of option data and experimental property values for the combinations are used as training data, for example, a machine learning model with high prediction accuracy can be generated. Accurate predicted property values, therefore, can be obtained using the machine learning model.

<OtherAspects>

Although the display systems 1000 to 6000 according to the present disclosure have been described on the basis of embodiments, the present disclosure is not limited to these embodiments. The scope of the present disclosure also includes modes obtained by modifying the embodiments in various ways conceivable by those skilled in the art and modes constructed by combining together components from different embodiments, insofar as the spirit of the present disclosure is not deviated from.

For example, the property display apparatus according to each of the above embodiments is a part of a display system, but may include all the components of the display system, instead. For example, the property display apparatus may include the input unit 1, the predictor database 2, the experiment database 3, and the like. The property display apparatus may also include processors. The property display apparatus may be implemented as a single computer apparatus, or may be implemented as computer apparatuses communicably connected to each other. That is, the components included in the property display apparatus according to each of the above embodiments need not be included in the same apparatus, and may be distributed to different apparatuses, instead.

In each of the above embodiments, each of the components may be achieved by dedicated hardware or by executing a software program suitable therefor. Each of the components may be achieved by reading and executing a software program stored in a storage medium, such as a hard disk or a semiconductor memory, using a program execution unit such as a CPU or a processor, instead. Here, the program for achieving the property display apparatus and the like according to each of the above embodiments may cause a processor to execute the steps included in at least one of the flowcharts of Figs. 16, 20, 23, 27, 31, and 36.

(Hardware Configuration)

The display systems 1000 to 6000 may each be specifically achieved by a computer system including a microprocessor, a ROM (read-only memory), a RAM (random-access memory), a hard disk drive, a display unit, a keyboard, a mouse, and the like. The RAM or the hard disk drive stores a display program. The display systems 1000 to 6000 each achieve the functions thereof when the microprocessor operates in accordance with the display program. The display program is constructed by combining together command codes indicating instructions to the computer in order to achieve certain functions.

Furthermore, a subset or all of the components of each of the display systems 1000 to 6000 may be achieved by a single system LSI (large scale integration). A system LSI is a super-multifunction LSI fabricated by integrating components on a single chip and, more specifically, is a computer system including a microprocessor, a ROM, a RAM, and the like. The RAM stores a computer program, The system LSI achieves functions thereof when the microprocessor operates in accordance with the computer program.

A subset or all of the components of each of the display systems 1000 to 6000 may be achieved by an IC card removably attached to a computer or an independent module. The IC card or the module is a computer system including a microprocessor, a ROM, a RAM, and the like. The IC card or the module may include the super-multifunction LSI. The IC card or the module achieves functions thereof when the microprocessor operates in accordance with a computer program. The IC card or the module may be tamper-resistant.

The present disclosure may be a display method executed by the display systems 1000 to 6000, instead. The display method may be achieved by executing a display program using a computer, or by a digital signal including the display program.

Furthermore, the present disclosure may be achieved by a non-transitory storage medium with which a computer can read the display program or the digital signal. The storage medium may be, for example, a flexible disk, a hard disk, a CD-ROM, an MO, a DVD, a DVD-ROM, a DVD-RAM, a BD (Blu-ray (registered trademark) Disc), a semiconductor memory, or the like. The display program may be achieved by the digital signal stored in the non-transitory storage medium, instead.

The present disclosure may be implemented by transferring the display program or the digital signal via electrical communication lines, wireless or wired communication lines, a network typified by the Internet, data broadcasting, or the like.

The present disclosure may be a computer system including a microprocessor and a memory, instead. The memory may store a display program, and the microprocessor may operate in accordance with the display program.

The present disclosure may be implemented by another independent computer system by storing the display program or the digital signal in the non-transitory storage medium and transporting the non-transitory storage medium or by transporting the display program or the digital signal over the network or the like, instead.

The display system may be achieved by a server and a terminal that is owned by the user and that is connected to the server over a network.

Industrial Applicability

The present disclosure is effective in displaying experiment values or predicted values of a material property in a search area while improving traceability and utilizing prediction in material development based on machine learning or the like.

Reference Signs List

1 input unit
2 predictor database (DB)
3 experiment database (DB)
4 selection reception unit
5 compound information database (DB)
6 display unit
21 edit information input unit
31 save input unit
32 read input unit
33 save database (DB)
41 correction input unit

42 converter database (DB)
51 prediction model selection reception unit
100, 200, 300, 400, 500, 600 property display apparatus
101 property value obtaining unit
102 image generation unit
103 reduced image generation unit
104 data obtaining unit
105 data image generation unit
106 image arrangement determination unit
201 edit image generation unit
301 save data generation unit
302 save data reconfiguration unit
401 data correction unit
402 corrected image generation unit
501 prediction model image generation unit
601 predicted data obtaining unit
1000, 2000, 3000, 4000, 5000, 6000 display system


## Claims

1. A property display method comprising:

   a first step of obtaining variables for determining compositions of compounds and pieces of option data indicating possible values or elements of each of the variables;
   a second step of determining the compositions of the compounds by selecting one of the pieces of option data for each of the variables and obtaining predicted property values of the compounds corresponding to the determined compositions of the compounds;
   a third step of obtaining experimental property values of the compounds corresponding to the determined compositions of the compounds;
   a fourth step of generating a map indicating, at positions corresponding to the determined compositions of the compounds, the predicted property values of the compounds corresponding to the compositions, generating a first image by superimposing the experimental property values upon the map at positions corresponding to the compositions of the compounds according to the experimental property values, and outputting the first image to a display unit;
   a fifth step of obtaining a piece of compound identification information corresponding to a selected one of the experimental property values on the map;
   a sixth step of obtaining a piece of compound information data associated with the piece of compound identification information and relating to the selected experimental property value; and
   a seventh step of generating a second image including a compound data image indicating the piece of compound information data and the first image and outputting the second image to the display unit.

2. The property display method according to claim 1, further comprising:

   a step of generating a reduced first image, which is obtained by reducing the first image, before the second image is generated in the seventh step,
   wherein, in the seventh step, the second image including the compound data image and the reduced first image is generated and output to the display unit.

3. The property display method according to claim 1,

   wherein a screen in which the first image and the second image are displayed in the fourth and seventh steps, respectively, includes a first area and a second area,
   wherein, in the fourth step, the first image disposed in the first area is output to the display unit, and
   wherein, in the seventh step, the second image is generated by disposing the first image in the first area and the compound data image in the second area and output to the display unit.

4. The property display method according to any of claims 1 to 3,

wherein the piece of compound information data includes a piece of compound composition data and a piece of compound graph data relating to the experimental property value.

5. The property display method according to claim 4,

wherein, in the fifth step, two or more of the experimental property values superimposed upon the map are selected and a piece of the compound identification information corresponding to each of the two or more experimental property values is obtained,
wherein, in the sixth step, a piece of the compound information data associated with each of the pieces of compound identification information is obtained, and
wherein, in the seventh step, the compound data image is generated by superimposing a piece of the compound graph data corresponding to each of the obtained pieces of compound information data.

6. The property display method according to claim 5,

wherein the compound data image in the seventh step includes
a graph superimposition image in which the piece of compound graph data corresponding to each of the obtained pieces of compound information data is superimposed, and
a list image in which the piece of compound identification information associated with each of the obtained pieces of compound information data is shown on a list.

7. The property display method according to claim 5,

wherein the compound data image in the seventh step includes
a graph superimposition image in which the piece of compound graph data corresponding to each of the obtained pieces of compound information data is superimposed, and
a tab switch image including tabs for switching and displaying the piece of compound composition data corresponding to each of the pieces of the compound graph data.

8. The property display method according to claim 6 or 7,

wherein, after the seventh step, at least one of the superimposed pieces of compound graph data shown in the graph superimposition image is edited, and
wherein a third image is generated on a basis of the edit and output to the display unit.

9. The property display method according to claim 8,
wherein the edit includes switching a state of, among the superimposed pieces of compound graph data, a selected one of the pieces of compound graph data and the piece of compound identification information associated with the selected pieces of the compound graph data between a display state and a non-display state.

10. The property display method according to claim 8 or 9,
wherein the edit includes selecting an area in the graph superimposition image and enlarging and displaying the selected area.

11. The property display method according to claim 4,
wherein, after the seventh step, an area in an image that indicates the piece of compound graph data and that is included in the compound data image is selected and the selected area is enlarged and displayed.

12. The property display method according to any of claims 1 to 11,
wherein, after at least one of the first image or the second image is output to the display unit, the output image is converted into a file in a certain format and saved.

13. The property display method according to claim 12,

wherein the saved file is obtained, and
wherein a fourth image is generated by reconfiguring the obtained file as an image and output to the display unit.

14. The property display method according to any of claims 1 to 13,

wherein, in the seventh step, if information included in the piece of compound information data shown in the second image is corrected after the second image is output, the corrected information is output to a compound information database storing the piece of compound information data before the correction, and a fifth image is generated by correcting the second image on a basis of the corrected information and output to the display unit.

15. The property display method according to any of claims 1 to 14,

wherein, in the fourth step, a prediction model image based on the predicted property values and at least one of the experimental property values shown in the first image is generated after the first image is output,
wherein a sixth image including the first image and the prediction model image is generated and output to the display unit,
wherein, in a case where one of the at least one experimental property value shown in the prediction model image is selected, a piece of the compound identification information corresponding to the selected experimental property value is obtained,
wherein a piece of the compound information data associated with the piece of compound identification information is obtained, and
wherein a seventh image including an image indicating the piece of compound information data and the sixth image is generated and output to the display unit.

16. The property display method according to any of claims 1 to 15,
wherein, in the sixth step, a piece of predicted property data corresponding to the piece of compound identification information is calculated while obtaining the piece of compound information data associated with the piece of compound identification information, an eighth image is generated on a basis of the piece of compound information data and the piece of predicted property data, and a ninth image including the eighth image and the first image is generated and output to the display unit.

17. The property display method according to any of claims 1 to 16,
wherein a number of, among the variables, first variables for which the pieces of option data indicating chemical elements are substituted is four or more.

18. The property display method according to any of claims 1 to 17,
wherein a number of, among the variables, second variables for which the pieces of option data indicating values are substituted is four or more.

19. The property display method according to claim 18,

wherein the map includes an image map defined by a first axis and a second axis corresponding to two of the four or more second variables,
wherein the image map includes image element maps arranged in a matrix along the first and second axes,
wherein each of the image element maps is defined by a third axis and a fourth axis corresponding to other two of the four or more second variables, and
wherein the predicted property values of the compounds corresponding to the determined compositions of the compounds are indicated by colors or shades of color on the image element maps at the positions corresponding to the compositions of the compounds.

20. The property display method according to any of claims 1 to 19,

wherein, in the second step, the predicted property values of the compounds corresponding to the determined compositions of the compounds are obtained by inputting the compositions of the compounds to a machine learning model based on a certain calculation algorithm, and
wherein the machine learning model is a model subjected to machine learning such that the predicted property values of the compounds corresponding to the determined compositions of the compounds are output in response to an input of the compositions of the compounds.

21. A property display method comprising:

a first step of obtaining a map indicating a position corresponding to each of compositions of compounds;
a second step of obtaining an experimental property value of each of at least one of the compositions of the compounds;

a third step of generating a first image by superimposing the obtained experimental property value upon the map at a position corresponding to the composition corresponding to the experimental property value and outputting the first image to a display unit;

a fourth step of obtaining a piece of compound information data corresponding to a selected one of the at least one experimental property value on the map; and

a fifth step of generating a second image including an image indicating the piece of compound information data and the first image and outputting the second image to the display unit.

**22.** The property display method according to claim 21,

wherein the map obtained in the first step indicates a predicted property value of each of the compositions of the compounds at a position corresponding to the composition,

the property display method further comprising:

a sixth step of obtaining a piece of compound identification information corresponding to the experimental property value selected on the map,

wherein, in the fourth step, the piece of compound information data associated with the piece of compound identification information and relating to the selected experimental property value is obtained, and

wherein, in the fifth step, the second image including the image indicating the piece of compound information data and the first image is generated and output to the display unit.

**23.** The property display method according to claim 22, further comprising:

a seventh step of obtaining a predicted property value of each of the compositions of the compounds,

wherein, in the first step, the map is obtained by generating the map using the obtained predicted property value.

**24.** A property display apparatus comprising:

a first obtaining unit that obtains variables for determining compositions of compounds and pieces of option data indicating possible values or elements of each of the variables;

a second obtaining unit that determines the compositions of the compounds by selecting one of the pieces of option data for each of the variables and that obtains predicted property values of the compounds corresponding to the determined compositions of the compounds;

a third obtaining unit that obtains experimental property values of the compounds corresponding to the determined compositions of the compounds;

a first image processing unit that generates a map indicating, at positions corresponding to the determined compositions of the compounds, the predicted property values of the compounds corresponding to the compositions, that generates a first image by superimposing the experimental property values upon the map at positions corresponding to the compositions of the compounds according to the experimental property values, and that outputs the first image to a display unit;

a fourth obtaining unit that obtains a piece of compound identification information corresponding to a selected one of the experimental property values on the map;

a fifth obtaining unit that obtains a piece of compound information data associated with the piece of compound identification information and relating to the selected experimental property value; and

a second image processing unit that generates a second image including a compound data image indicating the piece of compound information data and the first image and that outputs the second image to the display unit.

**25.** An information processing apparatus comprising:

a memory; and

a processor,

wherein the processor performs, using the memory,

a first step of obtaining variables for determining compositions of compounds and pieces of option data indicating possible values or elements of each of the variables;

a second step of obtaining, using a certain calculation algorithm, a predicted property value of the compound corresponding to each of combinations of pieces of option data obtained by selecting one of the pieces of option data for each of the variables;

a third step of obtaining an experimental property value of the compound corresponding to each of at least one of the combinations;

a fourth step of generating a map indicating the predicted property value corresponding to each of the combinations, generating a first image by superimposing each of the at least one obtained experimental property value upon the map at a position corresponding to the combination corresponding to the experimental property value, and outputting the first image to a display unit;

a fifth step of obtaining a piece of compound identification information corresponding to a selected one of the at least one experimental property value on the map;

a sixth step of obtaining a piece of compound information data associated with the piece of compound identification information and relating to the selected experimental property value; and

a seventh step of generating a second image including a compound data image indicating the piece of compound information data and the first image in one screen and outputting the second image to the display unit.

**26.** A program causing a computer to perform a process comprising:

a first step of obtaining variables for determining compositions of compounds and pieces of option data indicating possible values or elements of each of the variables;

a second step of determining the compositions of the compounds by selecting one of the pieces of option data for each of the variables and obtaining predicted property values of the compounds corresponding to the determined compositions of the compounds;

a third step of obtaining experimental property values of the compounds corresponding to the determined compositions of the compounds;

a fourth step of generating a map indicating, at positions corresponding to the determined compositions of the compounds, the predicted property values of the compounds corresponding to the compositions, generating a first image by superimposing the experimental property values upon the map at positions corresponding to the compositions of the compounds according to the experimental property values, and outputting the first image to a display unit;

a fifth step of obtaining a piece of compound identification information corresponding to a selected one of the experimental property values on the map;

a sixth step of obtaining a piece of compound information data associated with the piece of compound identification information and relating to the selected experimental property value; and

a seventh step of generating a second image including a compound data image indicating the piece of compound information data and the first image in one screen and outputting the second image to the display unit.

**27.** A property display method comprising:

causing a display to display a first image including graphs including a first graph; and

causing, in a case where first data included in the first graph is selected, the display to display a second image including an image obtained by reducing the first screen and information relating to the first data,

wherein the graphs are a $graph_{11}$ to a $graph_{pq}$ to a $graph_{rs}$ ($2 \le r$, $2 \le s$, r is a natural number, s is a natural number, $1 \le p \le r$, $1 \le q \le s$, p is a natural number, and q is a natural number),

wherein values $e_{pq(1,1)}$ to $e_{pq(i,j)}$ to $e_{pq(n,m)}$ are shown in the $graph_{pq}$ (n is a natural number larger than or equal to 2, m is a natural number larger than or equal to 2, i is a natural number, and j is a natural number),

wherein the value $e_{pq(i,j)}$ is a value of a predetermined property corresponding to a chemical formula including a first value, a second value, a third value, and a fourth value, the first value corresponding to p, the second value corresponding to q, the third value corresponding to i, the fourth value corresponding to j,

wherein the chemical formula includes a first chemical formula including a first chemical symbol and a second chemical formula including a second chemical symbol,

wherein the first value is a subscript value added to the first chemical formula, the second value is a subscript value added to the second chemical formula, the third value is a subscript value added to the first chemical symbol, and the fourth value is a subscript value added to the second chemical symbol,

wherein a predictor is tuned using pieces of training data including first training data,

wherein the first training data is a value e of the predetermined property of a produced compound, a ninth value, a tenth value, an eleventh value, and a twelfth value for determining a fifth value, a sixth value, a seventh value, and an eighth value included in a chemical formula of a target compound corresponding to the produced compound, the target compound being a compound to be produced,

wherein the fifth value corresponds to the first value, the sixth value corresponds to the second value, the seventh value corresponds to the third value, and the eighth value corresponds to the fourth value,

wherein the value e is calculated on a basis of first data obtained by measuring a first property of the produced compound and second data obtained by measuring a second property of the produced compound, the predetermined property being different from the first property, the predetermined property being different from the second property,

wherein, in a case where a value corresponding to p, a value corresponding to q, a value corresponding to i, and a value corresponding to j are input to the tuned predictor, the tuned predictor outputs the value $e_{pq(i,j)}$, the first data being the value e, and

wherein the information relating to the first data includes a first graph including the first data and a second graph including the second data.

## FIG. 1

# FIG. 2

DATA REPRESENTATION: $Li_{2-3a-4b}(M3_{1-x}M3'_{x})_{a}(M4_{1-y}M4'_{y})_{1+b}O_{3}$

**AREA VARIABLES**

CATEGORICAL VARIABLES

| VARIABLE NAMES | OPTIONS | NUMBER OF WAYS |
|---|---|---|
| M3, M3' | La,Al,Ga,In | 6 |
| M4, M4' | Ti,Zr,Hf | 3 |

DISCRETE VARIABLES

| VARIABLE NAMES | OPTIONS | NUMBER OF WAYS |
|---|---|---|
| a | 0.0, 0.05, 0.1, 0.15, 0.2 | 5 |
| b | 0.0, 0.1, 0.2, 0.3 | 4 |

CONTINUOUS VARIABLES

| VARIABLE NAMES | MINIMUM VALUE | MAXIMUM VALUE | STEP WIDTH | NUMBER OF WAYS |
|---|---|---|---|---|
| x | 0.0 | 1.0 | 0.1 | 11 |
| y | 0.0 | 1.0 | 0.1 | 11 |

## FIG. 3

EXPERIMENTAL DATA

| formula | ID | exp.data |
|---|---|---|
| Li1.45La0.045Ti1.1Al0.005O3 | 000001-00001-001 | 2.349 |
| Li1.45La0.045Zr0.22Ti0.88Al0.005O3 | 000002-00001-001 | 2.462 |
| Li1.45La0.045Zr0.55Ti0.55Al0.005O3 | 000003-00001-001 | 2.578 |
| Li1.45La0.045Zr0.99Ti0.11Al0.005O3 | 000004-00001-001 | 2.621 |
| Li1.45La0.045Zr1.1Al0.005O3 | 000005-00001-001 | 2.594 |

FIG. 4A

EP 4 206 971 A1

FIG. 4B

FIRST IMAGE

IMAGE ARRAY VARIABLES (COMBINATIONS BETWEEN M3 AND M3')

IMAGE ARRAY VARIABLES (COMBINATIONS BETWEEN M4 AND M4')

# FIG. 4C

[A] TO [R]

b=0.3 / b=0.2 / b=0.1 / b=0.0

a=0.0  a=0.05  a=0.1  a=0.15  a=0.2

band_gap [eV]: 4.0 / 3.5 / 3.0 / 2.5 / 2.0 / 1.5 / 1.0 / 0.5

A: Ti Hf Al-Ga  $Li_{2-3a-4b}(Al_{1-x}Ga_x)_a(Ti_{1-y}Hf_y)_{1+b}O_3$

B: Ti Hf Al-In  $Li_{2-3a-4b}(Al_{1-x}In_x)_a(Ti_{1-y}Hf_y)_{1+b}O_3$

C: Ti Hf Ga-In  $Li_{2-3a-4b}(Ga_{1-x}In_x)_a(Ti_{1-y}Hf_y)_{1+b}O_3$

D: Ti Hf La-Al  $Li_{2-3a-4b}(La_{1-x}Al_x)_a(Ti_{1-y}Hf_y)_{1+b}O_3$

E: Ti Hf La-Ga  $Li_{2-3a-4b}(La_{1-x}Ga_x)_a(Ti_{1-y}Hf_y)_{1+b}O_3$

F: Ti Hf La-In  $Li_{2-3a-4b}(La_{1-x}In_x)_a(Ti_{1-y}Hf_y)_{1+b}O_3$

G: Ti Zr Al-Ga  $Li_{2-3a-4b}(Al_{1-x}Ga_x)_a(Ti_{1-y}Zr_y)_{1+b}O_3$

H: Ti Zr Al-In  $Li_{2-3a-4b}(Al_{1-x}In_x)_a(Ti_{1-y}Zr_y)_{1+b}O_3$

I: Ti Zr Ga-In  $Li_{2-3a-4b}(Ga_{1-x}In_x)_a(Ti_{1-y}Zr_y)_{1+b}O_3$

J: Ti Zr La-Al  $Li_{2-3a-4b}(La_{1-x}Al_x)_a(Ti_{1-y}Zr_y)_{1+b}O_3$

K: Ti Zr La-Ga  $Li_{2-3a-4b}(La_{1-x}Ga_x)_a(Ti_{1-y}Zr_y)_{1+b}O_3$

L: Ti Zr La-In  $Li_{2-3a-4b}(La_{1-x}In_x)_a(Ti_{1-y}Zr_y)_{1+b}O_3$

M: Zr Hf Al-Ga  $Li_{2-3a-4b}(Al_{1-x}Ga_x)_a(Zr_{1-y}Hf_y)_{1+b}O_3$

N: Zr Hf Al-In  $Li_{2-3a-4b}(Al_{1-x}In_x)_a(Zr_{1-y}Hf_y)_{1+b}O_3$

O: Zr Hf Ga-In  $Li_{2-3a-4b}(Ga_{1-x}In_x)_a(Zr_{1-y}Hf_y)_{1+b}O_3$

P: Zr Hf La-Al  $Li_{2-3a-4b}(La_{1-x}Al_x)_a(Zr_{1-y}Hf_y)_{1+b}O_3$

Q: Zr Hf La-Ga  $Li_{2-3a-4b}(La_{1-x}Ga_x)_a(Zr_{1-y}Hf_y)_{1+b}O_3$

R: Zr Hf La-In  $Li_{2-3a-4b}(La_{1-x}In_x)_a(Zr_{1-y}Hf_y)_{1+b}O_3$

EP 4 206 971 A1

# FIG. 5A

EP 4 206 971 A1

# FIG. 5B

FIRST IMAGE

CATEGORICAL VARIABLES (COMBINATIONS BETWEEN M4 AND M4')

CATEGORICAL VARIABLES (COMBINATIONS BETWEEN M3 AND M3')

# FIG. 6

COMPOUND INFORMATION DATA

COMPOUND COMPOSITION DATA

| SAMPLE ID | 000486-00001-001 |
|---|---|
| RESEARCHER/EXPERIMENTER | TANAKA/SATO |
| REGISTRATION DATE | 02/03/2020 |
| TARGET COMPOSITION | Li1.7Al0.04Ga0.06Ti0.2Zr0.8O3 |
| ACTUAL COMPOSITION | Li1.706Al0.041Ga0.059Ti0.2Zr0.8O3.003 |
| RAW MATERIALS | Li2O / La2O3 / Al2O3 / TiO2 |
| CALCINATION CONDITIONS | 1000℃-5 h / 1000℃-5 h / 100℃-5 h |
| IMPEDANCE | 235 Ω |
| OXIDATION POTENTIAL | 3.5 V |
| CRYSTALLINE PHASE | cubic-perovskite |

COMPOUND GRAPH DATA

| SAMPLE ID | 000486-00001-001 |
|---|---|
| PROPERTY 1 (PEIS) | {"Re(Z) / Ohm":[10, 20, 50, 100, 200, ⋯], "-Im(Z) / Ohm":[-5, -2, 0, 10, 20, ⋯]} |
| PROPERTY 2 (CV) | {"E / V":[2.0, 1.9, 1.8, 1.7, 1.6, ⋯], "I / mA":[-0.001, -0.001, -0.002, -0.002, -0.002, ⋯]} |
| PROPERTY 3 (XRD) | {"2 theta / degree":[10, 10.01, 10.02, 10.03, ⋯], "Intensity / a.u.":[283, 294, 274, 291, ⋯]} |

EP 4 206 971 A1

# FIG. 7

COMPOUND COMPOSITION DATA IMAGE

COMPOUND DATA IMAGE

COMPOUND GRAPH DATA IMAGE

## BASIC INFORMATION

| SAMPLE ID | 000486-00001-001 |
|---|---|
| RESEARCHER/EXPERIMENTER | TANAKA/SATO |
| REGISTRATION DATE | 02/03/2020 |
| TARGET COMPOSITION | Li1.7Al0.04Ga0.06Ti0.2Zr0.8O3 |
| ACTUAL COMPOSITION | Li1.706Al0.041Ga0.059Ti0.2Zr0.8O3.003 |
| RAW MATERIALS | Li2O / Al2O3 / Ga2O3 / TiO2/ ZrO2 |
| CALCINATION CONDITIONS | 1000 ℃-5 h / 1000 ℃-5 h / 100 ℃-5 h |
| IMPEDANCE | 235 Ω |
| OXIDATION POTENTIAL | 3.5 V |
| CRYSTALLINE PHASE | cubic-perovskite |

EP 4 206 971 A1

# FIG. 8

SECOND IMAGE

FIRST IMAGE
(REDUCED FIRST IMAGE)

COMPOUND
DATA IMAGE

FIG. 9

## FIG. 10A

FIRST IMAGE

$Li_{2-3a-4b}(Al_{1-x}Ga_x)_a(Ti_{1-y}Zr_y)_{1+b}O_3$

$Li_{2-3a-4b}(La_{1-x}Ga_x)_a(Ti_{1-y}Zr_y)_{1+b}O_3$

SELECT EXPERIMENTAL PROPERTY VALUE

REDUCED FIRST IMAGE

SECOND IMAGE

BASIC INFORMATION   PROPERTY 1 (PEIS)   PROPERTY 2 (CV)   PROPERTY 3 (XRD)

COMPOUND DATA IMAGE

FIG. 10B

FIG. 10C

FIRST IMAGE

SECOND IMAGE

REDUCED FIRST IMAGE

COMPOUND DATA IMAGE

SELECT EXPERIMENTAL PROPERTY VALUE

PROPERTY 3 (XRD)

PROPERTY 2 (CV)

PROPERTY 1 (PEIS)

BASIC INFORMATION

FIG. 11

# FIG. 12

LIST IMAGE

COMPOUND DATA IMAGE

GRAPH SUPERIMPOSITION IMAGE

EP 4 206 971 A1

# FIG. 13

COMPOUND DATA IMAGE

TAB SWITCH IMAGE

GRAPH SUPERIMPOSITION IMAGE

## BASIC INFORMATION

| SAMPLE ID | 000486-00001-001 |
|---|---|
| RESEARCHER/EXPERIMENTER | TANAKA/SATO |
| REGISTRATION DATE | 02/03/2020 |
| TARGET COMPOSITION | Li1.7Al0.04Ga0.06Ti0.2Zr0.8O3 |
| ACTUAL COMPOSITION | Li1.706Al0.041Ga0.059Ti0.2Zr0.8O3.003 |
| RAW MATERIALS | Li2O / Al2O3 / Ga2O3 / TiO2/ ZrO2 |
| CALCINATION CONDITIONS | 1000 ℃-5 h / 1000 ℃-5 h / 100 ℃-5 h |
| IMPEDANCE | 235 Ω |
| OXIDATION POTENTIAL | 3.5 V |
| CRYSTALLINE PHASE | cubic-perovskite |

PROPERTY 1 (PEIS)

PROPERTY 2 (CV)

PROPERTY 3 (XRD)

# FIG. 14

FIRST IMAGE

$Li_{2-3a-4b}(Al_{1-x}Ga_x)_a(Ti_{1-y}Zr_y)_{1+b}O_3$

$Li_{2-3a-4b}(La_{1-x}Ga_x)_a(Ti_{1-y}Zr_y)_{1+b}O_3$

SELECT EXPERIMENTAL PROPERTY VALUE

SECOND IMAGE

REDUCED FIRST IMAGE

BASIC INFORMATION    PROPERTY 1 (PEIS)    PROPERTY 2 (CV)    PROPERTY 3 (XRD)

COMPOUND DATA IMAGE

# FIG. 15

FIRST IMAGE

SELECT EXPERIMENTAL PROPERTY VALUE

SECOND IMAGE

REDUCED FIRST IMAGE

BASIC INFORMATION    PROPERTY 1 (PEIS)    PROPERTY 2 (CV)    PROPERTY 3 (XRD)

COMPOUND DATA IMAGE

# FIG. 16

START

OBTAIN PROPERTY VALUES — S101

GENERATE FIRST IMAGE — S102

DISPLAY FIRST IMAGE — S103

RECEIVE SELECTION OF EXPERIMENTAL PROPERTY VALUE — S104

OBTAIN COMPOUND INFORMATION DATA — S105

GENERATE COMPOUND DATA IMAGE — S106

REDUCE FIRST IMAGE? — S107   NO

YES

GENERATE REDUCED FIRST IMAGE — S108

ARRANGE IMAGES — S109

DISPLAY SECOND IMAGE — S110

END

## FIG. 17

# FIG. 18

SECOND IMAGE

REDUCED FIRST IMAGE

COMPOUND DATA IMAGE

COMPOUND GRAPH DATA IMAGE
(GRAPH SUPERIMPOSITION IMAGE)

BASIC INFORMATION

PROPERTY 1 (PEIS)

PROPERTY 2 (CV)

PROPERTY 3 (XRD)

000486-00001-001
000824-00001-001
000833-00001-001
000835-00001-001

SELECT ID

SELECT DRAWING RANGE

EP 4 206 971 A1

# FIG. 19

FIG. 20

START

OBTAIN PROPERTY VALUES ⟩ S201

GENERATE FIRST IMAGE ⟩ S202

DISPLAY FIRST IMAGE ⟩ S203

RECEIVE SELECTION OF EXPERIMENTAL PROPERTY VALUE ⟩ S204

OBTAIN COMPOUND INFORMATION DATA ⟩ S205

GENERATE COMPOUND DATA IMAGE ⟩ S206

REDUCE FIRST IMAGE? ⟩ S207 — NO

YES

GENERATE REDUCED FIRST IMAGE ⟩ S208

ARRANGE IMAGES ⟩ S209

DISPLAY SECOND IMAGE ⟩ S210

RECEIVE INPUT EDIT INFORMATION ⟩ S211

GENERATE EDIT IMAGE ⟩ S212

ARRANGE IMAGES ⟩ S213

DISPLAY THIRD IMAGE ⟩ S214

END

# FIG. 21

EP 4 206 971 A1

# FIG. 22

FIRST IMAGE

EP 4 206 971 A1

FIG. 23

START

OBTAIN PROPERTY VALUES — S301

GENERATE FIRST IMAGE — S302

DISPLAY FIRST IMAGE — S303

RECEIVE DATA SAVE INPUT — S304    NO

YES

GENERATE AND SAVE DATA — S305

RECEIVE DATA READ INPUT — S306    NO

YES

RECONFIGURE DATA — S307

ARRANGE IMAGES — S308

DISPLAY FOURTH IMAGE — S309

RECEIVE SELECTION OF EXPERIMENTAL PROPERTY VALUE — S310

OBTAIN COMPOUND INFORMATION DATA — S311

GENERATE COMPOUND DATA IMAGE — S312

REDUCE FIRST IMAGE? — S313    NO

YES

GENERATE REDUCED FIRST IMAGE — S314

ARRANGE IMAGES — S315

DISPLAY SECOND IMAGE — S316

RECEIVE DATA SAVE INPUT — S304    NO

YES

GENERATE AND SAVE DATA — S305

RECEIVE DATA READ INPUT — S306    NO

YES

RECONFIGURE DATA — S307

ARRANGE IMAGES — S308

DISPLAY FOURTH IMAGE — S309

END

70

# FIG. 24

EP 4 206 971 A1

# FIG. 25

SELECTED EXPERIMENTAL PROPERTY VALUE

SECOND IMAGE

FIRST IMAGE (REDUCED FIRST IMAGE)

$Li_{2-3a-4b}(Al_{1-x}Ga_x)_a(Ti_{1-y}Zr_y)_{1+b}O_3$

$Li_{2-3a-4b}(La_{1-x}Ga_x)_a(Ti_{1-y}Zr_y)_{1+b}O_3$

COMPOUND DATA IMAGE

CORRECT DATA — C

## BASIC INFORMATION

| SAMPLE ID | 000486-00001-001 |
|---|---|
| RESEARCHER/EXPERIMENTER | TANAKA/SATO |
| REGISTRATION DATE | 02/03/2020 |
| TARGET COMPOSITION | Li1.7Al0.04Ga0.06Ti0.2Zr0.8O3 |
| ACTUAL COMPOSITION | Li1.706Al0.041Ga0.059Ti0.2Zr0.8O3.003 |
| RAW MATERIALS | Li2O / Al2O3 / Ga2O3 / TiO2 / ZrO2 |
| CALCINATION CONDITIONS | 1000 ℃-5 h / 1000 ℃-5 h / 100 ℃-5 h |
| IMPEDANCE | 235 Ω |
| OXIDATION POTENTIAL | 3.5 V |
| CRYSTALLINE PHASE | cubic-perovskite |

PROPERTY 1 (PEIS)

PROPERTY 2 (CV)

PROPERTY 3 (XRD)

EP 4 206 971 A1

# FIG. 26A

# FIG. 26B

FIG. 27

START

OBTAIN PROPERTY VALUES ⟋ S401

GENERATE FIRST IMAGE ⟋ S402

DISPLAY FIRST IMAGE ⟋ S403

RECEIVE SELECTION OF EXPERIMENTAL PROPERTY VALUE ⟋ S404

OBTAIN INFORMATION DATA ⟋ S405

GENERATE COMPOUND DATA IMAGE ⟋ S406

S407 ⟍
REDUCE FIRST IMAGE? — NO

YES

GENERATE REDUCED FIRST IMAGE ⟋ S408

ARRANGE IMAGES ⟋ S409

DISPLAY SECOND IMAGE ⟋ S410

RECEIVE INPUT CORRECTION ⟋ S411

CORRECT DATA ⟋ S412

GENERATE CORRECTED IMAGE ⟋ S413

ARRANGE IMAGES ⟋ S414

DISPLAY FIFTH IMAGE ⟋ S415

END

## FIG. 28

EP 4 206 971 A1

# FIG. 29

# FIG. 30

FIRST IMAGE (REDUCED FIRST IMAGE)   PREDICTION MODEL IMAGE

CLICK
"DISPLAY PREDICTION MODEL"
BUTTON

| DISPLAY PREDICTION MODEL |

d

SIXTH
IMAGE

SELECT EXPERIMENTAL
PROPERTY VALUE

SEVENTH
IMAGE

REDUCED
SIXTH IMAGE

| BASIC INFORMATION | PROPERTY 1 (PEIS) | PROPERTY 2 (CV) | PROPERTY 3 (XRD) |

COMPOUND
DATA IMAGE

EP 4 206 971 A1

FIG. 31

```
                    ( START )
                         │
                         ▼
        ┌──────────────────────────────┐
        │     OBTAIN PROPERTY VALUES    │────── S502
        └──────────────────────────────┘
                         │
                         ▼
        ┌──────────────────────────────┐
        │      GENERATE FIRST IMAGE     │────── S502
        └──────────────────────────────┘
                         │
                         ▼
        ┌──────────────────────────────┐
        │       DISPLAY FIRST IMAGE     │────── S503
        └──────────────────────────────┘
                         │
                         ▼
        ┌──────────────────────────────┐
        │ RECEIVE SELECTION OF PREDICTION MODEL │──── S504
        └──────────────────────────────┘
                         │
                         ▼
        ┌──────────────────────────────┐
        │  GENERATE PREDICTION MODEL IMAGE │──── S505
        └──────────────────────────────┘
                         │
                         ▼
              ◇ S506                        NO
        ◇ REDUCE PREDICTION MODEL IMAGE? ◇─────────┐
                         │                          │
                        YES                         │
                         ▼                          │
        ┌──────────────────────────────┐           │
        │ GENERATE REDUCED PREDICTION MODEL IMAGE │─ S507
        └──────────────────────────────┘           │
                         │◄─────────────────────────┘
                         ▼
              ◇ S508                        NO
        ◇      REDUCE FIRST IMAGE?      ◇──────────┐
                         │                          │
                        YES                         │
                         ▼                          │
        ┌──────────────────────────────┐           │
        │   GENERATE REDUCED FIRST IMAGE │──── S509  │
        └──────────────────────────────┘           │
                         │◄─────────────────────────┘
                         ▼
        ┌──────────────────────────────┐
        │         ARRANGE IMAGES        │──── S510
        └──────────────────────────────┘
                         │
                         ▼
        ┌──────────────────────────────┐
        │       DISPLAY SIXTH IMAGE     │──── S511
        └──────────────────────────────┘
                         │
                         ▼
        ┌──────────────────────────────────────┐
        │ RECEIVE SELECTION OF EXPERIMENTAL PROPERTY VALUE │── S512
        └──────────────────────────────────────┘
                         │
                         ▼
        ┌──────────────────────────────┐
        │  OBTAIN COMPOUND INFORMATION DATA │── S513
        └──────────────────────────────┘
                         │
                         ▼
        ┌──────────────────────────────┐
        │  GENERATE COMPOUND DATA IMAGE │──── S514
        └──────────────────────────────┘
                         │
                         ▼
              ◇ S515                        NO
        ◇      REDUCE SIXTH IMAGE?      ◇──────────┐
                         │                          │
                        YES                         │
                         ▼                          │
        ┌──────────────────────────────┐           │
        │   GENERATE REDUCED SIXTH IMAGE │──── S516  │
        └──────────────────────────────┘           │
                         │◄─────────────────────────┘
                         ▼
        ┌──────────────────────────────┐
        │         ARRANGE IMAGES        │──── S517
        └──────────────────────────────┘
                         │
                         ▼
        ┌──────────────────────────────┐
        │      DISPLAY SEVENTH IMAGE    │──── S518
        └──────────────────────────────┘
                         │
                         ▼
                    (  END  )
```

## FIG. 32

# FIG. 33

COMPOUND GRAPH DATA IMAGE

FIG. 34

COMPOUND GRAPH DATA IMAGE

PROPERTY 3 (XRD)

EP 4 206 971 A1

**FIG. 35**

FIG. 36

START

OBTAIN PROPERTY VALUES ⟋ S601

GENERATE FIRST IMAGE ⟋ S602

DISPLAY FIRST IMAGE ⟋ S603

RECEIVE SELECTION OF EXPERIMENTAL PROPERTY VALUE ⟋ S604

OBTAIN COMPOUND INFORMATION DATA ⟋ S605

OBTAIN PREDICTED PROPERTY DATA ⟋ S606

GENERATE EIGHTH IMAGE ⟋ S607

REDUCE FIRST IMAGE? ⟋ S608   NO

YES

GENERATE REDUCED FIRST IMAGE ⟋ S609

ARRANGE IMAGES ⟋ S610

DISPLAY NINTH IMAGE ⟋ S611

END

<div align="center">**INTERNATIONAL SEARCH REPORT**</div>

| International application No. |
| --- |
| **PCT/JP2021/030303** |

**A. CLASSIFICATION OF SUBJECT MATTER**

*G06F 30/12*(2020.01)i; *G06F 30/27*(2020.01)i
FI: G06F30/12; G06F30/27

According to International Patent Classification (IPC) or to both national classification and IPC

**B. FIELDS SEARCHED**

Minimum documentation searched (classification system followed by classification symbols)

G06F30/00-30/28; G16C20/00-20/90; G16C60/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2019/060268 A1 (COVESTRO LLC) 28 March 2019 (2019-03-28) claims | 1-27 |
| A | JP 2001-503546 A (3 DIMENSIONAL PHARMACEUTICALS INC.) 13 March 2001 (2001-03-13) claims | 1-27 |
| A | JP 4780554 B2 (YAMATO, Hiroshi) 28 September 2011 (2011-09-28) claims | 1-27 |

☐ Further documents are listed in the continuation of Box C.   ☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **12 October 2021** | **26 October 2021** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2021/030303**

| Patent document cited in search report | | | Publication date (day/month/year) | Patent family member(s) | | | Publication date (day/month/year) |
|---|---|---|---|---|---|---|---|
| WO | 2019/060268 | A1 | 28 March 2019 | US | 2020/0210056 | A1 | |
| | | | | JP | 2020-535566 | A | |
| | | | | KR | 10-2020-0052949 | A | |
| | | | | CN | 111417948 | A | |
| JP | 2001-503546 | A | 13 March 2001 | WO claims | 1998/020459 | A1 | |
| | | | | US | 6295514 | B1 | |
| JP | 4780554 | B2 | 28 September 2011 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4009670 B

**Non-patent literature cited in the description**

- **KEI TERAYAMA ; KOJI TSUDA ; RYO TAMURA.** Efficient recommendation tool of materials by an executable file based on machine learning. *Japanese Journal of Applied Physics,* 2019, vol. 58 (098001